(19) Europäisches Patentamt
European Patent Office
Office européen des brevets

(11) EP 2 676 952 A2

(12) DEMANDE DE BREVET EUROPEEN

(43) Date de publication:
**25.12.2013 Bulletin 2013/52**

(21) Numéro de dépôt: **13177661.9**

(22) Date de dépôt: **18.12.2003**

(51) Int Cl.:
***C07C 317/50*** (2006.01) ***C07C 315/06*** (2006.01)
***A61K 31/165*** (2006.01) ***A61P 25/00*** (2006.01)
*C07C 315/04* (2006.01) *C07C 317/28* (2006.01)

(84) Etats contractants désignés:
**AT BE BG CH CY CZ DE DK EE ES FI FR GB GR HU IE IT LI LU MC NL PT RO SE SI SK TR**

(30) Priorité: **20.12.2002 FR 0216412**

(62) Numéro(s) de document de la (des) demande(s) initiale(s) en application de l'article 76 CBE:
**03799631.1 / 1 572 635**

(71) Demandeur: **Teva Santé**
**92931 La Défense Cedex (FR)**

(72) Inventeurs:
- **Neckebrock, Olivier**
  **77340 Pontault Combault (FR)**
- **Courvoisier, Laurent**
  **F-60290 Laigneville (FR)**
- **Graf, Stéphanie**
  **F-95270 Belloy en France (FR)**
- **Serrure, Gilles**
  **F-94230 Cachan (FR)**
- **Coquerel, Gérard**
  **F-76520 Boos (FR)**
- **Rose, Sébastien**
  **F-60190 Arsy (FR)**
- **Besselievre, Christine**
  **F-91440 Bures sur Yvette (FR)**
- **Mallet, Franck**
  **F-76340 Blangy sur Bresle (FR)**
- **Van Langevelde, Adriaan Jan**
  **NL-1325 NG Alkere (NL)**
- **Leproust, Pierre**
  **94000 Creteil (FR)**

(74) Mandataire: **Oates, Edward Christopher**
**Carpmaels & Ransford LLP**
**One Southampton Row**
**London**
**WC1B 5HA (GB)**

Remarques:
•Cette demande a été déposée le 23-07-2013 comme demande divisionnaire de la demande mentionnée sous le code INID 62.
•Revendications déposées après la date de dépôt de la demande (règle 68(4) CBE).

(54) **Procédé de préparation et formes cristallines IV des énantiomères optiques du modafinil**

(57) L'invention a pour objet un procédé de préparation de formes cristallines des énantiomères optiques du modafinil, comprenant les étapes consistant à :i) dissoudre l'un des énantiomères optiques du modafinil dans un solvant autre que l'éthanol ;ii) cristalliser l'énantiomère du modafinil ;iii) récupérer la forme cristalline de l'énantiomère du modafinil ainsi obtenue. L'invention vise également un procédé de préparation des énantiomères optiques du modafinil.

EP 2 676 952 A2

## Description

L'invention concerne un procédé d'obtention de formes cristallines des énantiomères du modafinil, ainsi que les formes cristallines susceptibles d'être obtenues selon ce procédé.

L'invention vise également un nouveau procédé de préparation des. énantiomères optiques du modafinil à partir de l'acide (±) modafinique.

Le brevet US 4,177,290 décrit le modafinil sous forme racémique, encore dénommé (±) 2-(benzhydrylsulfinyl) acétamide ou (±) 2-[(di-phénylméthyl)sulfinyl] acétamide, à titre de composé ayant des propriétés stimulantes du système nerveux central.

Le brevet US 4,927,855 décrit les deux énantiomères optiques du modafinil. Il décrit plus particulièrement l'énantiomère lévogyre et son utilisation en tant qu'agent antidépresseur ou stimulant dans le traitement de l'hypersomnie et des troubles liés à la maladie d'Alzheimer. Le procédé de préparation des deux énantiomères optiques du modafinil à partir de l'acide (±) modafinique ou acide (±)-benzhydrylsulfinylacétique décrit dans ce document est représenté dans le schéma de synthèse suivant :

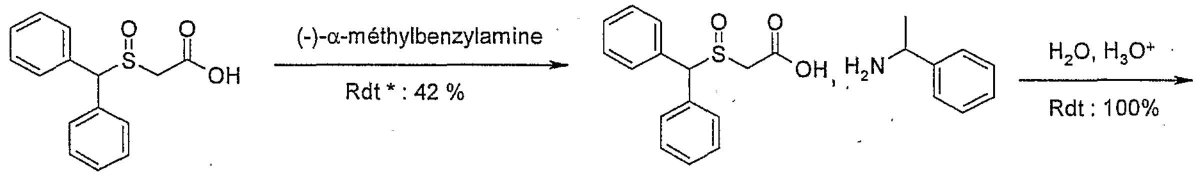


Acide (±) benzhydrylsulfinylacétique

(-)-benzhydrylsulfinylacétate de (-)-α-méthylbenzylamine

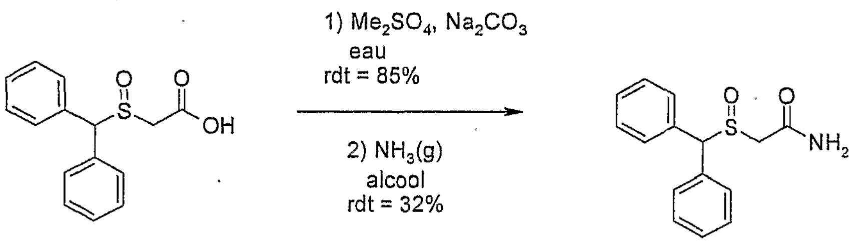


acide (-)-benzhydrylsulfinylacétique

(-)-benzhydrylsulfinylacétamide

**par rapport à l'acide (-)-benzhydrylsulfinylacétique*

Ce procédé consiste à réaliser, dans une première étape, un dédoublement des énantiomères optiques de l'acide (±) modafinique via la formation de diastéréoisomères avec l'agent optiquement actif α-méthylbenzylamine.

Le (-)-benzhydrylsulfinylacétate de (-)-α-méthylbenzylamine est ensuite converti par hydrolyse acide en acide (-)-benzhydrylsulfinylacétique. Celui-ci est estérifié en présence de diméthyl sulfate puis amidifié en présence d'ammoniac (gaz). L'énantiomère (-) ou I (lévogyre) du modafinil est obtenu par ce procédé avec un rendement global de 5,7% par rapport à l'acide (±) modafinique, calculé sur la base des rendements correspondant à chaque étape.

Le terme "énantiomère" désigne des molécules stéréoisomères qui sont des images en miroir l'une de l'autre non superposables. Les énantiomères sont typiquement désignés soit par (+) et (-) soit par (d) et (I), ce qui indique un pouvoir rotatoire optique au centre chiral.

La stéréoisomérie peut également être notée soit par (D) ou (L) soit par (R) et (S), qui sont des descriptifs de la configuration absolue.

Dans ce qui suit, l'énantiomère lévogyre du modafinil sera indifféremment désigné énantiomère I ou (-), l'énantiomère dextrogyre étant pour sa part désigné énantiomère d ou (+).

Il a maintenant été découvert un procédé permettant d'obtenir différentes formes cristallines des énantiomères optiques du modafinil. De façon plus spécifique, les inventeurs ont montré que la forme cristalline obtenue dépendait principalement de la nature du solvant de recristallisation mis en oeuvre.

Le terme "forme cristalline" désigne indifféremment, au sens de la présente description, une forme polymorphique ou un solvate.

Par "forme polymorphique", on entend une structure organisée n'impliquant que des molécules de soluté, possédant une empreinte cristalline caractéristique.

Le terme "solvate" désigne une structure organisée possédant une empreinte cristalline caractéristique impliquant à la fois des molécules de soluté et des molécules de solvant. Les solvates impliquant une molécule de soluté pour une molécule de solvant sont appelés solvates vrais.

Par ailleurs, les inventeurs ont montré que le l-modafinil et le d-modafinil préparés selon les conditions décrites dans le brevet US 4,177,290 sont obtenus sous forme d'une même forme polymorphique désignée forme I, laquelle correspond à la forme polymorphique thermodynamiquement la plus stable, dans les conditions normales de température et de pression.

La forme I présente le spectre de diffraction X ci-dessous dans lequel d représente la distance réticulaire et le rapport (I/Io) l'intensité relative.

| CRL 40982 FORME I | | |
|---|---|---|
| 2 Theta (degrés) | d(Å) | I/Io (%) |
| 9.8 | 13.40 | 32 |
| 15.4 | 8.54 | 87 |
| 20.8 | 6.34 | 24 |
| 26.4 | 5.01 | 14 |
| 28.3 | 4.68 | 19 |
| 28.7 | 4.62 | 16 |
| 29.9 | 4.44 | 45 |
| 31.1 | 4.27 | 100 |
| 31.6 | 4.20 | 23 |
| 32 | 4.15 | 14 |
| 33.1 | 4.02 | 78 |
| 33.4 | 3.98 | 84 |
| 34.1 | 3.90 | 16 |
| 35.1 | 3.80 | 15 |
| 39 | 3.43 | 22 |
| Diffractomètre: Miniflex Rigaku (Elexience) | | |

Les formes cristallines d'un composé donné présentent généralement des propriétés physiques, pharmaceutiques, physiologiques et biologiques très distinctes les unes des autres.

En ce sens, les formes cristallines du modafinil optiquement actif, en particulier les formes polymorphiques, sont intéressantes en ce qu'elles présentent des caractéristiques avantageuses et différentes par rapport à la forme I.

Il a maintenant été découvert, selon un autre aspect, un nouveau procédé de préparation des énantiomères optiques du modafinil à partir de l'acide (±)-modafinique, ledit procédé permettant d'isoler chaque énantiomère avec des rendements et une pureté optique nettement supérieurs à ceux décrits dans le brevet US 4,927,855.

De façon particulièrement avantageuse, il a maintenant été mis au point un procédé de dédoublement des deux énantiomères optiques de l'acide (±)-modafinique par cristallisation préférentielle, avantageusement applicable à l'échelle préparative.

Ce procédé de dédoublement de l'acide (±)-modafinique présente de nombreux avantages :

- il évite l'utilisation d'un agent chiral intermédiaire onéreux dont la préparation ultérieure implique des pertes rarement inférieures à 10% (De Min., M., Levy, G. et Micheau J.-C., 1988 ; J. Chem. Phys. 85, 603-19) ;
- les deux énantiomères sont obtenus directement, contrairement à la méthode mettant en oeuvre le dédoublement classique par formation de sels diastéréoisomères ;
- le rendement est théoriquement quantitatif par suite des recyclages successifs des eaux-mères ;
- la purification des cristaux d'énantiomères bruts est aisée.

L'invention vise donc à fournir un procédé de préparation des formes cristallines des énantiomères du modafinil.
L'invention a également pour but de proposer un nouveau procédé de préparation des énantiomères optiques du modafinil, et notamment de l'énantiomère lévogyre du modafinil.

• <u>PROCÉDÉ DE PRÉPARATION DES POLYMORPHES DU I-MODAFINIL</u>

Ces buts et d'autres sont atteints par la présente invention qui vise plus précisément, selon un premier aspect, un procédé de préparation de formes cristallines des énantiomères optiques du modafinil, comprenant les étapes suivantes :

i) dissoudre l'un des énantiomères optiques du modafinil dans un solvant autre que l'éthanol ;
ii) cristalliser ledit énantiomère du modafinil ; et
iii) récupérer la forme cristalline dudit énantiomère du modafinil ainsi obtenue.

Au sens de la présente invention, le solvant mis en oeuvre à l'étape i) du procédé, encore dénommé "solvant de recristallisation" est un solvant capable d'assurer la cristallisation dudit énantiomère optique du modafinil, de préférence à la pression atmosphérique. Il s'agit, en d'autres termes, de tout solvant A capable de former, à pression donnée, avec au moins l'un des énantiomères

- dans un premier domaine de température et de concentration, un système monophasé comprenant au moins l'un des énantiomères en solution diluée dans le solvant A ;
- dans un deuxième domaine de température et de concentration distinct du précédent, un deuxième système biphasé comprenant des cristaux dudit énantiomère en présence de solution saturée,

les deux domaines étant séparés l'un de l'autre par la courbe de solubilité dudit énantiomère T (°C) = f (concentration en énantiomère) à la pression considérée.
En général, la cristallisation de l'étape ii) consiste à passer du système monophasé au système biphasé en faisant varier la température et la concentration.
A titre illustratif et non limitatif de solvants pouvant convenir au procédé de recristallisation selon l'invention, on peut notamment citer les solvants alcooliques, les solvants esters d'acide carboxylique, les solvants éthérés, les solvants chlorés, les solvants aromatiques, les solvants cétoniques aliphatiques inférieurs. D'autres solvants sont, par exemple, les solvants acides carboxyliques, les solvants polaires non protiques, les hydrocarbures alicycliques, les hydrocarbures aliphatiques, les carbonates, les hétéroaromatiques, l'eau.
Parmi les solvants alcooliques, on peut citer notamment les alcools d'alkyles inférieurs tels que le méthanol, l'éthanol, le propanol, l'isopropanol, le butanol, l'isobutanol, le 2-méthyl-2-pentanol, le 1,2-propanediol, l'alcool de t-amyle, le méthanol, le propanol et l'isopropanol étant particulièrement préférés.
Parmi les solvants de type esters d'acide carboxylique, on peut citer notamment les acétates d'alkyle tels que l'acétate de méthyle, l'acétate d'éthyle, l'acétate de n-propyle, l'acétate d'isopropyle, l'acétate de n-butyle, les formates d'alkyle tels que le formate d'éthyle, l'acétate d'éthyle étant particulièrement préféré.
Sont utiles à titre de solvants éthérés de recristallisation, le diéthyléther, le tétrahydrofuranne (THF), le dioxanne, le dibutyléther, l'isopropyléther, le t-butylméthyléther, le tétrahydropyrane, le tétrahydrofuranne étant particulièrement préféré.
Parmi les solvants chlorés, on peut citer les hydrocarbures chlorés, notamment le chloroforme, le 1,2-dichloroéthane, le dichlorométhane et les aromatiques chlorés tels que le chlorobenzène.
Comme exemples de solvants aromatiques, on peut citer l'ortho, le méta, le paraxylène ou un mélange d'ortho, de méta et paraxylène, le méthoxybenzène, le nitrobenzène, le trifluorotoluène, le toluène, l'ortho, le méta et le paraxylène étant particulièrement préférés.
Sont utiles à titre de solvants cétoniques les solvants tels que l'acétone, la méthyléthylcétone, la méthylisobutylcétone, la butan-2-one, la cyclopentanone, l'isobutylméthylcétone, la 2-pentanone, la 3-pentanone.
Comme exemple de solvant acide carboxylique, on peut mentionner en particulier l'acide acétique.
A titre d'exemple de solvant hétéroaromatique, on peut citer en particulier la pyridine.
Des exemples de solvants polaires non protiques sont notamment l'acétonitrile, le propionitrile, la 4-méthylmorpholine, la N,N-dimé-thylacétamide, le nitrométhane, la triéthylamine, la N-méthyl-pyrrolidone (NMP).
Des exemples d'hydrocarbures aliphatiques sont notamment l'heptane, le 2,2-4-triméthylpentane.
Des exemples d'hydrocarbures alicycliques sont notamment le cyclopentane, le cyclohexane.
Des exemples de carbonates sont notamment les alkylcarbonates tels que le diméthylcarbonate.
Selon un mode de réalisation préférentiel du procédé selon l'invention, les solvants de cristallisation sont choisis parmi l'acétone, le méthanol, le dioxanne 1-4, l'acétate d'éthyle, les mélanges d'ortho, méta, paraxylène, l'isopropanol,

le n-propanol, le diméthylcarbonate, le tétrahydrofuranne, le chloroforme et la méthyléthylcétone, l'eau et les mélanges alcool/$H_2O$.

Ainsi, les formes cristallines des énantiomères optiques du modafinil peuvent être obtenues par recristallisation des énantiomères dans certains solvants, dont la nature et éventuellement les conditions de cristallisation déterminent principalement le type de forme cristalline obtenue.

Le solvant de recristallisation, par son interaction avec les groupements fonctionnels et les substituants électro-attracteurs ou électro-donneurs, peut en effet favoriser certains arrangements moléculaires qui donneront naissance à une forme cristalline déterminée dans des conditions de cristallisation données.

Généralement, on chauffe le solvant de recristallisation mis en oeuvre à l'étape i), notamment au reflux, jusqu'à obtenir la dissolution complète de l'énantiomère optique du modafinil dans le solvant. Si la concentration de l'énantiomère optique du modafinil à l'étape i) ne constitue pas un facteur critique de la cristallisation, on préfère cependant opérer en présence d'une concentration en énantiomère optique du modafinil proche de la concentration de saturation dans le solvant de recristallisation considéré.

Selon un mode de réalisation, l'énantiomère optique du modafinil est dissout en chauffant le solvant à reflux et une quantité supplémentaire dudit énantiomère optique est ensuite ajoutée par fractions de manière à atteindre la saturation. Du solvant supplémentaire peut être rajouté pour assurer une dissolution complète.

Selon un autre mode de réalisation, l'énantiomère optique du modafinil est mis en suspension dans le solvant chauffé au reflux, et une quantité supplémentaire de solvant est ensuite ajoutée par fractions de manière à obtenir une solution homogène et atteindre ainsi la saturation.

Le processus de cristallisation de l'énantiomère optique du modafinil à l'étape ii) peut être accéléré selon des techniques connues de l'homme du métier, à savoir le refroidissement de la solution, l'évaporation d'une partie du solvant, l'addition d'un anti-solvant ou l'ensemencement de la solution avec des cristaux de modafinil optiquement actif de même forme cristalline que celle attendue. Le plus souvent, le mélange est maintenu sous agitation tout au long du processus de cristallisation, de manière à obtenir une suspension homogène et un renouvellement rapide de la liqueur-mère autour de chaque cristallite.

Le processus de cristallisation du procédé selon l'invention peut être réalisé dans des conditions thermodynamiques ou cinétiques.

Au sens de la présente description, on entend par "cristallisation dans des conditions thermodynamiques" une cristallisation réalisée dans des conditions où l'équilibre entre la solution homogène, d'une part, et la solution saturée en présence de cristaux de l- ou d-modafinil, d'autre part, est maintenu.

A titre d'exemple, une cristallisation thermodynamique peut être réalisée en refroidissant lentement la solution obtenue à l'étape i), typiquement en laissant refroidir la solution à température ambiante ou en appliquant une vitesse ou une rampe de refroidissement inférieure ou égale à 0,75°C/mn, de préférence à 0,6°C et plus préférentiellement à 0,5°C/mn.

On entend par "cristallisation réalisée dans des conditions cinétiques", au sens de la présente description, une cristallisation dans laquelle l'équilibre entre la solution homogène, d'une part, et la solution saturée en présence de cristaux de d ou l-modafinil, d'autre part, est déplacé brutalement vers ce dernier domaine biphasé, i. e. vers la formation de cristaux.

A titre illustratif, une cristallisation dite cinétique peut être réalisée notamment par refroidissement rapide, par exemple en appliquant une rampe de refroidissement de 300°C/mn, ou bien par précipitation par ajout d'un antisolvant de la solution obtenue à l'étape i).

A titre illustratif et non limitatif, ces deux types de cristallisation thermodynamique ou cinétique sont réalisés dans la présente description par refroidissement lent ou rapide.

Bien entendu, tout autre technique de cristallisation telle que l'évaporation du solvant ou la précipitation, permettant de se placer dans des conditions cinétiques et/ou thermodynamiques, entre également dans le cadre du procédé selon l'invention.

Ainsi, selon un mode de réalisation particulier, la cristallisation à l'étape ii) peut être réalisée par précipitation, éventuellement en présence de germes de cristaux de la forme cristalline désirée.

Les inventeurs ont en outre montré que certains solvants peuvent conduire à des formes cristallines, plus spécifiquement à des formes polymorphiques, distinctes selon que la cristallisation est réalisée dans des conditions cinétiques ou thermodynamiques.

Selon un mode de réalisation privilégié, la cristallisation consiste en un refroidissement de la solution obtenue à l'étape i).

Le cas échéant, selon un premier mode, le refroidissement est rapide et correspond généralement à un trempage de la solution obtenue à l'étape i) dans un bain de température inférieure ou égale à 0°C tel qu'un bain d'eau glacé pendant un temps suffisant pour permettre une cristallisation complète de la solution, ou bien encore à un refroidissement avec une rampe de refroidissement comprise par exemple entre - 1°C et - 5°C/mn.

Selon un deuxième mode de réalisation, le refroidissement est lent. Dans ce cadre, on laisse généralement la

solution refroidir depuis la température de reflux du solvant jusqu'à température ambiante ou bien on refroidit la solution avec une rampe de refroidissement comprise de préférence entre - 0,1°C/mn et - 0,8°C/mn, et plus préférentiellement proche de - 0,5°C/mn, jusqu'à généralement une température de 15° à 20°C.

Parmi les combinaisons de solvants / antisolvants préférées selon l'invention, on peut citer notamment les combinaisons eau / acétone, acétonitrile / eau, éthanol/eau, méthanol / eau, acide acétique / eau.

Enfin, les formes cristallines des énantiomères optiques du modafinil peuvent être isolées selon des méthodes classiques telles que la filtration et la centrifugation.

A titre illustratif et non limitatif, le procédé de préparation selon l'invention est mis plus particulièrement en oeuvre avec l'énantiomère lévogyre du modafinil.

Selon un mode de réalisation particulier, la forme cristalline obtenue selon ce procédé est une forme polymorphique.

On notera à ce sujet que, de façon générale, chacun des énantiomères (l) et (d) d'un composé chimique donné conduisent, lorsqu'ils sont recristallisés dans les mêmes conditions expérimentales, à des formes cristallines, notamment polymorphiques, ayant des spectres de diffraction X réalisés sur poudre identiques.

A ce propos, on se référera notamment à l'ouvrage de J. Bernstein « Polymorphism in molecular crystals » 2002, University Press, Oxford, UK, et à la publication de G. Coquerel, Enantiomer, 2000; 5(5): 481-498 ; Gordon and Breach Science Publishers.

Pour ce motif, la forme dextrogyre dont les spectres de diffraction X des formes cristallines sont identiques à ceux de la forme lévogyre exposés ci-après et réciproquement font partie de l'invention.

Dans ce qui suit, les formes polymorphiques désignées formes I, II, III, IV et V couvrent ainsi les formes CRL40982 formes I, II, III, IV, V obtenues à partir de l'énantiomère lévogyre et les formes CRL40983 formes I, II, III, IV, V obtenues à partir de l'énantiomère dextrogyre.

Forme I

Dans ce cadre, le procédé mettant en oeuvre un solvant choisi parmi l'acétone, l'éthanol, le dioxanne 1-4, l'acétate d'éthyle et les mélanges d'ortho, méta, paraxylène, et une étape de cristallisation par refroidissement lent conduit à l'obtention de la forme I ou CRL40982 forme I.

Le procédé mettant en oeuvre un solvant choisi parmi le méthanol, l'eau ou les mélanges alcool/eau, en particulier méthanol/eau et éthanol/eau, ainsi qu'une étape de cristallisation par refroidissement rapide conduit à l'obtention de la forme I ou CRL 40982 Forme I.

Selon une autre variante également préférée de l'invention, le procédé mettant en oeuvre le méthanol et une étape de cristallisation par précipitation par ajout d'eau froide comme antisolvant du méthanol conduit à la forme I.

Forme II

Selon un autre mode de réalisation de l'invention, le procédé mettant en oeuvre un solvant à l'étape i) choisi parmi l'isopropanol, l'acétate d'éthyle, le n-propanol, ou de l'éthanol dénaturé au toluène, et une étape de cristallisation par refroidissement rapide conduit à une forme polymorphique désignée Forme II ou CRL 40982 forme II.

Selon une variante du procédé, la forme II peut être également obtenue par refroidissement lent dans l'isopropanol.

Il peut être ainsi remarqué que l'obtention de la forme II dans l'isopropanol ne dépend pas des conditions de cristallisation (thermodynamiques ou cinétiques).

Forme III

Selon une autre variante du procédé de l'invention, le solvant mis en oeuvre à l'étape i) est l'acétone, et l'étape de cristallisation ii) consiste en un refroidissement rapide, ceci conduisant apparemment à l'obtention d'une forme polymorphique désignée forme III ou CRL 40982 forme III.

Forme IV

En variante du procédé selon l'invention, le solvant mis en oeuvre à l'étape i) est choisi parmi le tétrahydrofuranne, le chloroforme et la méthyléthylcétone, et l'étape de cristallisation ii) consiste en un refroidissement lent de la solution, ce par quoi on obtient une forme polymorphique désignée forme IV ou CRL 40982 forme IV.

Le procédé de recristallisation des énantiomères optiques du modafinil peut, selon la nature du solvant mis en oeuvre, conduire à l'obtention de solvates.

Forme V

En variante du procédé selon l'invention, le solvant mis en oeuvre à l'étape i) est choisi parmi la 2-pentanone, et le tétrahydrofuranne, et l'étape de cristallisation ii) consiste en un refroidissement lent de la solution dans la 2-pentanone et rapide dans le THF, ce par quoi on obtient une forme polymorphique désignée forme V.

Solvate de diméthylcarbonate

Ainsi, selon un mode de réalisation particulier de l'invention, lorsque le solvant mis en oeuvre à l'étape i) est le diméthylcarbonate et que la cristallisation consiste en un refroidissement lent, on obtient un solvate de (-)-modafinil de diméthylcarbonate.

Solvate d'acide acétique

Selon un autre mode de réalisation particulier de l'invention, lorsque le solvant mis en oeuvre à l'étape i) est l'acide acétique et que la cristallisation consiste en un refroidissement lent ou rapide, un solvate d'acide acétique est obtenu.

• **FORMES POLYMORPHIQUES DU (-)-MODAFINIL**

L'invention a également pour objet la forme polymorphique de l'énantiomère lévogyre du modafinil désignée CRL 40982 forme II, caractérisée en ce qu'elle produit un spectre de diffraction X comprenant des raies d'intensité aux distances réticulaires : 11,33 ; 8,54 ; 7,57 ; 7,44 ; 4,56 ; 3,78 ; 3,71 Å, les raies d'intensité correspondant aux distances réticulaires de : 8,54 ; 7,57 ; 7,44 ; 4,56 ; 3,78 ; 3,71 Å étant particulièrement caractéristiques.

Plus précisément, le spectre de diffraction X suivant, dans lequel d représente la distance réticulaire et I/Io l'intensité relative :

| CRL 40982 FORME II | | |
|---|---|---|
| 2 Theta (degrés) | d (Å) | I/Io (%) |
| 11,6 | 11,33 | 54 |
| 15,4 | 8,54 | 58 |
| 17,4 | 7,57 | 41 |
| 17,7 | 7,44 | 34 |
| 23,3 | 5,67 | 19 |
| 24,8 | 5,33 | 26 |
| 27,4 | 4,83 | 19 |
| 28,9 | 4,59 | 36 |
| 29,1 | 4,56 | 97 |
| 29,8 | 4,45 | 23 |
| 32,8 | 4,05 | 29 |
| 34,3 | 3,88 | 23 |
| 35,3 | 3,78 | 100 |
| 35,9 | 3,71 | 40 |
| 40,1 | 3,34 | 21 |
| 47,7 | 2,83 | 20 |

(suite)

| CRL 40982 FORME II | | |
|---|---|---|
| 2 Theta (degrés) | d (Å) | I/Io (%) |
| 53,7 | 2,53 | 32 |
| Diffractomètre: Minitlex Rigaku (Elexience) | | |

L'invention a également pour objet la forme polymorphique de l'énantiomère lévogyre du modafinil désignée CRL 40982 forme III, caractérisée par le spectre de diffraction X comprenant des raies d'intensité aux distances réticulaires d suivantes : 13,40 ; 12,28 ; 8,54 ; 7,32 ; 6,17 ; 5,01 ; 4,10 ; 3,97 ; 3,42 ; 3,20 Å, et les distances réticulaires : 12,28 ; 8,54 ; 5,01 ; 4,10 ; 3,97 ; 3,42 ; 3,20 Å correspondant aux raies d'intensité les plus caractéristiques.
Dans ce cadre, l'invention vise plus particulièrement la forme III du (-)-modafinil produisant le spectre de diffraction X suivant dans lequel d représente la distance réticulaire et I/Io l'intensité relative :

| CRL 40982 FORME III | | |
|---|---|---|
| 2 Theta (degrés) | d (Å) | I/Io (%) |
| 9,8 | 13,40 | 40 |
| 10,7 | 12,28 | 39 |
| 15,4 | 8,54 | 100 |
| 18,0 | 7,32 | 33 |
| 21,4 | 6,17 | 23 |
| 25,9 | 5,11 | 26 |
| 26,4 | 5,01 | 87 |
| 29,6 | 4,48 | 26 |
| 29,9 | 4,44 | 20 |
| 31,1 | 4,27 | 34 |
| 31,7 | 4,19 | 20 |
| 32,4 | 4,10 | 77 |
| 33,1 | 4,02 | 23 |
| 33,5 | 3,97 | 64 |
| 36,5 | 3,66 | 38 |
| 39,1 | 3,42 | 40 |
| 41,9 | 3,20 | 32 |
| 46,4 | 2,91 | 23 |
| 52,7 | 2,58 | 25 |
| Diffractomètre: Miniflex Rigaku (Elexience) | | |

L'invention vise également la forme polymorphique de l'énantiomère lévogyre du modafinil désignée CRL 40982 forme IV, caractérisée en ce qu'elle produit un spectre de diffraction X comprenant des raies d'intensité aux distances réticulaires : 12,38; 8,58; 7,34; 6,16; 5,00; 4,48; 4,09; 3,66 Å, les raies les plus caractéristiques correspondant aux distances réticulaires de 12,38; 8,58; 7,34; 5,00; 4,09 Å.
Plus précisément, la forme IV du (-)-modafinil se caractérise en ce qu'elle produit le spectre de diffraction X suivant, dans lequel d représente la distance réticulaire et I/Io l'intensité relative comprenant des raies d'intensité aux distances réticulaires :

| CRL 40982 FORME IV | | |
|---|---|---|
| 2 Theta (degrés) | d (Å) | I/Io (%) |
| 6,37 | 13,88 | 26 |
| 7,14 | 12,38 | 69 |
| 8,60 | 10,27 | 23 |
| 10,30 | 8,58 | 100 |
| 12,04 | 7,34 | 49 |
| 14,37 | 6,16 | 24 |
| 15,65 | 5,66 | 11 |
| 17,30 | 5,12 | 29 |
| 17,72 | 5,00 | 60 |
| 19,12 | 4,64 | 15 |
| 19,81 | 4,48 | 25 |
| 20,82 | 4,26 | 10 |
| 21,24 | 4,18 | 12 |
| 21,70 | 4,09 | 51 |
| 23,28 | 3,82 | 9 |
| 24,30 | 3,66 | 30 |
| 25,18 | 3,53 | 9 |
| 26,02 | 3,42 | 21 |
| 27,13 | 3,28 | 9 |
| 27,90 | 3,20 | 15 |
| Diffractomètre : Siemens AG. | | |

L'invention vise également la forme polymorphique de l'énantiomère dextrogyre du modafinil désignée CRL 40983 forme V, caractérisée en ce qu'elle produit un spectre de diffraction X comprenant des raies d'intensité aux distances réticulaires : 9,63 ; 5,23; 5,03 ; 4,74 ; 4,66 ; 4,22 ; 4,10 ; 3,77 (Å).

| CRL 40983 FORME V | | |
|---|---|---|
| 2 Theta (degrés) | d (Å) | I/Io (%) |
| 6,65 | 13,27 | 22 |
| 7,24 | 12,21 | 5 |
| 9,17 | 9,63 | 51 |
| 10,38 | 8,51 | 19 |
| 12,28 | 7,20 | 15 |
| 14,33 | 6,17 | 14 |
| 15,81 | 5,60 | 4 |
| 16,95 | 5,23 | 68 |
| 17,64 | 5,03 | 100 |
| 18,69 | 4,74 | 51 |

(suite)

| CRL 40983 FORME V | | |
|---|---|---|
| 2 Theta (degrés) | d (Å) | I/Io (%) |
| 19,03 | 4,66 | 58 |
| 20,06 | 4,42 | 3 |
| 21,06 | 4,22 | 91 |
| 21,67 | 4,10 | 64 |
| 22,39 | 3,97 | 17 |
| 23,61 | 3,77 | 55 |
| 24,64 | 3,61 | 8 |
| 25,40 | 3,50 | 13 |
| 26,21 | 3,40 | 20 |
| 26,95 | 3,31 | 18 |
| Diffractomètre : Bruker GADDS | | |

L'invention a également pour objet le solvate de diméthylcarbonate du (-)-modafinil, caractérisé par le spectre de diffraction suivant dans lequel d représente la distance réticulaire et I/Io l'intensité relative :

| SOLVATE DE DIMÉTHYLCARBONATE | | |
|---|---|---|
| 2 Theta (degrés) | d (Å) | I/Io (%) |
| 7,17 | 12,31 | 38 |
| 9,12 | 9,69 | 29 |
| 9,72 | 9,09 | 16 |
| 10,35 | 8,54 | 35 |
| 12,17 | 7,27 | 100 |
| 14,25 | 6,21 | 16 |
| 16,26 | 5,45 | 10 |
| 17,36 | 5,10 | 13 |
| 17,72 | 5,00 | 21 |
| 18,35 | 4,83 | 9 |
| 19,16 | 4,63 | 9 |
| 19,88 | 4,46 | 14 |
| 21,04 | 4,22 | 12 |
| 21,49 | 4,13 | 25 |
| 21,73 | 4,09 | 24 |
| 23,49 | 3,78 | 22 |
| 24,55 | 3,62 | 35 |
| 25,24 | 3,53 | 8 |
| 26,05 | 3,42 | 9 |
| 26,88 | 3,32 | 7 |
| 27,48 | 3,24 | 13 |

(suite)

| SOLVATE DE DIMÉTHYLCARBONATE | | |
|---|---|---|
| 2 Theta (degrés) | d (Å) | I/Io (%) |
| 27,81 | 3,21 | 10 |
| 28,79 | 3,10 | 8 |
| Diffractomètre : Siemens AG. | | |

L'invention a également pour objet le solvate d'acide acétique des énantiomères lévogyre et dextrogyre du modafinil susceptible d'être obtenu selon le procédé de recristallisation de l'invention, caractérisée en ce qu'il produit un spectre de diffraction X comprenant des raies d'intensité aux distances réticulaires : 9,45 ; 7,15 ; 5,13 ; 4,15 ; 3,67 (Å).

| SOLVATE D'ACIDE ACÉTIQUE | | |
|---|---|---|
| 2-Theta (degrés) | d (Å) | I/Io % |
| 6,64 | 13,30 | 8,5 |
| 7,15 | 12,35 | 15 |
| 9,36 | 9,45 | 100 |
| 10,43 | 8,48 | 6,5 |
| 12,38 | 7,15 | 25 |
| 14,38 | 6,16 | 15 |
| 16,37 | 5,41 | 8 |
| 17,29 | 5,13 | 28 |
| 17,82 | 4,97 | 21 |
| 18,24 | 4,86 | 16 |
| 18,96 | 4,68 | 7 |
| 19,24 | 4,61 | 6 |
| 20,09 | 4,42 | 20 |
| 21,40 | 4,15 | 75 |
| 22,55 | 3,94 | 21 |
| 23,42 | 3,80 | 7 |
| 24,25 | 3,67 | 40 |
| 24,92 | 3,57 | 12 |
| 25,21 | 3,53 | 9,5 |
| 26,15 | 3,40 | 11 |
| 26,78 | 3,33 | 8 |
| 26,99 | 3,30 | 6 |
| 28,43 | 3,14 | 13 |
| 28,79 | 3,10 | 14 |
| 29,63 | 3,01 | 7 |
| 30,03 | 2,97 | 4 |
| 32,33 | 2,77 | 9 |
| 33,13 | 2,70 | 7 |

(suite)

| SOLVATE D'ACIDE ACÉTIQUE | | |
|---|---|---|
| 2-Theta (degrés) | d (Å) | I/Io % |
| 34,29 | 2,61 | 3 |
| 34,86 | 2,57 | 7 |
| 35,90 | 2,50 | 7 |
| Diffractomètre : Bruker GADDS | | |

Selon un autre aspect, l'invention a également pour objet un procédé de conversion d'une première forme cristalline d'un des énantiomères du modafinil en une deuxième forme cristalline distincte de la première, ledit procédé comprenant les étapes consistant à :

i) suspendre la forme cristalline dudit énantiomère du modafinil dans un solvant ;
ii) récupérer la forme cristalline obtenue.

A titre de solvants pouvant convenir à ce procédé, on peut notamment citer l'acétonitrile.

De façon générale, la forme cristalline initiale est maintenue en suspension à une température inférieure à la température d'homogénéisation, pendant une durée suffisante pour permettre la conversion totale de la forme initiale. Cette durée peut varier notamment selon la nature du solvant, de la forme cristalline initiale, de la température du milieu. De façon classique, la forme cristalline est maintenue en suspension pendant au moins 24 heures à température ambiante, sous pression atmosphérique, le plus souvent pendant 72 heures environ.

A titre illustratif, ce procédé est mis en oeuvre avec le (-)-modafinil.

Dans ce cadre, selon un mode de réalisation particulier de l'invention, le procédé met en oeuvre la forme I dans l'acétonitrile à l'étape i), ce par quoi on obtient un solvate d'acétonitrile du (-)-modafinil.

A titre indicatif, la forme I est maintenue en suspension pendant plusieurs jours, de préférence pendant 3 jours à température ambiante, à pression atmosphérique.

L'invention a également pour objet le solvate d'acétonitrile du (-)-modafinil susceptible d'être obtenu selon le procédé de recristallisation de l'invention. Il est caractérisé par le spectre de diffraction suivant dans lequel d représente la distance réticulaire et I/Io l'intensité relative :

| SOLVATE D'ACETONITRILE | | |
|---|---|---|
| 2 Theta (degrés) | d (Å) | I/Io (%) |
| 5,46 | 16,17 | 46 |
| 6,25 | 14,14 | 95 |
| 7,17 | 12,32 | 51 |
| 8,28 | 10,66 | 81 |
| 9,02 | 9,79 | 68 |
| 9,51 | 9,29 | 53 |
| 10,34 | 8,54 | 53 |
| 10,84 | 8,15 | 63 |
| 11,33 | 7,80 | 79 |
| 12,47 | 7,09 | 53 |
| 14,02 | 6,31 | 45 |
| 15,20 | 5,83 | 35 |
| 15,76 | 5,62 | 34 |
| 16,37 | 5,41 | 40 |
| 17,37 | 5,10 | 51 |

(suite)

| SOLVATE D'ACETONITRILE | | |
|---|---|---|
| 2 Theta (degrés) | d (Å) | I/Io (%) |
| 18,10 | 4,90 | 46 |
| 19,05 | 4,66 | 44 |
| 19,36 | 4,58 | 37 |
| 19,89 | 4,46 | 39 |
| 20,48 | 4,33 | 59 |
| 21,14 | 4,20 | 55 |
| 22,10 | 4,02 | 100 |
| 22,65 | 3,92 | 60 |
| 23,17 | 3,835 | 42 |
| 23,89 | 3,72 | 33 |
| 24,72 | 3,60 | 38 |
| 24,93 | 3,57 | 37 |
| 25,81 | 3,45 | 37 |
| 26,73 | 3,33 | 55 |
| 27,52 | 3,24 | 30 |
| 27,97 | 3,19 | 30 |
| 28,89 | 3,09 | 31 |
| 29,44 | 3,03 | 27 |
| Diffractomètre : Siemens AG. | | |

**• COMPOSITIONS PHARMACEUTIQUES COMPRENANT LES FORMES POLYMORPHIQUES II, III, IV et V DU (-)-MODAFINIL, DU (+)-MODAFINIL RESPECTIVEMENT**

L'invention vise également les compositions pharmaceutiques comprenant les formes polymorphiques CRL 40982 forme II, CRL 40982 forme III, CRL 40982 forme IV ou CRL 40982 forme V du (-)-modafinil, CRL 40983 forme II, CRL 40983 forme III, CRL 40983 forme IV et CRL 40983 forme V, respectivement, éventuellement en association avec un véhicule pharmaceutiquement acceptable.

Ces compositions peuvent être administrées par voie orale, par voie muqueuse (par exemple, oculaire, intranasale, pulmonaire, gastrique, intestinale, rectale, vaginale, ou bien via l'appareil urinaire) ou par voie parentérale (par exemple, sous-cutanée, intradermique, intramusculaire, intraveineuse, ou intrapéritonéale).

Selon un mode préférentiel, les compositions pharmaceutiques selon l'invention sont administrées par voie orale, sous forme de comprimés, de pilules, de gélules ou de granules à libération immédiate ou à libération contrôlée, sous forme de poudre, de capsules, de suspension dans un liquide ou dans un gel, d'émulsion, ou bien de lyophilisat, plus préférentiellement sous forme de comprimés, de capsules, de suspension dans un liquide ou dans un gel. Le véhicule d'administration peut comprendre un ou plusieurs excipients pharmaceutiquement acceptables qui sont susceptibles d'assurer la stabilité des formes polymorphiques (par exemple, une suspension d'un polymorphe dans une huile).

Les compositions pharmaceutiques selon l'invention comprennent les formes polymorphiques du (-)-modafinil et du (+)-modafinil, II, III, IV ou V, respectivement, éventuellement en mélange les unes avec les autres et/ou avec un ou plusieurs excipients pharmaceutiquement acceptables.

Une composition solide pour une administration par voie orale est préparée par addition au principe actif d'un ou de plusieurs excipients, notamment d'une charge, et, le cas échéant d'un liant, d'un agent délitant, d'un lubrifiant, d'un surfactant et d'un émulsifiant, d'un solubilisant, d'un colorant, d'un succédané de sucre ou d'un correcteur de goût et par mise en forme du mélange par exemple sous forme de comprimé ou de capsule.

Des exemples de charges englobent le lactose, le sucrose, le mannitol ou le sorbitol ; des préparations à base de cellulose, telles que par exemple de l'amidon de maïs, de l'amidon de riz, de l'amidon de pomme de terre.

Des exemples de liants englobent la gélatine, la gomme adragante, la méthylcellulose, l'hydroxypropylcellulose, la carboxyméthylcellulose de sodium, et/ou la polyvinyle pyrrolidone (PVP), la povidone, la copovidone, le dextrane, la dextrine, la cyclodextrine et ses dérivés tels que l'hydroxypropyl-β-cyclodextrine.

Des exemples de succédanés de sucre englobent l'aspartame, la saccharine, le cyclamate de sodium.

Des exemples d'agents correcteurs de goût englobent le cacao en poudre, la menthe sous forme d'herbe, la poudre aromatique, la menthe sous forme d'huile, le bornéol et la cannelle en poudre.

Des exemples de surfactants et d'émulsifiants englobent en particulier le polysorbate 20, 60, 80, le sucroester (7-11-15), le poloxamère 188, 407, le PEF 300, 400 et le stéarate de sorbitane.

Des exemples d'agents solubilisants englobent le miglyole 810, 812, les glycérides et leurs dérivés, le propylène glycol.

Des exemples d'agents délitants englobent, par exemple, la polyvinyle pyrrolidone, la carmelose de sodium ou bien l'acide alginique ou un sel de celui-ci tel que l'alginate de sodium.

Des exemples d'agents lubrifiants englobent le stéarate de magnésium, le fumarate de magnésium stéarilé, l'acide béhénique, et ses dérivés.

Les compositions pharmaceutiques de la présente invention peuvent également contenir une autre forme cristalline du (-)-modafinil ou du (+)-modafinil, respectivement, dont notamment la forme I et/ou un autre principe actif ou inactif en mélange avec un ou plusieurs autres formes polymorphiques du modafinil, telles que la forme III, la forme II, la forme IV et la forme V.

Au sens de la présente invention, l'expression "véhicule pharmaceutiquement acceptable" couvre des solvants, des milieux de dispersion, des agents antifongiques et antibactériens, des agents isotoniques et des agents retardant l'absorption. L'utilisation de tels milieux et agents pour les substances pharmaceutiquement actives est bien connue de l'homme du métier.

L'invention vise également l'utilisation des formes CRL 40982 forme II, CRL 40982 forme III, CRL 40982 forme IV ou CRL 40982 forme V du (-)-modafinil CRL 40983 forme II, CRL 40983 forme III, CRL 40983 forme IV ou CRL 40983 forme V du (+)-modafinil, respectivement, pour la fabrication d'un médicament destiné à la prévention et/ou au traitement d'une affection choisie parmi l'hypersomnie, dont notamment l'hypersomnie idiopathique et l'hypersomnie chez les patients atteints d'un cancer traités par des analgésiques morphiniques pour soulager la douleur ; les apnées du sommeil, la somnolence excessive associée à une maladie, les apnées du sommeil obstructives, la narcolepsie ; les somnolences, les somnolences excessives, les somnolences excessives liées à la narcolepsie ; les troubles du système nerveux central tels que la maladie de Parkinson ; la protection du tissu cérébral vis-à-vis de l'ischémie, les troubles de la vigilance, dont notamment les troubles de la vigilance liés à la maladie de Steinert, les troubles de l'attention, par exemple liés à l'hyperactivité (ADHD) ; l'état de fatigue, en particulier celui lié à des scléroses multiples et autres maladies dégénératives ; la dépression, l'état dépressif lié au faible ensoleillement, la schizophrénie, le travail par roulement, le décalage horaire ; les troubles du comportement alimentaire, dans lequel le modafinil agit en tant que stimulateur d'appétit, la stimulation des fonctions cognitives à faibles doses.

## • PROCÉDÉ DE PRÉPARATION DU MODAFINIL OPTIQUEMENT ACTIF

Selon un autre aspect, l'invention vise un procédé de préparation des énantiomères optiques du modafinil à partir de l'acide (±) modafinique, ledit procédé comprenant les étapes suivantes :

i) réaliser un dédoublement des deux énantiomères optiques de l'acide (±) modafinique et récupérer au moins un des énantiomères ;
ii) mettre en contact l'un des deux énantiomères obtenus avec un haloformiate d'alkyle inférieur et d'un alcool en présence d'une base ;
iii) récupérer le produit obtenu ;
iv) convertir l'ester obtenu à l'étape iii) en amide ;
v) récupérer le produit obtenu à l'étape iv).

De façon préférentielle, l'haloformiate d'alkyle inférieur est un chloroformiate d'alkyle inférieur et, mieux encore, il s'agit du chloroformiate de méthyle.

Avantageusement, les haloformiates d'alkyle inférieurs, dont notamment le chloroformiate de méthyle, mis en oeuvre dans ce procédé pour réaliser l'estérification de l'acide modafinique, sont moins toxiques que le diméthylsulfate décrit dans le procédé de l'art antérieur US 4,927,855 pour des rendements équivalents, voire améliorés. Le procédé est donc plus facile à mettre en oeuvre et plus adapté à une application industrielle.

De préférence, on opère en présence d'une quantité équimolaire d'haloformiate d'alkyle inférieur et de base

à l'étape ii) par rapport à l'acide modafinique optiquement actif.

On préfère particulièrement mettre en oeuvre des bases organiques, plus préférentiellement des bases azotées.

A titre de base particulièrement préférée, on peut citer notamment la triéthylamine, la diisopropylamine, la diéthylméthylamine, la diisopropyléthyl amine, la 1,8-diazabicyclo[5.4.0]undec-7-ène (DBU).

De préférence, le solvant mis en oeuvre à l'étape ii) est un alcool aliphatique inférieur tel que le méthanol, l'éthanol, le propanol, le méthanol étant particulièrement préféré.

Selon un mode de réalisation particulier, l'ester obtenu à l'issue de l'étape ii) est cristallisé par addition d'eau glacée.

La conversion de l'ester en amide à l'étape iv) consiste de préférence en une ammonolyse, c'est-à-dire un traitement avec de l'ammoniaque.

Dans ce cadre, on préfère généralement opérer avec un excès d'ammoniaque.

Selon une variante privilégiée de l'invention, l'ammoniaque mis en oeuvre est sous forme gazeuse.

La réaction d'ammonolyse est, selon un mode préférentiel, réalisé dans un solvant polaire, de préférence protique tel que les alcools aliphatiques inférieurs, par exemple dans le méthanol ou l'éthanol, le méthanol étant particulièrement préféré.

Les récupérations de l'ester de l'acide (+) ou (-)modafinique à l'étape iii) et respectivement du (+) ou (-)modafinil à l'étape iv) sont réalisées selon des méthodes conventionnelles connues de l'homme du métier.

Selon un autre aspect, l'invention a pour objet un procédé de préparation des énantiomères optiques du modafinil comprenant les étapes suivantes :

a. réaliser un dédoublement des deux énantiomères optiques de l'acide (±) modafinique ou des sels de celui-ci selon un procédé de cristallisation préférentielle ;
b. convertir ledit énantiomère isolé en amide ;
c. récupérer l'énantiomère du modafinil obtenu.

Selon un mode de réalisation préféré, l'étape b) est réalisée en deux temps :

b1) convertir ledit énantiomère en ester d'alkyle inférieur ;
b2) convertir le produit obtenu à l'étape b1) en amide.

Selon un mode de réalisation particulièrement préféré, l'étape b1) est réalisée en présence d'haloformiate d'alkyle inférieur, d'un alcool et d'une base, selon les conditions décrites précédemment.

Selon un mode particulièrement avantageux, lorsque b1) est réalisé en présence de chloroformiate de méthyle, d'une base et d'un alcool, et c1) consiste en une ammonolyse telle que décrite précédemment, ce procédé, dans lequel l'acide (±)modafinique est dédoublé par cristallisation préférentielle, se traduit par un rendement global généralement de l'ordre de 25%. Ainsi, le rendement d'obtention par ce procédé de l'énantiomère (-)-modafinique notamment est nettement supérieur à celui obtenu dans le brevet US 4,927,855.

La technique de cristallisation préférentielle est une technique largement utilisée dans les laboratoires et dans l'industrie.

Cette méthode repose sur la cristallisation alternée de deux composés chiraux dénommés R et S, réalisant un conglomérat dans un solvant A et pour une gamme donnée de température $D_T$. Ceci signifie que, dans cette gamme de température, tout mélange, en équilibre thermodynamique des deux antipodes avec la solution, est constitué de deux types de cristaux ne contenant chacun que des molécules de même configuration, incorporant ou non des molécules de solvant (solvates). L'existence d'un tel conglomérat, sans miscibilité à l'état solide, sera implicitement admise dans ce qui suit, au moins dans l'intervalle de température $D_T$ et pour le solvant A.

Deux classes de facteurs influencent la cristallisation des antipodes optiques, d'une part, les paramètres liés aux équilibres hétérogènes ternaires et, d'autre part, les facteurs intervenant sur la cinétique de cristallisation.

Les paramètres liés aux équilibres hétérogènes ternaires comprennent :

- les positions des nappes de cristallisation des espèces solides qui se déposent à chaque température et, plus particulièrement, les valeurs des solubilités des phases stables et métastables, du mélange racémique s(±) et des antipodes s(+) = s(-), en fonction de la température, et le rapport des solubilités $\alpha$ = s (±) / s(+) ;
- l'étendue des domaines stables et métastables de solutions solides, de racémate, de solvate racémique, de solvates actifs et des variétés polymorphiques des solides cristallisés.

Les facteurs intervenant sur la cinétique de cristallisation comprennent :

- des facteurs internes aux cristaux, en relation avec les liaisons entre les molécules, qui sont non modifiables par

l'expérimentateur ;

- des facteurs externes qui sont modifiables par l'expérimentateur ; il s'agit de la nature du solvant, de la nature et de la concentration des impuretés, de la sursaturation acquise en fonction du temps, de l'intervalle de température $D_T$, de la vitesse et du mode d'agitation, de la masse et de la granulométrie des germes, de l'effet de paroi, etc.

Ces deux classes de facteurs influencent directement le rendement, la pureté des phases obtenues et le déroulement des opérations de séparation. La faisabilité de la filtration sera ainsi dépendante du spectre granulométrique et de l'habitus des cristaux, de la viscosité de la suspension, de la tension de vapeur du solvant, des sursaturations acquises par chacun des antipodes et de la présence éventuelle d'un racémate vrai à caractère métastable. Ces choix pourront également intervenir sur la cinétique de racémisation des antipodes ou de dégradation de la molécule.
Pour chaque ensemble constitué du couple d'antipodes (R et S) et du solvant (A), les facteurs intervenant sur la cinétique sont un cas d'espèce.
On distingue principalement deux types de méthodes de cristallisation préférentielle :

- les procédés classiques désignés SIPC pour "Seeded Isothermal Preferential Crystallization" et leurs variantes polythermiques, d'une part ;
- le procédé désigné AS3PC pour "Auto-Seeded Polythermic Programmed Preferential Crystallization", d'autre part.

Dans la méthode de cristallisation préférentielle AS3PC dite auto-ensemencée, le système est mis dans des conditions telles qu'il génère lui-même ses germes pour la réalisation de l'énantiomère recherché, tandis que dans la méthode SIPC ces germes sont introduits par ensemencement. Les deux types de procédés seront décrits de façon plus détaillée ci-après.
Pour plus d'informations concernant les procédés de dédoublement par cristallisation préférentielle selon les méthodes AS3PC, on pourra se référer notamment aux documents de G. Coquerel, M.-N. Petit et R. Bouaziz, Brevet EP 0720595 B1, 1996 ; E. Ndzié, P. Cardinaël, A.-R. Schoofs et G. Coquerel, Tetrahedron Asymmetry, 1997, 8(17), 2913-2920 ; L. Courvoisier, E. Ndzié, M.-N. Petit, U. Hedtmann, U. Sprengard et G. Coquerel, Chemistry Letters, 2001, 4, 364-365.
Selon un mode de réalisation particulier, le procédé de dédoublement des énantiomères optiques de l'acide ($\pm$) modafinique ou des sels de celui-ci est un procédé ensemencé SIPC ou S3PC, ledit procédé comprenant les étapes suivantes :

a) homogénéiser à une température $T_D$ un ensemble composé du mélange racémique de cristaux sous forme de conglomérat, du premier énantiomère de l'acide modafinique et de solvant, dont le point figuratif E, défini par les variables concentration et température $T_D$, se trouve dans le domaine monophasé de la solution diluée ;
b) refroidir rapidement la solution préparée à l'étape a) initialement à la température $T_D$ jusqu'à la température $T_F$ ;
c) ensemencer la solution obtenue à l'étape b) pendant (i. e. entre $T_L$ et $T_F$) ou en fin de refroidissement (i. e. à $T_F$) en germes très purs du premier énantiomère ;
d) récolter les cristaux du premier énantiomère ;
e) additionner aux liqueurs-mères résultant de la récolte réalisée à l'étape d) le mélange racémique de cristaux sous forme de conglomérat, et homogénéiser le nouvel ensemble en chauffant à une température $T_D$, de sorte que le point figuratif E' soit symétrique de E par rapport au plan de mélange racémique du système solvant, antipode (-), antipode (+), ledit point E' se situant dans le domaine monophasé de la solution diluée ;
f) refroidir rapidement la solution obtenue à l'étape e) initialement à la température $T_D$, jusqu'à la température $T_F$ ;
g) ensemencer la solution obtenue à l'étape f) en germes très purs du second énantiomère ;
h) récolter les cristaux du second énantiomère ;
i) additionner aux liqueurs-mères résultant de la récolte cristalline réalisée à l'étape h) le mélange racémique sous forme de conglomérat de cristaux et homogénéiser le nouvel ensemble en chauffant à une température $T_D$ pour obtenir une composition identique à celle de l'ensemble du point figuratif E initial ;
j) répéter les étapes a), b), c), d), e), f), h) et j) pour obtenir successivement le premier puis le second des deux énantiomères.

On se réfère souvent à ces deux méthodes en les désignant respectivement "SIPC" et "S3PC", laquelle est une variante de SIPC, comme décrit de manière détaillée dans la suite de la description.
Dans ce qui suit, au sens de la présente invention, sont désignés

- par $T_F$ la température de fin de cristallisation et filtration, située dans le domaine triphasé ;
- par $T_L$ la température d'homogénéisation du mélange racémique ;
- par $T_D$ la température de départ pour laquelle le mélange de départ est une solution homogène ;

- par antipode, un énantiomère.

A titre préférentiel, le procédé de dédoublement des deux énantiomères optiques de l'acide (±) modafinique ou des sels de ceux-ci par cristallisation préférentielle est un procédé auto-ensemencé AS3PC, ledit procédé comprenant les étapes suivantes :

a) réaliser un ensemble composé du mélange racémique de cristaux sous forme de conglomérat, du premier énantiomère de l'acide modafinique et de solvant, dont le point figuratif E, défini par les variables concentration et température $T_B$, se situe dans le domaine biphasé de l'énantiomère en excès, et est en équilibre avec sa solution saturée ;
b) appliquer une loi de programmation du refroidissement de la température au mélange biphasé préparé à l'étape a), ladite loi de programmation étant telle que les liqueurs-mères gardent une faible sursaturation qui privilégie la croissance de l'énantiomère présent sous forme de cristaux, tout en interdisant la nucléation spontanée du second énantiomère présent dans la solution ;
c) adapter pendant toute la durée de la croissance cristalline de l'étape b) une vitesse d'agitation légèrement croissante en fonction du temps, de façon à ce que celle-ci soit à tout moment suffisamment lente pour favoriser une croissance du premier énantiomère en évitant de générer des forces de striction trop importantes provoquant une nucléation non maîtrisée et suffisamment rapide pour réaliser une suspension homogène et un renouvellement rapide de la liqueur-mère autour de chaque cristallite du premier énantiomère ;
d) récolter les cristaux du premier énantiomère ;
e) additionner aux liqueurs-mères résultant de la récolte réalisée à l'étape d) le mélange racémique de cristaux sous forme de conglomérat, et porter le nouvel ensemble à un palier de température $T_B$ pendant la durée nécessaire à l'obtention de l'équilibre thermodynamique, de sorte que le point figuratif E' soit symétrique de E par rapport au plan des mélanges racémiques du système solvant, antipode (-), antipode (+), ledit point E' se situant dans le domaine biphasé du second énantiomère en excès et en équilibre avec sa solution saturée ;
f) appliquer la même loi de programmation du refroidissement qu'à l'étape b) au mélange biphasé préparé à l'étape e) contenant le second énantiomère, de sorte que les liqueurs-mères gardent une faible sursaturation pendant la cristallisation afin de privilégier la croissance de l'énantiomère présent sous forme de cristaux tout en interdisant la nucléation spontanée du premier énantiomère présent dans la solution ;
g) adapter, pendant toute la durée de la croissance cristalline de l'étape f), une vitesse d'agitation légèrement croissante en fonction du temps, de façon à ce que celle-ci soit, à tout moment, suffisamment lente pour favoriser la croissance du second énantiomère en évitant de générer des forces de striction trop importantes provoquant une nucléation non maîtrisée, et suffisamment rapide pour obtenir une suspension homogène et un renouvellement rapide de la liqueur-mère autour de chaque cristallite du second énantiomère ;
h) récolter les cristaux du second énantiomère ;
i) additionner aux liqueurs-mères résultant de la récolte cristalline réalisée à l'étape g) le mélange racémique de cristaux sous forme de conglomérat pour obtenir un ensemble dont la composition est identique à celle de l'ensemble E initial;
j) répéter les étapes a), b), c), d), e), f) g), h) et i) pour obtenir successivement le premier puis le second des deux énantiomères.

Dans ce qui suit, au sens de la présente invention, est désignée par $T_{HOMO}$ la température d'homogénéisation de l'ensemble constitué du mélange racémique, du premier énantiomère et du solvant.

Ainsi, à l'étape (a) du procédé de l'invention, le choix du ou des solvants et de la gamme de température de travail sont définis de façon à avoir simultanément :

- des antipodes qui réalisent un conglomérat et dont l'éventuel racémate est métastable dans la gamme de température de travail ;
- des liqueurs suffisamment concentrées mais de faibles viscosité et de faible tension de vapeur ;
- une absence de solvolyse et de racémisation ;
- une stabilité des solvates si ceux-ci sont présents à l'équilibre et s'il s'agit d'énantiomères dédoublables.

Aux étapes (a) et (e) du procédé de l'invention, la température $T_B$ est supérieure à la température $T_L$ d'homogénéisation de la quantité de mélange racémique contenue dans la suspension initiale, et en ce que, à partir de la courbe de variation de $T_{HOMO}$ en fonction de l'excès énantiomérique et pour une concentration constante en mélange racémique $X_L$, ladite température $T_B$ est définie de façon à ce que la masse de fins cristaux du premier énantiomère des étapes (a) et (i) et du second énantiomère de l'étape (e), en équilibre avec leur solution saturée, représente au maximum 50% et de préférence entre environ 25% et 40% de la récolte attendue.

Aux étapes (b) et (f) du procédé de l'invention, la loi de programmation du refroidissement de la température $T_B$ à $T_F$, adaptée au montage expérimental, est définie de façon :

- à obtenir une faible sursaturation pendant toute la durée de la cristallisation de l'énantiomère présent sous forme de cristaux au début de chaque cycle, cette faible sursaturation provoquant une croissance et une nucléation secondaires douces ;
- à atteindre à $T_F$ le maximum de sursaturation de l'autre énantiomère sans nucléation primaire ;
- à obtenir une récolte en cristaux aux étapes (d) et (h) qui, après addition de mélange racémique et compensation aux étapes (e) et (i), permet la cyclicité des opérations.

En effet, chaque montage expérimental influe sur les capacités de sursaturation des mélanges utilisés et l'efficacité de l'agitation, et, en conséquence, la loi de programmation du refroidissement est adaptée aux circonstances dans lesquelles le procédé est mis en oeuvre. Pour autant, la température $T_B$, les solubilités du mélange racémique en fonction de la température, la courbe $T_{HOMO}$ en fonction de l'excès énantiomérique pour une concentration constante en mélange racémique $X_L$, sont, elles, totalement indépendantes du montage expérimental.

La loi de programmation du refroidissement, qui est la fonction reliant la température au temps, est déterminée pour sa partie de $T_L$ à $T_F$ par refroidissement de la solution de concentration $X_L$ de $T_L$ + 1 °C à $T_F$, $T_F$ étant inférieur à $T_L$ - ($T_{HOMO}$ - $T_L$), afin d'obtenir une solution saturée stable sans nucléation primaire tout en permettant une récolte double de l'excès énantiomérique initial, et en ce que ladite loi de programmation du refroidissement est déterminée pour sa partie de $T_B$ à $T_L$ par extrapolation de cette même loi déterminée de $T_L$ + 1 °C à $T_F$.

Le procédé de cristallisation préférentielle de l'acide (±)-modafinique ou des sels de celui-ci présente d'autres caractéristiques avantageuses, seules ou en combinaison, telles que :

- aux étapes (a) et (i), la masse de fins cristaux du premier énantiomère en équilibre avec sa solution saturée représente entre environ 25% et 40% de la récolte attendue, 50% représentant une limite maximale ;
- à l'étape e), la masse de fins cristaux du second énantiomère en équilibre avec sa solution saturée représente entre environ 25% et 40% de la récolte attendue, 50% représentant une limite maximale ;
- aux étapes (b) et (f), la thermicité accompagnant le dépôt du premier énantiomère et du second énantiomère est intégrée dans la loi de programmation en température ;
- aux étapes (e) et (i), on effectue des compensations en solvant ;
- aux étapes (a), (e) et (i), les fins cristaux du mélange racémique sous forme de conglomérat qui sont ajoutés ont subi, avant d'être introduits, un traitement préalable accélérant l'étape de dissolution, tel qu'un broyage et un tamisage, un traitement par des ondes ultra-sonores, une lyophilisation partielle ; ces traitements ayant également pour but de fournir de fins cristaux propres à générer une surface de croissance cristallines élevée ;
- aux étapes (a), (e) et (i), comportant une dissolution, la vitesse d'agitation est élevée par rapport aux étapes (c) et (g).

Outre les données des équilibres hétérogènes requises pour mettre en oeuvre le procédé AS3PC, les opérations restent également soumises à des contraintes cinétiques ajustables, notamment la loi de refroidissement, qui sont, pour chaque ensemble solvant / énantiomères, un cas d'espèce.

Selon un mode de réalisation, le solvant mis en oeuvre à l'étape a) des procédés SIPC, S3PC ou AS3PC est l'éthanol absolu ou dénaturé, éventuellement en mélange avec une base organique ou minérale, voire avec un ou plusieurs solvant(s), susceptibles d'améliorer la solubilité du mélange racémique dans l'éthanol.

En variante, le solvant mis en oeuvre à l'étape a) des procédés SIPC, S3PC ou AS3PC est le 2-méthoxyéthanol ou le méthanol, éventuellement en mélange avec une base organique ou minérale, et/ou un ou plusieurs solvant(s), susceptibles d'améliorer la solubilité du mélange racémique dans l'éthanol.

Selon un mode de réalisation privilégié, le solvant mis en oeuvre à l'étape a) du procédé SIPC ou AS3PC est l'éthanol, le 2-méthoxyéthanol ou le méthanol. La température de filtration $T_F$ est de préférence comprise entre et 40°C pour l'acide (±)-modafinique.

Dans le cas de l'éthanol, la température $T_F$ est de préférence comprise entre et 25°C, et mieux encore elle est proche de 18°C ou 17°C.

Dans le cas du 2-méthoxyéthanol ou du méthanol, la température $T_F$ est de préférence comprise entre 20°C et 35°C et notamment proche de 30°C.

De préférence, la concentration du mélange racémique à l'étape a) est alors comprise entre 2 et 50 % massique, plus préférentiellement entre 2 et 30 % massique, et, mieux encore, proche de 5,96% massique dans le cas de l'éthanol, 15,99 % dans le cas du 2-méthoxyéthanol et de 25,70 % dans le cas du méthanol.

Dans ce cadre, on préfère tout particulièrement que l'excès énantiomérique à l'étape a) soit compris entre 1 et 50 % massique, plus préférentiellement entre 1 et 20 % massique, et, mieux encore, proche de 11 % massique dans le cas de l'éthanol, 8 % massique dans le cas du 2-méthoxyéthanol et 10 % massique dans le cas du méthanol.

La température $T_D$ des procédés SIPC et S3PC, température pour laquelle le mélange de départ est une solution homogène, dépend de la concentration et est alors généralement comprise entre 35° et 50°C au reflux du solvant. Le refroidissement de la température $T_D$ à $T_F$ est très rapide de manière à rester dans le domaine monophasé et réalisé de préférence en moins de 20 mn, par exemple par trempe.

Selon un mode préférentiel du procédé AS3PC, la température $T_B$ est alors comprise entre les températures $T_L$ et $T_{HOMO}$. La température $T_B$ peut être notamment comprise entre 25°C et 50°C.

A titre d'exemple, dans le cas de l'éthanol, lorsque l'excès énantiomérique est proche de 11 % massique, la température $T_B$ est de préférence comprise entre 25°C et 40°C, notamment entre 30,1 °C et 36,2°C et plus préférentiellement proche de 33,5°C ou bien de 31,5°C.

Dans le cas du 2-méthoxyéthanol, lorsque l'excès énantiomérique est proche de 8% massique, la température $T_B$ est de préférence comprise entre 35°C et 50°C, notamment entre 39,1 °C et 47,9°C et plus préférentiellement proche de 41 °C.

Dans le cas du méthanol, lorsque l'excès énantiomérique est proche de 10% massique, la température $T_B$ est de préférence comprise entre 40°C et 55°C, notamment entre 45,1 °C et 53,9 °C et plus préférentiellement proche de 46,5°C.

On préfère tout particulièrement que le refroidissement de $T_B$ à $T_F$ à l'étape b) soit réalisé en un temps suffisamment long pour que la masse moyenne des cristaux d'énantiomère désiré récoltée soit importante, mais suffisamment court pour empêcher que le contre énantiomère cristallise et obtenir ainsi une pureté optique élevée, en particulier supérieure à 85 %. Le refroidissement est donc généralement suivi par polarimétrie afin de déterminer le moment propice de la filtration. De préférence, le refroidissement est compris entre 50 et 70 minutes, mieux encore, il est de 60 minutes lorsque le solvant mis en oeuvre est l'éthanol.

De même, la durée du palier à la température $T_F$ pour les procédés SIPC, AS3PC et S3PC est de préférence suffisamment importante pour permettre de récolter une masse de cristaux d'énantiomères désirés élevée, mais pas trop importante de manière à empêcher que le contre énantiomère cristallise en même temps que l'énantiomère désiré, et obtenir ainsi une pureté optique élevée.

Selon un mode préférentiel, la durée du palier de température $T_F$ est comprise entre 15 et 60 minutes, de préférence égale à environ 60 minutes.

L'homme du métier pourra adapter la vitesse d'agitation au type de réacteur mis en oeuvre dans les procédés SIPC, S3PC ou AS3PC. A titre indicatif, pour un réacteur de 2 ou 10 litres, la vitesse d'agitation du milieu peut être maintenue entre 150 et 250 tours/mn.

De façon particulièrement intéressante, ces méthodes de cristallisation préférentielles permettent d'isoler les énantiomères optiques du modafinil dont notamment l'énantiomère lévogyre avec des rendements très supérieurs à ceux obtenus par dédoublement avec un agent chiral. Les rendements obtenus sont généralement de l'ordre de 90 %, voire même supérieurs, par rapport à l'énantiomère optique (+) ou (-), soit de l'ordre de 45 %, voire plus, par rapport au mélange racémique.

• **METHODES AS3PC, SIPC ET S3PC**

Sont décrites ci-après les méthodes AS3PC et SIPC mentionnées plus haut.

<u>Les équilibres hétérogènes ternaires : antipodes R et S, et solvant A</u>

Par exemple, l'ouvrage de J. E. Ricci (Ed. Dover Publication Inc. New York, 1966, The Phase Rule and Heterogeneous Equilibrium) traite du cas général des équilibres hétérogènes dans les systèmes ternaires. La description ci-après sera limitée aux aspects particuliers du système ternaire : A (solvant achiral), R et S (énantiomères non racémisables dans le domaine de température exploité), nécessaires à la compréhension des différents procédés de cristallisation préférentielle.

Afin de mettre en évidence le rôle particulier du solvant, ce système ternaire sera représenté à partir d'un prisme droit à section triangle rectangle isocèle, sur lequel la température est portée sur un axe perpendiculaire au plan des concentrations.

L'identité des variables thermodynamiques des deux énantiomères : Tf, ΔHf, solubilité dans un solvant achiral, etc., fait que la représentation des domaines est symétrique par rapport au plan vertical A-TS-T, rassemblant les mélanges optiquement inactifs, de la figure 1. Afin de faciliter une première description de ce système, les simplifications suivantes ont été adoptées :

- les seules phases qui cristallisent sont les constituants purs dans un arrangement donné (absence de racémate, de solvate et de polymorphisme pour les antipodes) ;
- la miscibilité entre les constituants indépendants est nulle à l'état solide ;

- le solvant possède un point de fusion nettement inférieur à celui des antipodes ;
- dans le domaine de la température exploité, la solubilité d'un antipode n'est pas influencée par la présence du second dans la solution (loi de Meyerhoffer respectée), ce qui se traduit par une valeur du rapport $\alpha = 2$).

Représentation des équilibres ternaires en fonction de la température

La figure 1 permet de visualiser les domaines de phases suivants :

- le domaine monophasé de la solution diluée ($\phi = 1$) ;
- les trois nappes de cristallisation des constituants délimitant les domaines biphasés ($\phi = 2$). La surface de dépôt du solvant est confinée au voisinage de A, car le point de fusion de ce constituant est nettement plus bas que celui des autres constituants, conformément aux conditions mentionnées précédemment ;
- les trois courbes monovariantes ($\phi = 3$) ou vallées eutectiques issues des points eutectiques binaires ;
- l'invariant eutectique ternaire à $T\varepsilon$ ($\phi = 4$), au-dessous duquel les trois constituants sont cristallisés.

La figure 2 représente de façon superposée deux coupes isothermes à $T_D$ et $T_F$ du ternaire visualisé à la figure 1. A chaque température, la coupe est composée de quatre domaines comme il est détaillé dans le tableau ci-dessous.

| Température | Limite du domaine | Nature des phases à l'équilibre | Nombre de phases à l'équilibre |
|---|---|---|---|
| $T_D$ | A - $S_D$ - $I_D$ - $S'_D$ | solution diluée | 1 |
| $T_D$ | R - $S_D$ - $I_D$ | solution + cristaux de R | 2 |
| $T_D$ | S - $S'_D$ - $I_D$ | solutions + cristaux de S | 2 |
| $T_D$ | $I_D$ - R - S | solution + cristaux de R et S | 3 |
| $T_F$ | A - $S_F$ - $I_F$ - $S'_F$ | solution diluée | 1 |
| $T_F$ | R - $S_F$ - $I_F$ | solution + cristaux de R | 2 |
| $T_F$ | S - $S'_F$ - $I_F$ | solution + cristaux de S | 2 |
| $T_F$ | $I_F$ - R - S | solution + cristaux de R et S | 3 |

Coupe isoplèthe RYT

La figure 3 représente la coupe isoplèthe R-Y-T qui est fondamentale dans la compréhension de la cristallisation conduite par refroidissement de solutions ternaires, en quasi-équilibre thermodynamique. Cette même coupe est également nécessaire pour le suivi des procédés hors équilibre, SIPC, variantes et AS3PC. Ce plan est le lieu géométrique des points vérifiant la relation :

$X_A / X_S = (1-Y) / Y =$ constante, avec $X_A$ et $X_S$ donnant les fractions massiques en solvant et en antipodes S.

A partir de la figure 3, on distingue :

- le domaine monophasé de la solution ternaire ;
- le liquidus de l'antipode R, cette courbe représente l'intersection du plan R-Y de la figure 2 avec la nappe de cristallisation de ce constituant. Cette courbe d'équilibre stable prend naissance à la fusion de l'antipode R (non représentée) et se trouve limitée vers les basses températures par le point L, appartenant à la vallée eutectique ternaire des mélanges racémiques. Cette dernière courbe et la trace du conoïde à $T_L$ (segment horizontal à $T_L$) délimitent le domaine biphasé : solution saturée plus cristaux de R ; elle se prolonge dans le domaine triphasé sous-jacent par une courbe de solubilité à caractère métastable du même antipode R (en traits discontinus) ;
- le domaine triphasé : cristaux de T et de S, plus solution saturée. Ce domaine est limité en haut par la trace horizontale du conoïde de R, vers le bas par la trace du plan invariant eutectique ternaire, à gauche par la trace Lm de l'un des conoïdes relatifs à l'antipode S.
- La trace KL de la nappe de cristallisation de l'antipode S qui limite dans la partie supérieure le domaine biphasé : solution saturée plus cristaux de S. Ce domaine est limité dans sa partie inférieure par les traces des deux conoïdes de S : gm et Lm. La localisation de cette seconde trace Lm du conoïde de S par rapport à la courbe de solubilité métastable de R prolongement de EL sera discutée plus loin à propos de la position relative de F1 et F en fonction

du rapport des solubilités $\alpha$ ;

- l'invariant ternaire à la température $T\varepsilon$ au-dessous duquel se trouvent les trois constituants cristallisés A, R et S.

Evolution au refroidissement et en quasi-équilibre thermodynamique de solutions ternaires présentant un faible excès énantiomérique

Il est considéré dans ce qui suit que le point ensemble du système (i. e. le point représentatif de la composition globale du mélange) se situe sur la verticale passant par le point E des figures 2 et 3, sa position précise est définie par sa température (ou cote). Seul l'intervalle de température suivant est envisagé :

- $T_D$ : température pour laquelle le mélange de départ est une solution homogène, et
- $T_F$ : température de fin de cristallisation et filtration, située dans le domaine triphasé.

Cette composition globale E correspond à une solution racémique légèrement enrichie par une masse M d'antipode R et réalisant une masse totale Mt (l'excès énantiomérique R - S / R + S est généralement compris entre 4 % et 9 %). Les conditions d'équilibre sont obtenues par un refroidissement très lent et par un ensemencement en phase (s) solide(s) dès que le point ensemble E figuratif du mélange parvient dans un domaine où cette (ces) phase(s) est (sont) présente(s) à l'équilibre.

A la température de départ $T_D$, la solution est homogène. Au refroidissement, on observe successivement :

- la cristallisation de l'antipode R seul, de $T_{HOMO}$ Jusqu'à $T_L$, simultanément le point solution se déplace sur la courbe de solubilité de l'antipode R, à savoir du point E à la cote $T_{HOMO}$, au point L à l'intérieur de la coupe isoplèthe R-Y. Au point L, la masse M de cristaux R en équilibre avec la solution saturée est donnée par Mt ($X_E$ - $X_L$ / 1 - $X_L$) = M et correspond à l'excès énantiomérique présent dans la solution initiale (figure 3) ; les abscisses des points L, E et R correspondent aux compositions, et 1 (figure 3).
- à partir de $T_L$, le point solution évolue de L vers $I_F$ sur la courbe monovariante contenant les solutions de composition racémique, représentée à la figure 2, sortant ainsi de la coupe isoplèthe R-Y de la figure 3 ; les cristaux de R et de S se déposent alors simultanément et en égale quantité.

Le dédoublement ne peut pas être réalisé dans des conditions d'équilibre pour des températures inférieures à $T_L$.

Evolution du point solution lors du dédoublement par entraînement conventionnel, conformément au procédé SIPC

Cristallisation du premier antipode en excès

La solution E précédente est homogénéisée à la température $T_D$ (figures 4 et 5). Afin de la rendre sursaturée, elle est refroidie rapidement à la température $T_F$ sans qu'aucune cristallisation n'apparaisse. Cette solution, hors équilibre thermodynamique, est ensuite ensemencée par des germes d'antipode R très purs de même chiralité que celle de l'antipode en excès. La cristallisation isotherme de l'antipode R s'établit et le point représentatif de la solution évolue à l'intérieur de la coupe R-Y-T de E à la cote $T_F$ avec lequel il est d'abord confondu, jusqu'en F où l'on procède rapidement à une filtration. La masse d'antipode R récupérée est de 2M ou encore égale à Mt ($X_E$ - $X_F$ / 1 - $X_F$).

Cristallisation du second antipode, cyclicité des opérations

L'opération fondamentale précédente a donc créé une solution F enrichie en antipodes S. En ajoutant une masse 2M de mélange racémique (égale à celle de l'antipode récupéré) et en chauffant ce mélange à la température $T_D$, on obtient une solution homogène E' symétrique de E par rapport au plan vertical A-(RS)-T. Le processus permettant d'obtenir une masse 2M d'antipode S sera lui aussi représenté par un cheminement symétrique du précédent par rapport à ce plan médian. On procède donc en séquence aux opérations suivantes :

- la solution E' homogène à la température $T_D$ est d'abord refroidie à $T_F$, puis,
- ensemencée en germes très purs d'antipode S ; la croissance de cet antipode déplace le point représentatif de la solution sur le segment horizontal E'F' (à la cote $T_F$) ;
- quand le point solution est confondu avec F', la solution est filtrée et fournit une masse 2M d'antipode S ;
- après un nouvel ajout d'une masse 2M de mélange racémique et un nouveau chauffage à $T_D$, on obtient à nouveau une solution homogène dont le point représentatif est confondu avec le point initial E à la cote $T_D$;
- la suite du processus revient simplement à reproduire ce cycle d'opérations.

Variantes du procédé SIPC

La littérature (Amiard, G., 1956, Bull. Soc. Chim. Fr. 447 ; Collet, A., Brienne, M. J., Jacques, J., 1980, Chemical reviews 80, 3, 215-30 ; Noguchi Institute, 1968, brevet GB 1 197 809) repose sur le schéma général précédent ; les principales modifications apparues dans la littérature sont classées de la façon suivante :

a) Nucléation primaire spontanée de l'antipode en excès Lors du dédoublement de la (±) thréonine (Amiard, G., 1956, Bull. Soc. Chim. Fr. 447), la nucléation primaire de l'antipode en excès intervient spontanément au sein de la solution homogène sursaturée. Cette nucléation primaire se produit alors que le point E figurant la composition de l'ensemble se situe dans le domaine triphasé et que la solution n'est pas agitée (Collet, A., Brienne, M. J., Jacques, J., 1980, Chemical Reviews 80, 3, 215-30).

b) Ensemencement pendant le refroidissement (S3PC)
Ce protocole est le plus fréquemment rencontré dans la littérature (Noguchi Institute, 1968, brevet GB 1 197 809) quand le procédé diffère de SIPC. Parmi les procédures citées, des différences apparaissent ; toutefois, on peut dégager les grandes lignes communes suivantes :

- refroidissement de la solution homogène de $T_D$ à une température inférieure à $T_L$ mais supérieure à $T_F$ ;
- ensemencement, par des germes de même chiralité que celle de l'antipode en excès, de la solution homogène sursaturée située dans le domaine triphasé ;
- refroidissement jusqu'à $T_F$. Dans certains cas, cette dernière étape est contrôlée par une programmation précise de la température (Noguchi Institute, 1968, brevet GB 1 197 809).

On regroupera ces protocoles sous le même terme "S3PC" pour "Seeded polythermic programmed preferential crystallization" bien que la programmation de la température soit inexistante ou limitée à la deuxième phase du refroidissement.

Evolution du point solution lors du dédoublement par entraînement programmé et auto-ensemencé selon le procédé de l'invention AS3PC

Afin de mieux comparer les procédés classiques et le procédé AS3PC, le point initial E est choisi arbitrairement sur les figures 6 et 7, identique au cas précédent ; toutefois, comme cela apparaîtra dans les exemples qui suivront, le procédé AS3PC permet de prendre un point E plus écarté du plan A-(RS)-T et donc avec un excès énantiomérique plus important et ainsi d'améliorer la récolte en cristaux de chaque opération.

Cristallisation du premier antipode en excès

Au début du processus, et contrairement aux protocoles classiques, l'ensemble, cristaux plus solution, n'est plus homogénéisé mais est porté à la température $T_B$. La solution initiale est alors en équilibre avec les cristaux d'énantiomères en excès (par exemple R sur la figure 7). Les points figuratifs de la solution ($S_E$) et de l'ensemble (E) ne sont donc pas confondus dès le début du processus. Ce mélange biphasé est soumis à une loi programmée de descente en température sans ajout de germes cristallins. Le point représentatif de la solution décrit une courbe $S_EF$, contenue dans le plan R-Y-T, dépendante de la cinétique de refroidissement (figure 7). Avec une cinétique correctement ajustée, on obtient au début une croissance des cristaux d'énantiomère en excès, la cristallisation évolue ensuite vers un régime simultané de croissance plus nucléation secondaire. Quand le point représentatif de la solution atteint le point F, on procède à la filtration pour récupérer une masse 2M de cristaux d'antipode R.

Cristallisation du second antipode, cyclicité des opérations

A partir du point F, qui correspond à la solution-mère précédente, on passe au point E', symétrique de E par rapport au plan vertical A-(RS)-T, par ajout d'une masse 2M de mélange racémique et chauffage à la température $T_B$. L'excès énantiomérique est mis à profit pour se placer dans le domaine biphasé contenant la solution saturée et les cristaux de l'antipode en excès. Au préalable, le mélange racémique ajouté lors du passage de F à E' (comme de F' à E) sera broyé et tamisé afin d'accélérer l'étape de dissolution des deux antipodes et plus particulièrement de l'antipode en défaut, et de permettre ainsi la formation d'un nombre important de cristaux de l'antipode en excès jouant le rôle des semences introduites lors des procédés classiques.

La solution saturée $S'_E$, symétrique de SE, par rapport au plan A-(RS)-T, est soumise à la même loi de refroidissement. Les cristaux présents dès le début du refroidissement croissent et participent ensuite à un double mécanisme

de croissance + nucléation secondaire. Comme dans le cas de la première cristallisation, aucun ensemencement n'est donc nécessaire.

Pendant ce temps, le point représentatif de la solution se déplace sur une courbe $S_{E'}F'$contenue dans le plan de la coupe isoplèthe S-Y'-T symétrique par rapport au plan bisecteur A-(RS)-T.

Au moment où la solution acquiert le point représentatif situé en F', on procède à la filtration pour récolter une masse 2M de mélange racémique broyé et tamisé suivie d'une élévation de température à $T_B$ redonne le mélange biphasé à l'équilibre de départ.

La continuation du processus revient alors à répéter ce cycle d'opérations donnant alternativement les cristaux d'antipode R et S.

Conditions nécessaires pour la mise en oeuvre du procédé AS3PC

a) Le mélange équimolaire des antipodes optiques réalise, dans le solvant utilisé et pour l'intervalle de température $T_B$ - $T_F$, un conglomérat (antipodes purs ou solvates) ; toutefois, l'existence d'un racémate métastable n'est pas un handicap.

b) Les molécules à dédoubler sont stables dans ce solvant et dans la gamme de températures utilisées entre $T_B$ et $T_F$.

c) Une détermination des températures d'équilibre ternaire $T_L$ et $T_{HOMO}$ est nécessaire. La température $T_L$ est la température de dissolution du mélange racémique en l'absence de tout excès énantiomérique dans la solution. $T_L$ étant déterminée, la température $T_{HOMO}$ correspond à la température d'homogénéisation de la solution. Elle dépend de l'excès énantiomérique de départ et du rapport $\alpha$ des solubilités du mélange racémique et de l'antipode à $T_L$. La connaissance des capacités de sursaturation des solutions entre $T_L$ et $T_F$ est également nécessaire, suivant la cinétique de refroidissement, le mode d'agitation, la nature du récipient et la granulométrie des cristaux de l'antipode en excès. En première approximation, le temps d'apparition des cristaux par nucléation primaire dans la solution racémique L homogène, refroidie à partir d'une température légèrement supérieure à $T_L$ avec la même cinétique, donne une indication sur la capacité de sursaturation tolérée par le conglomérat dans ces conditions expérimentales. Cette façon d'opérer a été prise en compte dans les exemples.

d) La connaissance de la cinétique de dissolution d'une masse connue de mélange racémique (de granulométrie donnée) dispersée dans la solution à la température $T_B$. Quelques essais suffisent pour connaître cette durée.

Dans ce qui suit, les exemples et figures sont donnés à titre illustratif mais non limitatif de la présente invention.

FIGURES

La Figure 1 est une représentation en perspective du système ternaire solvant A - antipode R - antipode S, en fonction de la température ainsi que des nappes de cristallisation de chaque constituant et des compositions des solutions doublement saturées (courbes monovariantes) ; sur cette figure sont également représentées les isothermes aux températures $T_D$ et à $T_F$ et le plan d'eutexie ternaire à la température $T\varepsilon$ et renfermant quatre phases.

La Figure 2 est une projection, sur le plan des concentrations, des équilibres à $T_D$ et $T_F$, ainsi qu'une représentation de la trace de la coupe isoplèthe RY, sur laquelle le point E matérialise la composition du mélange initial légèrement enrichi en antipode R et devant déposer ce même antipode.

La Figure 3 est la coupe verticale isoplèthe RY de la figure 2 contenant les points composition de l'antipode en excès et celui de la solution initiale E, sur laquelle est représenté le cheminement, à l'équilibre et au refroidissement, du point-solution pour un mélange de composition $X_E$ (en trait gras). Pour $T < T_L$, le point-solution n'appartient plus à cette coupe.

La Figure 4 est une projection sur le plan des concentrations du cheminement du point-solution (en trait gras) lors du dédoublement alterné par entraînement isotherme à la température $T_F$ et ensemencé, selon la méthode SIPC.

La Figure 5 est la coupe verticale isoplèthe contenant la droite RY de la figure 4 et illustrant le cheminement du point-solution (en trait gras) de E à F lors de l'entraînement isotherme (à $T_F$) et ensemencé, selon la méthode SIPC.

La Figure 6 est une projection sur le plan des concentrations du cheminement du point-solution (en trait gras) lors

du dédoublement par le procédé polythermique programmé et auto-ensemencé (AS3PC).

La Figure 7 est la coupe verticale isoplèthe contenant la droite RY de la Figure 6 et illustrant le cheminement du point-solution (en trait gras) de SE à F lors du dédoublement par le procédé de l'invention polythermique programmé et auto-ensemencé (AS3PC).

La Figure 8 est une projection sur le plan des concentrations du cheminement du point-solution (en trait gras) lors du dédoublement par le procédé polythermique programmé et auto-ensemencé (AS3PC) et vérifiant la relation s ($\pm$) < 2 - $\alpha$.

Toutes coupes isothermes et isoplèthes représentées dans ces figures possèdent des variables compositions exprimées en fractions massiques.

La Figure 9 représente le spectre de diffraction par rayons X réalisé sur poudre correspondant à la forme II de l'énantiomère lévogyre, dextrogyre du modafinil respectivement (diffractomètre : Miniflex Rigaku (Elexience).

La Figure 10 représente le spectre de diffraction par rayons X réalisé sur poudre correspondant à la forme III de l'énantiomère lévogyre, dextrogyre du modafinil respectivement (diffractomètre : Miniflex Rigaku (Elexience).

La Figure 11 représente le spectre de diffraction par rayons X réalisé sur poudre correspondant à la forme IV de l'énantiomère lévogyre, dextrogyre du modafinil respectivement (diffractomètre : Siemens AG).

La Figure 12 représente le spectre de diffraction par rayons X réalisé sur poudre correspondant au solvate de diméthylcarbonate de l'énantiomère lévogyre, dextrogyre du modafinil respectivement (diffractomètre : Siemens AG).

La Figure 13 représente le spectre de diffraction par rayons X réalisé sur poudre correspondant au solvate d'acétonitrile de l'énantiomère lévogyre, dextrogyre du modafinil respectivement (diffractomètre : Siemens AG).

La figure 14 représente le spectre de diffraction par rayons X réalisé sur poudre correspondant à la forme V de l'énantiomère lévogyre du modafinil (diffractomètre : Bruker GADDS).

La figure 15 représente le spectre de diffraction par rayons X réalisé sur poudre correspondant au solvate d'acide acétique de l'énantiomère lévogyre, dextrogyre du modafinil respectivement (diffractomètre : Bruker GADDS).

La figure 16 représente le spectre de diffraction par rayons X réalisé sur poudre correspondant à la forme amorphe de l'énantiomère lévogyre, dextrogyre du modafinil respectivement (diffractomètre : Bruker GADDS).

EXEMPLES

PRÉPARATION DES FORMES CRISTALLINES DE L'ÉNANTIOMÈRE (-)-MODAFINIL, DU (+)-MODAFINIL RESPECTIVEMENT

GENERALITES

Les nouvelles formes cristallines des énantiomères du modafinil ont été caractérisées respectivement par spectroscopie de diffraction par rayons X sur poudre, laquelle fournit une empreinte digitale unique, caractéristique de la forme cristalline étudiée et permet de différencier celle-ci des énantiomères du modafinil amorphes et de toute autre forme cristalline des énantiomères du modafinil.

Les données de diffraction aux rayons X ont été mesurées soit :

- en utilisant un système D5005 en tant que diffractomètre de poudre aux rayons X (Siemens AG, Karlsruhe, Allemagne, méthode d'analyse de données Eva 5.0), avec une radiation de cuivre filtrée au nickel de $\lambda$ = 1.540 Å (avec une vitesse d'accélérateur de 40 KV, courant de tubes de 40 mA) avec une rotation de l'échantillon durant la mesure (angle : 3 à 40° [2 theta] ; à une vitesse de 0.04° [2 theta].$s^{-1}$, la taille du pas étant de 0.04° ; préparation de l'échantillon avec une orientation préférentielle).
- en utilisant un système Miniflex Rigaku (Elexience) en tant que diffractomètre de poudre aux rayons X, avec une radiation de chrome, une vitesse d'accélérateur de 30 KV, un courant de tubes de 15 mA et avec une rotation de

l'échantillon durant la mesure (angle : 3 à 80° [2 theta] ; à une vitesse de 0.05° [2 theta]. $s^{-1}$, la taille du pas étant de 0.1° ; préparation de l'échantillon avec une orientation préférentielle).

- en utilisant un système GADDS en tant que diffractomètre de poudre aux rayons X (Bruker, the Netherlands), équipé avec un détecteur « Hi-Star area » et équipé pour l'analyse des plaques 96 puits. Les analyses sont effectuées à température ambiante en utilisant une radiation de cuivre $CuK_{alpha}$ dans la région des angles 2 théta compris entre 3 et 42°. Le spectre de diffraction pour chaque puit est collecté entre deux domaines de valeur d'angle 2 théta (3° ≤ 2 Théta ≤ 21° ° et 19° ≤ 2 Théta ≤ 42°) avec un temps d'exposition entre 50 et 250 secondes.

Bien entendu, les valeurs d'intensité peuvent varier en fonction de la préparation de l'échantillon, du montage et des instruments de mesure. La mesure en 2 theta peut également être affectée par les variations liées aux instruments de mesure, si bien que les pics correspondants peuvent varier de ± 0,04° à de ± 0,2° selon l'appareillage. Aussi, l'homme du métier appréciera de pouvoir disposer des distances réticulaires qui constituent des données essentielles des spectres de diffraction. Les distances réticulaires sont calculées en utilisant la relation de Bragg [(2d sin theta = nλ, dans laquelle d = la distance réticulaire (Å), λ = la longueur d'onde de la radiation de cuivre, theta = l'angle de rotation du cristal (en degrés)] lorsque cette relation est satisfaite.

❖ EXEMPLES 1 À 10 : PRÉPARATION DE LA FORME I DU (-)-MODAFINIL, DU (+)-MODAFINIL RESPECTIVEMENT

• Exemple 1 :

a) L'énantiomère I du modafinil a été solubilisé à reflux dans des solvants polaires : le méthanol, l'éthanol absolu, l'éthanol absolu contenant 3% d'eau, l'éthanol dénaturé au toluène (2.5%) et contenant 3% d'eau et l'eau, selon les conditions expérimentales détaillées dans le tableau 1.

Tableau 1

| Solvant | Quantité de I-modafinil (g) | Volume de solvant (ml) | Rendement % |
|---|---|---|---|
| Méthanol | 8,37 | ≤ 50 | 63 |
| Ethanol absolu | 7,85 | 115 | 56 |
| Ethanol absolu + 3% d'eau | 5 | 70 | 54 |
| Ethanol dénaturé au toluène+ 3% d'eau | 5 | 70 | 56 |
| Eau | 5 | ≥ 400 | 88 |

Après refroidissement rapide par trempe dans un bain d'eau et de glace pendant 30 minutes, le milieu a été filtré puis séché à l'étuve à 35°C. Le produit cristallisé a été identifié par son spectre de diffraction X sur poudre comme étant le polymorphe de forme I du I-énantiomère du modafinil.

b) L'énantiomère d du modafinil (555 g), traité dans les mêmes conditions expérimentales que l'exemple 1 a dans un mélange d'éthanol dénaturé au toluène (2 L) et d'eau (0,1 L), cristallise sous la forme polymorphique I telle qu'identifiée par son spectre de diffraction X sur poudre avec un rendement de 91 %.

• Exemple 2 : Recristallisation dans l'acétone

a) 2 g de (-)-modafinil sont suspendues dans l'acétone (20 ml) dans un ballon tricol équipé d'un réfrigérant, d'un thermomètre et d'un agitateur. Le mélange est chauffé au reflux. Le mélange réactionnel est agité pendant 30 mn à environ 56°C jusqu'à dissolution complète du (-)-modafinil. La solution est alors refroidie lentement avec une vitesse de -0,5°C/mn jusqu'à 10°C sous agitation. Le mélange réactionnel est filtré, et le solide obtenu est séché pour conduire à la forme I du (-)-modafinil identifié par son spectre de Diffraction X. Rendement 62%.

b) Les mêmes conditions expérimentales appliquées au (+)-modafinil conduisent à l'obtention d'un spectre de diffraction X identique.

• Exemple 3 : Recristallisation dans le méthanol

a) 1 g de (-)-modafinil est ajouté dans 7 ml de méthanol chauffé au reflux jusqu'à dissolution complète. Le mélange réactionnel est précipité en ajoutant 6 ml d'eau à 1°C. La suspension est maintenue sous agitation pendant 1 mn et ensuite filtrée sur un verre fritté (N° 3). Le solide isolé est séché pour conduire à la forme I du (-)-modafinil identifié par son spectre de Diffraction X. Rendement 55%.

b) Les mêmes conditions expérimentales appliquées au (+)-modafinil conduisent à l'obtention d'un spectre de diffraction X identique.

• Exemple 4 : Recristallisation dans le méthanol (2$^{e}$ exemple)

a) 2,5 g de (-)-modafinil sont ajoutés à 90 ml de méthanol chauffé au reflux jusqu'à dissolution complète du (-)-modafinil. La solution limpide est ajoutée à 200 ml d'eau à 1°C et laissée sans agitation pendant 10 mn. Le mélange réactionnel est filtré, et le solide récupéré est séché pour conduire à la forme I du (-)-modafinil identifié par son spectre de Diffraction X. Rendement 78%.

b) Les mêmes conditions expérimentales appliquées au (+)-modafinil conduisent à l'obtention d'un spectre de diffraction X identique.

• Exemple 5 : Recristallisation dans le dioxanne 1-4

a) Dans un ballon de 50 mL, on introduit 20 mL de dioxanne 1-4 que l'on porte à reflux. 2 g de (-)-modafinil sont ajoutés afin d'obtenir la saturation; l'agitation est assurée par un barreau magnétique (300 Tr/min). L'ensemble est refroidi après solubilisation totale du (-)-modafinil avec une rampe de refroidissement de -0,5°C/min jusqu'à 20°C. Les cristaux obtenus sont filtrés sur verre fritté et identifiés comme étant de la forme I par son spectre de Diffraction X. Rendement 51 %.

b) Les mêmes conditions expérimentales appliquées au (+)-modafinil conduisent à l'obtention d'un spectre de diffraction X identique.

• Exemple 6 : Recristallisation dans un mélange d'ortho, méta et para xylène

a) Dans un ballon de 250 mL, 180 mL d'un mélange d'ortho, méta et para xylène sont introduits et portés à reflux. 0,5 g de (-)-modafinil sont ajoutés afin d'obtenir la saturation; l'agitation est assurée par un barreau magnétique (300 Tr/min). L'ensemble est refroidi après solubilisation totale du (-)-modafinil avec une rampe de refroidissement de -0,5°C/min jusqu'à 15°C. Les cristaux obtenus sont filtrés sur verre fritté et identifiés comme étant de la forme I par son spectre de Diffraction X. Rendement 26%.

b) Les mêmes conditions expérimentales appliquées au (+)-modafinil conduisent à l'obtention d'un spectre de diffraction X identique.

• Exemple 7 : Recristallisation dans l'acétate d'éthyle

a) Dans un ballon de 250 mL, on introduit 100 mL d'acétate d'éthyle que l'on porte à reflux; 2 g de (-)-modafinil sont ajoutés afin d'obtenir la saturation; l'agitation est assurée par un barreau magnétique (300 Tr/min). L'ensemble est refroidi après solubilisation totale du (-)-modafinil avec une rampe de refroidissement de -0,5°C/mn jusqu'à 20°C. Les cristaux obtenus sont filtrés sur verre fritté et identifiés comme étant de la forme I par son spectre de Diffraction

X. Rendement 66%.

b) le (+)-modafinil (3 g) a été solubilisé à reflux dans l'acétate d'éthyl (100 ml). Après refroidissement par trempe dans un bain d'eau et de glace pendant 30 minutes, le milieu a été filtré puis séché à l'étuve à 50°C et sous vide. Le produit cristallisé a été identifié par son spectre de diffraction X sur poudre comme étant le polymorphe de forme I du (+)-modafinil.

• Exemple 8 : à partir d'autres formes polymorphiques

a) Les CRL40982 forme IV (0,5 g) et CRL40982 forme II (0,5 g) donnent la forme I par chauffage à 100°C.
De plus, la forme I pure du (-)-modafinil peut-être préparée par réempatage d'un mélange de (-)-modafinil forme I (0,5 g) et forme II (0,5 g) et forme III (0,5 g) dans l'acétone (20 ml) pendant un temps suffisant pour obtenir une transformation complète (3 jours).
Dans les deux procédures, la forme I a été identifiée par son spectre de diffraction X obtenu sur poudre.

b) La mise en oeuvre du (+)-modafinil (CRL 40983) dans les mêmes conditions conduit aux mêmes résultats.

• Exemple 9 : à partir du solvate d'acétonitrile

a) 1 g de solvate d'acétonitrile de (-)-modafinil chauffé à 100°C pendant 8 heures se transforme en un solide blanc identifié comme étant du (-)-modafinil forme I par son spectre de diffraction X sur poudre.
b) La mise en oeuvre du (+)-modafinil (CRL 40983) dans les mêmes conditions conduit aux mêmes résultats.

• Exemple 10 : à partir du solvate de carbonate de monodiméthyle

a) 1 g de solvate carbonate de monodiméthyle de (-)-modafinil chauffé à 110°C pendant 16 heures se transforme en un solide blanc identifié comme étant du (-)-modafinil forme I par son spectre de diffraction X sur poudre.

b) La mise en oeuvre du (+)-modafinil (CRL 40983) dans les mêmes conditions conduit aux mêmes résultats.

❖ EXEMPLES 11 À 12 : PRÉPARATION DE LA FORME II (CRL 40982 FORME II) DU (-)-MODAFINIL, (CRL 40983 FORME II) DU (+)-MODAFINIL RESPECTIVEMENT

• Exemple 11 par refroidissement rapide

a) L'énantiomère I du modafinil a été solubilisé à reflux dans des solvants: l'acétate d'éthyle, l'isopropanol, le n-propanol et l'éthanol dénaturé au toluène (2,5%), selon les conditions expérimentales détaillées dans le tableau 2.

Tableau 2

| Solvant | Quantité de I-modafinil (g) | Volume de solvant (ml) | Rendement % |
|---|---|---|---|
| Acétate d'éthyle | 6,33 | 385 | 53 |
| Isopropanol | 8 | 110 | 69 |
| n-propanol | 7,85 | 65 | 70 |
| Ethanol dénaturé au toluène (2,5 %) | 5 | 80 | 54 |

Après refroidissement par trempe dans un bain d'eau et de glace pendant 30 minutes, le milieu a été filtré puis séché à l'étuve à 35°C. Dans chaque procédure expérimentale, le produit cristallisé a été identifié par son spectre de diffraction X sur poudre comme étant le polymorphe de forme II (CRL40982 forme II) du I-énantiomère du modafinil.
b) L'énantiomère d du modafinil (3,02 g) a été solubilisé dans 100 ml d'isopropanol à reflux puis refroidit par trempe

dans un bain d'eau et de glace pendant 30 minutes, filtré et séché sous vide à l'étuve à 50°C. Dans ces conditions expérimentales, le (+)-modafinil a cristallisé sous la forme polymorphique II (CRL40983 forme II) identifiée par son spectre de diffraction X sur poudre.

• Exemple 12 : par refroidissement dans l'isopropanol

a) Dans un ballon de 250 mL, on introduit 100 mL d'isopropanol que l'on porte à reflux puis 3 g de (-)-modafinil sont ajoutés afin d'obtenir la saturation, le mélange est agité à l'aide d'un barreau magnétique (300 Tr/min). Après solubilisation totale du (-)-modafinil, la solution est refroidie lentement jusqu'à 20°C avec une rampe de refroidissement de -0,5°C/min. Les cristaux obtenus sont filtrés sur verre fritté. Le produit cristallisé a été identifié par son spectre de diffraction X sur poudre comme étant le polymorphe de forme II (CRL40982 forme II) du I-énantiomère du modafinil. Rendement 42%.

b) Les mêmes conditions expérimentales appliquées au (+)-modafinil conduisent à l'obtention d'un spectre de diffraction X identique.

❖ EXEMPLE 13 : PRÉPARATION DE LA FORME III (CRL 40982 FORME III) DU (-)-MODAFINIL, (CRL 40983 FORME III) DU (+)-MODAFINIL RESPECTIVEMENT

• Exemple 13 : par refroidissement lent dans l'acétone

a) L'énantiomère I du modafinil (5 g) est solubilisé à reflux dans 90 ml d'acétone. Après refroidissement rapide par trempe dans un bain d'eau et de glace pendant 30 minutes, le milieu est filtré puis séché à l'étuve à 35°C. Le produit cristallisé a été identifié par son spectre de diffraction X sur poudre comme étant le polymorphe de forme III du I-énantiomère du modafinil. Rendement 61 %.

b) Les mêmes conditions expérimentales appliquées au (+)-modafinil conduisent à l'obtention d'un spectre de diffraction X identique.

❖ EXEMPLES 14 À 16 : PRÉPARATION DE LA FORME IV (CRL 40982 FORME IV) DU (-)-MODAFINIL, (CRL 40983 FORME III) DU (+)-MODAFINIL RESPECTIVEMENT

• Exemple 14 : Recristallisation dans le Chloroforme

a) Dans un ballon de 50 mL, on introduit 20 mL de Chloroforme que l'on porte à reflux. 1,5 g de (-)-modafinil sont ajoutés afin d'obtenir la saturation; l'agitation est assurée par un barreau magnétique (300 Tr/min). L'ensemble est refroidi lentement après solubilisation totale du (-)-modafinil avec une rampe de refroidissement de -0,5°C/min jusqu'à 20°C. Les cristaux obtenus sont filtrés sur verre fritté identifié comme étant du (-)-modafinil forme IV par son spectre de diffraction X sur poudre.

b) Les mêmes conditions expérimentales appliquées au (+)-modafinil conduisent à l'obtention d'un spectre de diffraction X identique.

• Exemple 15 : Recristallisation dans la méthyléthylcétone

a) Dans un ballon de 250 mL, on introduit 100 mL de méthyléthylcétone que l'on porte à reflux. 2 g de (-)-modafinil sont ajoutés afin d'obtenir la saturation; l'agitation est assurée par un barreau magnétique (300 Tr/min). L'ensemble est refroidi lentement après solubilisation totale du (-)-modafinil avec une rampe de refroidissement de -0,5°C/min jusqu'à 20°C. Les cristaux obtenus sont filtrés sur verre fritté et identifié comme étant du (-)-modafinil forme IV par son spectre de diffraction X sur poudre.

b) Les mêmes conditions expérimentales appliquées au (+)-modafinil conduisent à l'obtention d'un spectre de diffraction X identique.

• Exemple 16 : Recristallisation dans le tétrahydrofuranne

Dans un ballon de 50 mL, on introduit 20 mL de tétrahydrofuranne que l'on porte à reflux. 1 g de (-)-modafinil sont ajoutés afin d'obtenir la saturation; l'agitation est assurée par un barreau magnétique (300 Tr/min). L'ensemble est refroidi lentement après solubilisation totale du (-)-modafinil avec une rampe de refroidissement de -0,5°C/min jusqu'à 10°C. Les cristaux obtenus sont filtrés sur verre fritté et identifié comme étant du (-)-modafinil forme IV par son spectre de diffraction X sur poudre.

❖ EXEMPLES 17 ET 17 BIS : PRÉPARATION DE LA FORME V (CRL 40982 FORME V) DU (+)-MODAFINIL, DU (CRL 40983 FORME V) DU (+)-MODAFINIL RESPECTIVEMENT

• Mode opératoire pour les exemples 17 et 17 bis

Une solution méthanolique de l'énantiomère d du modafinil (150 mg/ml) est répartie en plaque de 96-puit puis le méthanol est évaporé sous vide léger avant d'ajouter 25 µL de solvants divers (concentration = 3,75 mg/25 µL de solvant) à température ambiante. Les plaques multipuits sont en acier inoxydable (316 L) et chaque puits scellé contient un volume total de 50 µL. La plaque est chauffée, jusqu'à une température initiale de 60°C selon un gradient de température de 4,8°C/mn. Au bout de 30 minutes, la plaque est refroidie lentement (- 0,6°C/mn) ou rapidement (-300°C/min) jusqu'à l'obtention d'une température finale de 3°C, puis est maintenue à cette température finale pendant un minimum de 1 heure ou un maximum de 48 heures. Le solvant est évaporé sous vide (atmosphère d'azote) et le produit cristallisé analysé.

• Exemple 17 : Recristallisation dans la 2-propanone

Le d-modafinil a cristallisé dans la 2-propanone selon les conditions opératoires ci-dessus, en procédant au refroidissement lent (- 0,6°C/mn) et en maintenant la température de 3°C pendant 1 heure. Les cristaux sont identifiés comme étant du (+)-modafinil forme V (CRL40983 forme V) par son spectre de diffraction X sur poudre.

• Exemple 17 bis : Recristallisation dans le tétrahydrofuranne (THF)

Le d-modafinil a cristallisé dans le THF selon les conditions opératoires ci-dessus, en procédant au refroidissement rapide (- 300°C/mn) et en maintenant la température de 3°C pendant 1 heure. Les cristaux sont identifiés comme étant du (+)-modafinil forme V (CRL40983 forme V) par son spectre de diffraction X sur poudre.

❖ EXEMPLES 18 A 19 : PRÉPARATION DES SOLVATES DU (-)-MODAFINIL ET DU (+)-MODAFINIL

• Exemple 18 : Préparation du solvate de diméthylcarbonate du (-)-modafinil

a) 2 g de (-)-modafinil sont ajoutés à 20 ml de diméthylcarbonate et chauffés au reflux. Le mélange réactionnel est agité pendant 10 mn jusqu'à la dissolution complète du (-)-modafinil. La solution est refroidie lentement (-0,5°C/mn) jusqu'à 10°C sous agitation. Le mélange réactionnel est ensuite filtré sur un verre fritté (N° 3). L'analyse du solvate de modafinil diméthylcarbonate montre une masse d'environ 24 % en partant d'environ 50°C jusqu'à 110°C. La stoechiométrie du solvate de diméthylcarbonate est donc 1-1. Il s'agit donc d'un solvate vrai, identifié comme étant le solvate de diméthylcarbonate du (-)-modafinil par son spectre de diffraction X sur poudre. Rendement 88%.

b) Les mêmes conditions expérimentales appliquées au (+)-modafinil conduisent à l'obtention d'un spectre de diffraction X identique.

• Exemple 19 : Préparation du solvate d'acétonitrile du (-)-modafinil

a) Des cristaux de (-)-modafinil de forme polymorphique I sont suspendus dans de l'acétonitrile pendant 3 jours à

20°C. Le solide récupéré est identifié comme un solvate d'acétonitrile par diffraction aux rayons X. Le solvate correspond à un solvate vrai de stoechiométrie : 1-1. identifié comme étant le solvate d'acétonitrile du (-)-modafinil par son spectre de diffraction X sur poudre. Rendement 92%.

b) Les mêmes conditions expérimentales appliquées au (+)-modafinil conduisent à l'obtention d'un spectre de diffraction X identique.

• Exemple 20 : Préparation du solvate d'acide acétique

a) 75 mg de d ou de l-modafinil ont été mis en suspension dans l'acide acétique dans des réacteurs Minimax pour obtenir une concentration de 15 % (poids/volume). Le milieu de cristallisation, sous agitation constante est porté à une température initiale de 60°C or 80°C selon une gradient de température de 3°C/mn. Au bout de 30 minutes le milieu est refroidi lentement (- 0,6°C/mn) ou rapidement (- 300°C/mn) jusqu'à l'obtention d'une température finale de 3°C, puis est maintenue à cette température finale pendant un minimum de 1 heure ou un maximum de 48 heures. Dans ces conditions expérimentales, le solvate d'acide acétique a été obtenu et identifiée par son spectre de diffraction X sur poudre.

b) Les mêmes conditions expérimentales appliquées au (+)-modafinil conduisent à l'obtention d'un spectre de diffraction X identique.

• Exemple 21 : Préparation de la forme amorphe du (-) et du (+)-modafinil

Le solvate de (-) ou de (+) modafinil obtenu dans l'exemple 20 a été transformé en forme amorphe par chauffage à 120°C pendant 3 heures. Le spectre de diffraction X sur poudre obtenu est représenté à la figure 16.

❖ EXEMPLES 22 À 29 : RÉSOLUTION DE L'ACIDE (±) MODAFINIQUE PAR CRISTALLISATION PRÉFÉRENTIELLE

Selon la méthode AS3PC dans l'éthanol

• Conditions liées aux équilibres

**[0219]** - Solubilité dans l'éthanol du mélange racémique :

| Température (°C) | 10,0 | 20,0 | 30,0 |
|---|---|---|---|
| Solubilité massique (%) | 3,0 | 4,1 | 5,96 |

- Solubilité de l'antipode pur (+) = 1,99 % à 20°C ; rapport $\alpha$ =2,06
- Coordonnées du point L = Concentration : 5,96 % ; température : 30°C •

Evolution de $T_{HOMO}$ avec l'excès énantiomérique = (mélange racémique / (solvant + mélange racémique)) = 5,96 % = constante

| Excès énantiomérique | 0 | 3,94 | 7,66 | 11,1 |
|---|---|---|---|---|
| $T_{HOMO}$ (°C) | $T_L$ = 30 | 32,4 | 34,5 | 36,3 |

• Conditions liées à la cinétique

En ajustant $T_B$ plus proche de $T_L$, environ 40 % de la récolte finale sous forme de fins cristaux peut être ainsi obtenue au début de l'expérience, il ne reste donc plus que 60 % de la masse finale attendue à produire. Cette opération

est aisée à réaliser lorsque le rapport Z est suffisamment élevé (supérieur ou égal à 0.8 par pourcentage d'excès énantiomérique).

Dans le cas de l'acide modafinique, la cristallisation s'opère correctement.

$$Z=[\frac{d(T_{HOMO})}{de.e}]_{(\pm)\text{constante}}=Z=[\frac{d(T_{HOMO})}{de.e}]_{TL\text{constante}}=\frac{5}{9}$$

Température $T_{B1}$ = 33,5°C et $T_{B2}$ = 31,5°C.
Température $T_F$ = 17°C.
Loi de refroidissement = T = f(t)

Loi de refroidissement de type I

| Température (°C) | 33,5 | 17 | 17 |
|---|---|---|---|
| t (min) | 0 | 60 | $T_{Filtration}$ |

Loi de refroidissement de type II

| Température (°C) | 31,5 | 17 | 17 |
|---|---|---|---|
| t (min) | 0 | 60 | $T_{Filtration}$ |

Dans les deux cas de figure, à partir de TB1 ou de TB2, la loi de refroidissement est un segment linéaire :

$T_1$ = 33,5 - 0,275 t (Type I)
$T_2$ = 31,5 - 0,24167 t (Type 2)

suivie d'un plateau à 17°C.

• <u>Exemple 22 : Résolution de l'acide (+)-modafinique par la méthode AS3PC à l'échelle 35 cc dans l'éthanol</u>

• Conditions initiales

Excès énantiomérique = 11%

| Masse de solvant | Masse (±) (g) | Masse (+) (g) | Loi de refroidissement |
|---|---|---|---|
| 38,38 | 2,43 | 0,3 | Type 1 |
| Durée du palier à $T_{B1}$ ou à $T_{B2}$ = 30 minutes.<br>Vitesse d'agitation =200 tr/mn | | | |

• Résultats

| N° | Masse d'antipode pur (g) | Pureté optique (%) |
|---|---|---|
| 1 | 0,61 | (+) 90,7 |
| 2 | 0,65 | (-) 89,4 |
| 3 | 0,68 | (+) 90,5 |
| 4 | 0,64 | (-) 90,6 |
| 5 | 0,65 | (+) 88,8 |

(suite)

| N° | Masse d'antipode pur (g) | Pureté optique (%) |
|---|---|---|
| 6 | 0,72 | (-) 91,5 |
| 7 | 0,71 | (+) 92,8 |
| - Masse moyenne des cristaux d'antipode pur = 0,66 g<br>- Pureté optique moyenne = 90,6 % | | |

• Exemple 23 : Résolution de l'acide (±) modafinique par la méthode de l'AS3PC à l'échelle de 400 cc dans l'éthanol

• Conditions initiales

Excès énantiomérique initial = 11 %

| Masse de solvant | Masse (±) (g) | Masse (+) (g) | Loi de refroidissement |
|---|---|---|---|
| 511 | 32,42 | 3,99 | Type I |
| Vitesse d'agitation = 200 tr/mn | | | |

• Résultats

| N° | Masse d'antipode pur (g) | Pureté optique (%) |
|---|---|---|
| 1 | 8,41 | (+) 89,4 |
| 2 | 8,69 | (-) 90,7 |
| 3 | 8,57 | (+) 89,8 |
| - Masse moyenne des cnstaux d'antipode pur = 8,55 g<br>- Pureté optique moyenne = 89,63% | | |

• Exemple 24 : Résolution de l'acide (±) modafinique par la méthode de l'AS3PC à l'échelle de 2 litres dans l'éthanol

• Conditions initiales

Excès énantiomérique initial = 11,1 %

| Masse de solvant | Masse (±) (g) | Masse (+) (g) | Loi de refroidissement |
|---|---|---|---|
| 1874 | 118,4 | 14,84 | Type I |

• Résultats

| N° | Masse d'antipode pur (g) | Pureté optique (%) |
|---|---|---|
| 1 | 32,1 | (+) 89,1 |
| 2 | 32,3 | (-) 90,3 |
| 3 | 32,5 | (+) 91,2 |
| 4 | 32,9 | (-) 89,7 |
| 5 | 33,1 | (+) 90,3 |

(suite)

| N° | Masse d'antipode pur (g) | Pureté optique (%) |
|---|---|---|
| 6 | 32,7 | (-) 90,7 |
| 7 | 32,9 | (+) 90,6 |
| - Masse moyenne des cristaux d'antipode pur = 32,6 g<br>- Pureté optique moyenne = 90,3 % | | |

• Exemple 25 : Résolution de l'acide (±) modafinique par la méthode de l'AS3PC à l'échelle de 10 litres dans l'éthanol

• Conditions initiales

Excès énantiomérique initial = 11,7%

| Masse de solvant | Masse (±) (g) | Masse (+) (g) | Loi de refroidissement |
|---|---|---|---|
| 6481 | 408 | 51,32 | Type I ou II |
| Vitesse d'agitation = 200 rpm durant tout le procédé avec un mobile d'agitation Impeller® | | | |

• Résultats

| N° | Masse d'antipode pur (g) | Pureté optique (%) | Durée d'un cycle | Loi de refroidissement |
|---|---|---|---|---|
| 1 | (+) 121,9 | 90,5 | 103 | I |
| 2 | (-) 121,1 | 92,2 | 104 | I |
| 3 | (+) 137,6 | 91,3 | 83 | II |
| 4 | (-) 134,7 | 90,8 | 84 | II |
| 5 | (+) 135,1 | 90,6 | 83 | II |
| 6 | (-) 134,5 | 91,2 | 82 | II |
| - Masse moyenne des cristaux d'antipode pur = 130,8 g<br>- Pureté optique moyenne = 89,9 % | | | | |

Selon la méthode AS3PC dans le 2-méthoxyéthanol

• Conditions liées aux équilibres

- Solubilité dans le 2-méthoxyéthanol du mélange racémique :

| Température (°C) | 10,0 | 20,0 | 30,0 | 40,0 |
|---|---|---|---|---|
| Solubilité massique (%) | 7,4 | 8 | 13,5 | 16 |
| - Solubilité de l'antipode pur (+) = 4 % à 20°C ; rapport $\alpha$ = 2,53<br>- Coordonnées du point L = Concentration : 16 % ; température : 39,4 °C | | | | |

•

Evolution de $T_{HOMO}$ avec l'excès énantiomérique = (mélange racémique / (solvant + mélange racémique)) = 16 % = constante

| Excès énantiomérique | 0 | 4% | 6% | 8% |
|---|---|---|---|---|
| $T_{HOMO}$ (°C) | $T_L$ = 39 | 44 | 46 | 48 |

• Exemple 26 : Résolution de l'acide (+)-modafinique dans le 2-méthoxyéthanol par la méthode AS3PC à l'échelle de 10 Litres

• Conditions initiales

Excès énantiomérique = 10%
Température initiale $T_B$ : 41°C
Température de filtration $T_F$: 30°C
Rampe de température linéaire de 41°C à 30°C en 1 heure

| Masse de solvant | Masse (±) (g) | Masse (+) (g) |
|---|---|---|
| 8000g | 1523 | 132 |

Vitesse d'agitation =200 tr/mn

• Résultats

| N° | Masse d'antipode pur (g) | Pureté optique (%) |
|---|---|---|
| 1 | 269.86 | (+) 100 |
| 2 | 300 | (-) 97 |
| 3 | 348.68 | (+) 100 |
| 4 | 369.2 | (-) 99.97 |
| 5 | 413.97 | (+) 100 |
| 6 | 453.2 | (-) 95.5 |
| 7 | 423.8 | (+) 98 |
| 8 | 456 | (-) 99.7 |
| 9 | 494.6 | (+) 99.3 |
| 10 | 485.4 | (-) 100 |
| 11 | 517 | (+) 92 |
| 12 | 487.97 | (-) 95.9 |
| 13 | 471.24 | (+) 99.5 |
| - Masse moyenne des cristaux d'antipode pur = 422,4 g<br>- Pureté optique moyenne = 98,2 % | | |

Selon la méthode AS3PC dans le méthanol

• Conditions liées aux équilibres

- Solubilité dans le méthanol du mélange racémique :

| Température (°C) | 10,0 | 20,0 | 30,0 | 40,0 |
|---|---|---|---|---|
| Solubilité massique (%) | 7,4 | 9,7 | 13,9 | 25,7 |
| - Solubilité de l'antipode pur (+) = 4,9 % à 20°C ; rapport $\alpha$ = 2,53<br>- Coordonnées du point L = Concentration : 25,6 % ; température : 46,5°C | | | | |

•

Evolution de $T_{HOMO}$ avec l'excès énantiomérique = (mélange racémique / (solvant + mélange racémique)) = 25,7 % = constante

| Excès énantiomérique | 0 | 4% | 6% | 8% | 10% |
|---|---|---|---|---|---|
| $T_{HOMO}$ (°C) | $T_L$ = 45 | 50 | 52 | 53 | 54 |

• Exemple 27 : Résolution de l'acide (+)-modafinique par la méthode AS3PC à l'échelle de 2 Litres dans le méthanol

• Conditions expérimentales

Excès énantiomérique = 10%
Température initiale $T_B$ : 46,5°C
Température filtration $T_F$ : 30°C
Rampe de température : linéaire de 39,4°C à 18°C pendant 1 heure.

| Masse de solvant | Masse (±) (g) | Masse (+) (g) |
|---|---|---|
| 1450g | 501,5 | 55,7 |
| Vitesse d'agitation =230 tr/mn | | |

• Résultats

| N° | Masse d'antipode pur (g) | Pureté optique (%) |
|---|---|---|
| 1 | 107,1 | (+) 99,7 |
| 2 | 90,9 | (-) 78,2 |
| 3 | 137,1 | (+) 72,7 |
| 4 | 125,5 | (-) 84,1 |
| 5 | 95,9 | (+) 94,0 |
| 6 | 91,6 | (-) 88,6 |

(suite)

| N° | Masse d'antipode pur (g) | Pureté optique (%) |
|---|---|---|
| 7 | 87,0 | (+) 85,7 |
| 8 | 92,2 | (-) 88,1 |
| 9 | 107,0 | (+) 104,2 |
| 10 | 130,6 | (-) 120,7 |
| 11 | 159,9 | (+) 111,0 |
| 12 | 123,3 | (-) 113,8 |
| 13 | 133,0 | (+) 130,3 |
| 14 | 143,0 | (-) 134,7 |
| 15 | 139,2 | (+) 128,5 |
| 16 | 159,4 | (-) 127,5 |
| 17 | 114,0 | (+) 111,5 |
| 18 | 123,4 | (-) 120,9 |
| 19 | 180,6 | (+) 99,3 |
| 20 | 114,2 | (-) 110,9 |
| 21 | 123,1 | (+) 120,6 |
| 22 | 118,4 | (-) 115,0 |
| 23 | 140,1 | (+) 135,9 |
| 24 | 186,2 | (-) 118,6 |
| 25 | 157,1 | (+) 106,8 |
| 26 | 121,2 | (-) 102,2 |
| 27 | 126,5 | (+) 122,5 |
| 28 | 106,6 | (-) 99,0 |
| - Masse moyenne des cristaux d'antipode pur = 108 g<br>- Pureté optique moyenne = 87,5 % | | |

Selon la méthode SIPC dans l'éthanol

Conditions liées aux équilibres (voir méthode AS3PC)

• Exemple 28 : Résolution de l'acide (±) modafinique par la méthode SIPC à l'échelle de 2 litres avec ensemencement en fin de refroidissement dans l'éthanol

• Conditions initiales

Excès énantiomérique initial = 11,8 %

Température pour laquelle le mélange de départ est une solution homogène $T_D$ = 40°C.

| Masse de solvant | Masse (±) (g) | Masse (+) (g) | Loi de refroidissement |
|---|---|---|---|
| 1874 | 118,4 | 14,84 | 20 mn de 40°C à 17°C = température d'ensemencement |
| - Temps (palier) à $T_F$ avant l'introduction des germes = 0 minute<br>- Masse des germes = 1 %<br>- Temps de cristallisation = refroidissement le plus rapide possible par trempe | | | |

Vitesse d'agitation = 200 rpm tout au long du procédé avec un mobile d'agitation Impeller®.

• Résultats

| N° | Masse d'antipode pur (g) | Pureté optique (%) |
|---|---|---|
| 1 | 30,9 | (+) 90,4 |
| 2 | 31,5 | (-) 90,7 |
| 3 | 31,3 | (+) 91,4 |
| 4 | 31,2 | (-) 90,9 |
| 5 | 31,6 | (+) 91,5 |
| - Masse moyenne des cristaux d'antipode pur = 31,28 g<br>- Pureté optique moyenne = 91 % | | |

• <u>Exemple 29 : Résolution de l'acide (+)-modafinique par la méthode S3PC à l'échelle 2 litres avec ensemencement pendant le refroidissement dans l'éthanol</u>

- Excès énantiomérique initial : 11,14 %

| Masse de solvant | Masse (±) (g) | Masse (+) (g) | Loi de refroidissement |
|---|---|---|---|
| 1874 | 118,4 | 14,84 | 20 mn de 40°C à 17°C |
| - Température d'ensemencement = 29°C<br>- Masse des germes = 1 %<br>- Temps de cristallisation = refroidissement le plus rapide possible par trempe | | | |

Vitesse d'agitation = 200 rpm tout au long du procédé avec un mobile d'agitation Impeller®.

• Résultats

| N° | Masse | Pureté optique (%) avant purification |
|---|---|---|
| 1 | 25,2 | (+) 84,5 |
| 2 | 24,9 | (-) 85,6 |
| 3 | 25,6 | (+) 84,6 |
| 4 | 25,2 | (-) 85,3 |

(suite)

| N° | Masse | Pureté optique (%) avant purification |
|---|---|---|
| 5 | 24,9 | (+) 85,8 |
| - Masse moyenne des cristaux d'antipodes purs = 25,2 g<br>- Pureté optique moyenne = 85,2 % | | |

❖ EXEMPLES 30 À 32 : CONVERSION DES ÉNANTIOMÈRES OPTIQUES DE L'ACIDE MODAFINIQUE EN ESTER D'ALKYLE

Cette étape est illustrée par la mise en oeuvre de l'acide (-)modafinique.

<u>Exemples 30 à 31 : Estérification de l'acide (-)-modafinique</u>

• <u>Exemple 30: en présence de diméthylsulfate</u>

Dans un ballon de 10 litres, on charge 3,3 litres d'acétone, 0,6 litre d'eau, 349 g de $Na_2CO_3$ (3,29 moles), 451 g d'acide (-)-modafinique (1,64 moles) et on chauffe pour atteindre le reflux. On coule alors en une demi-heure 330 ml de diméthylsulfate (3,29 moles). On prolonge le reflux une heure et on laisse ensuite revenir à l'ambiante en 20 heures.
On coule alors le milieu sur 6,6 kg de glace. La cristallisation est immédiate et après 3 heures d'agitation additionnelle une filtration livre un précipité blanc qui est lavé dans 6 litres d'eau.
On réempâte ensuite ce produit avec 6 litres d'eau et on filtre à nouveau. Le précipité est séché sous vide à 35°C et on obtient ainsi 436,3 g d'ester méthylique (Rendement = 92,3 %).

• <u>Exemple 31 : en présence de chloroformiate de méthyle</u>

Dans 450 ml de méthanol, on introduit 100 g d'acide (-)-modafinique (0,36 mole) et 21,6 ml de triéthylamine (0,36 mole). Sur la solution obtenue après dissolution du sel, on coule progressivement 30 ml de chloroformiate de méthyl (0,36 mole).
La coulée se fait en 15 mn en passant de 28°C à 35°C (dégagement de $CO_2$) On laisse agiter 2 heures et on jette sur de la glace pilée + eau (500 g / 500 ml).
L'ester cristallise ; après filtration et séchage, on obtient 94,5 g d'ester.
(Rendement = 90,1 %).

• <u>Exemple 32 : Ammonolyse de l'ester d'alkyle de l'acide modafinique optiquement actif</u>

Dans un réacteur double enveloppe de 4 litres, on charge 1,63 litre de méthanol dénaturé au toluène, 0,1 litre d'eau et 425,1 g d'ester méthylique (1,474 moles).
On amène la température à 30°C et on commence le barbotage d'ammoniac en régulant à cette température. Cette opération dure 1 h45 et la masse d'ammoniac introduite est de 200 g. On maintient l'agitation pendant 21 h30, puis on refroidit en plaçant la consigne à 0°C.
Le milieu est alors filtré sur verre fritté N°3 et on obtient 57,2 g de premier jet et un filtrat que l'on évapore à sec. Le résidu est repris par 1,2 litre d'éthanol dénaturé au toluène et après filtration on obtient un deuxième jet de 308,6 g.

Première cristallisation :

Les deux jets sont joints et recristallisés dans 1,83 litre d'éthanol dénaturé au toluène. Une filtration à chaud livre un filtrat qui, par refroidissement, donne un produit que l'on filtre et sèche sous vide à 30°C. On obtient 162,2 g de produit blanc.

Deuxième cristallisation :

On mélange ces 162,2 g avec 810 ml d'éthanol dénaturé toluène et on chauffe à reflux pour avoir une dissolution complète. On laisse ensuite cristalliser en glaçant puis on filtre sur verre fritté N°4 et on sèche sous vide à 30°C. On obtient 147,3 g de (-)-modafinil (CRL 40982).
Rendement = 36,6 %.

Caractéristiques :

Pouvoir rotatoire = - 18,6 (Solution à 4,9 % dans le méthanol)
Point de fusion = 163°C.

❖ EXEMPLES 33 À 34 : STRUCTURES CRISTALLINES

• Exemple 33 : Structure de l'acide modafinique

Des cristaux du modafinil ont été obtenus dans l'acétone. Cette phase a les caractéristiques suivantes :

- Hexagonal $P3_1$ ou $P3_2$ suivant l'énantiomère, le modafinil est donc un conglomérat ;
- a = 9,55, b = 9,55, c = 13,14 Å
- $\alpha$ = 90.000, $\beta$ = 90.000, $\gamma$ = 120.000°

Les intensités diffractées ont été mesurées à l'aide d'un diffractomètre automatique SMART APEX (Brucker) à 20°C.
La structure a été résolue avec la suite de logiciels Saintplus, Sadabs, Shelxs.
Il est à souligner le caractère inusuel de ce groupe d'espace pour les molécules chirales organiques.
Dans la maille cristalline, le motif se répète trois fois, soit encore Z = 1. Ces molécules sont reliées entre elles par des liaisons hydrogène, via les fonctions acide et sulfoxide. On peut remarquer que les interactions les plus fortes (les liaisons hydrogène) s'enroulent autour de l'axe hélicoïdal ternaire suivant la direction cristallographique z.

• Exemple 34 : Structure du (-) et (+)-modafinil forme I

La structure cristalline du (+) modafinil de forme 1, identifiée comme identique à celle du (-) modafinil forme I, a été déterminée. Elle possède les caractéristiques suivantes :

- Système cristallin = monoclinique ;
- Groupe d'espace = $P2_1$
- a = 5.6938, b = 26.5024, c= 9.3346 Å
- $\beta$ = 105.970°

Les intensités diffractées ont été mesurées à l'aide d'un diffractomètre automatique SMART APEX (Brucker) à 20°C.

**Aspects de l'invention**

1. Procédé de préparation de formes cristallines des énantiomères optiques du modafinil, comprenant les étapes suivantes:

   i) dissoudre l'un des énantiomères optiques du modafinil dans un solvant autre que l'éthanol;
   ii) cristalliser l'énantiomère du modafinil;
   iii) récupérer la forme cristalline de l'énantiomère du modafinil ainsi obtenue.

2. Procédé de préparation selon l'aspect 1, dans lequel l'énantiomère du modafinil est l'énantiomère lévogyre.

3. Procédé de préparation selon l'aspect 1, dans lequel l'énantiomère du modafinil est l'énantiomère dextrogyre.

4. Procédé selon l'un quelconque des aspects 1 à 3, dans lequel la forme cristalline obtenue est une forme polymorphique.

5. Procédé selon l'un quelconque des aspects 1 à 4, dans lequel la cristallisation est réalisée dans des conditions cinétiques ou thermodynamiques.

6. Procédé de préparation selon l'aspect 5, dans lequel la cristallisation est réalisée par précipitation, éventuellement en présence de germes de cristaux de la forme cristalline désirée.

7. Procédé de préparation selon l'aspect 5, dans lequel la cristallisation consiste en un refroidissement de la solution obtenue à l'étape i).

8. Procédé selon l'aspect 7, dans lequel le refroidissement est lent.

9. Procédé selon l'aspect 7, dans lequel le refroidissement est rapide.

10. Procédé selon les aspects 4 et 8, dans lequel le solvant mis en oeuvre à l'étape i) est choisi parmi l'acétone, le dioxanne 1-4, l'acétate d'éthyle, l'ortho, méta ou paraxylène, ou un mélange d'ortho, méta et/ou paraxylène, la forme polymorphique ainsi obtenue étant alors désignée Forme I.

11. Procédé selon les aspects 4 et 9, dans lequel le solvant mis en oeuvre à l'étape i) est choisi parmi le méthanol, l'eau ou les mélanges alcools/eau, la forme cristalline alors obtenue étant désignée Forme I.

12. Procédé selon les aspects 4 et 9, dans lequel le solvant mis en oeuvre à l'étape i) est l'isopropanol, le n-propanol, l'acétate d'éthyle ou de l'éthanol dénaturé au toluène, la forme polymorphique ainsi obtenue étant désignée Forme II.

13. Procédé selon les aspects 4 et 8, dans lequel le solvant mis en oeuvre à l'étape i) est l'isopropanol, la forme polymorphique ainsi obtenue étant désignée Forme II.

14. Procédé selon les aspects 4 et 9, dans lequel le solvant mis en oeuvre à l'étape i) est l'acétone, la forme polymorphique ainsi obtenue étant désignée Forme III.

15. Procédé selon les aspects 4 et 8, dans lequel le solvant mis en oeuvre à l'étape i) est choisi parmi le tétrahydrofuranne, le chloroforme, la méthyléthylcétone, la forme polymorphique ainsi obtenue étant désignée Forme IV.

16. Procédé selon l'un quelconque des aspects 1 à 3, dans lequel la forme cristalline obtenue est un solvate.

17. Procédé selon les aspects 8 et 16, dans lequel le solvant mis en oeuvre à l'étape i) est le diméthylcarbonate, le solvate ainsi obtenu étant désigné solvate de diméthylcarbonate.

18. Forme polymorphique de l'énantiomère lévogyre ou dextrogyre du modafinil désignée Forme II, caractérisée en ce qu'elle produit un spectre de diffraction X comprenant des raies d'intensité aux distances réticulaires : 8,54 ; 7,57 ; 7,44 ; 4,56 ; 3,78 ; 3,71 (Å).

19. Forme polymorphique selon l'aspect 18, caractérisée en ce qu'elle produit un spectre de diffraction X comprenant des raies d'intensité aux distances réticulaires: 11,33 ; 8,54 ; 7,57 ; 7,44 ; 5,67 ; 5,33 ; 4,83 ; 4,59 ; 4,56 ; 4,45 ; 4,05 ; 3,88 ; 3,78 ; 3,71 ; 3,34 ; 2,83 ; 2,53 (Å).

20. Forme polymorphique de l'énantiomère lévogyre ou dextrogyre du modafinil désignée Forme III, caractérisée en ce qu'elle produit un spectre de diffraction X comprenant des raies d'intensité aux distances réticulaires : 12,28 ; 8,54 ; 5,01 ; 4,10 ; 3,97 ; 3,20 (Å).

21. Forme polymorphique selon l'aspect 20, caractérisée en ce qu'elle produit un spectre de diffraction X comprenant des raies d'intensité aux distances réticulaires : 13,40 ; 12,28 ; 8,54 ; 7,32 ; 6,17 ; 5,11 ; 5,01 ; 4,65 ; 4,60 ; 4,48 ; 4,44 ; 4,27 ; 4,19 ; 4,10 ; 4,02 ; 3,97 ; 3,83 ; 3,66 ; 3,54 ; 3,42 ; 3,20 ; 2,91 ; 2,58 (Å).

22. Forme polymorphique de l'énantiomère lévogyre ou dextrogyre du modafinil désignée Forme IV, caractérisée en ce qu'elle produit un spectre de diffraction X comprenant des raies d'intensité aux distances réticulaires: 12,38; 8,58; 7,34; 5,00; 4,09 (Å).

23. Forme polymorphique selon l'aspect 22, caractérisée en ce qu'elle produit un spectre de diffraction X comprenant des raies d'intensité aux distances réticulaires : 13,88 ; 12,38 ; 10,27 ; 8,58 ; 7,34 ; 6,16 ; 5,660 ; 5,120 ; 5,00 ; 4,64 ; 4,48 ; 4,26 ; 4,18 ; 4,09 ; 3,82 ; 3,66 ; 3,53 ; 3,42, 3,28 ; 3,20 (Å).

24. Forme polymorphique de l'énantiomère lévogyre ou dextrogyre du modafinil, caractérisée en ce qu'elle produit un spectre de diffraction X comprenant des raies d'intensité aux distances réticulaires 9,63 ; 5,23 ; 5,03 ; 4,74 ; 4,66 ; 4,22 ; 4,10 ; 3,77 (Å).

25. Solvate de diméthylcarbonate de l'énantiomère lévogyre ou dextrogyre du modafinil, caractérisé en ce qu'il produit un spectre de diffraction X comprenant des raies d'intensité aux distances réticulaires : 12,31 ; 9,69 ; 9,09 ; 8,54 ; 7,27 ; 6,21 ; 5,45 ; 5,10 ; 5,00 ; 4,83 ; 4,63 ; 4,46 ; 4,22 ; 4,13 ; 4,09 ; 3,78 ; 3,62 ; 3,53 ; 3,42 ; 3,32 ; 3,24 ; 3,21 ; 3,10 (Å).

26. Procédé de conversion d'une première forme cristalline d'un des énantiomères du modafinil en une deuxième forme cristalline dudit énantiomère, distincte de la première, ledit procédé comprenant les étapes suivantes:

i) suspendre ladite première forme cristalline dudit énantiomère du modafinil dans un solvant adapté; et
ii) récupérer ladite deuxième forme cristalline obtenue.

27. Procédé selon l'aspect 26, dans lequel l'énantiomère mis en oeuvre est le (-)-modafinil.

28. Procédé selon l'aspect 27, dans lequel la première forme cristalline est la forme 1.

29. Procédé selon l'aspect 28, dans lequel le solvant mis en oeuvre à l'étape ii) est l'acétonitrile, ce par quoi on obtient un solvate d'acétonitrile.

30. Solvate d'acétonitrile de l'énantiomère (-)-modafinil ou (+)-modafinil, caractérisé en ce qu'il produit un spectre de diffraction X comprenant des raies d'intensité aux distances réticulaires: 16,17 ; 14,14 ; 12,32 ; 10,66 ; 9,79 ; 9,29 ; 8,54 ; 8,15 ; 7,80 ; 7,09 ; 6,31 ; 5,83 ; 5,62 ; 5,41 ; 5,10 ; 4,90 ; 4,66 ; 4,58 ; 4,46 ; 4,33 ; 4,20 ; 4,02 ; 3,92 ; 3,835 ; 3,72 ; 3,60 ; 3,57 ; 3,45 ; 3,33 ; 3,24 ; 3,19 ; 3,09 ; 3,03.

31. Procédé de préparation du modafinil optiquement actif à partir de l'acide modafinique, comprenant les étapes suivantes:

i) réaliser un dédoublement des deux énantiomères optiques de l'acide (±) modafinique et récupérer au moins un des énantiomères;
ii) mettre en contact l'un des deux énantiomères obtenus avec un haloformiate d'alkyle inférieur en présence d'alcool et d'une base organique;
iii) récupérer le produit obtenu;
iv) convertir l'ester obtenu en amide;
v) récupérer le produit obtenu à l'étape iv).

32. Procédé selon l'aspect 31, dans lequel l'haloformiate est un chloroformiate d'alkyle inférieur.

33. Procédé selon l'aspect 32, dans lequel le chloroformiate d'alkyle inférieur est le chloroformiate de méthyle.

34. Procédé selon l'un quelconque des aspects 31 à 33, dans lequel la base mise en oeuvre à l'étape ii) est choisi parmi la triéthylamine, la diisopropylamine, la diéthyl-méthylamine, DBU.

35. Procédé selon l'un quelconque des aspects 31 à 34, dans lequel le solvant mis en oeuvre à l'étape ii) est un alcool aliphatique inférieur, de préférence le méthanol.

36. Procédé selon l'un quelconque des aspects 31 à 35, dans lequel le solvant mis en oeuvre à l'étape iv) est un alcool aliphatique inférieur, de préférence le méthanol.

37. Procédé selon l'un quelconque des aspects 31 à 36, dans lequel le dédoublement des énantiomères optiques de l'acide (±) modafinique à l'étape i) est réalisé selon un procédé de cristallisation préférentielle.

38. Procédé selon l'aspect 37, dans lequel le procédé de dédoublement des deux énantiomères optiques de l'acide (±) modafinique ou des sels de celui-ci est un procédé ensemencé, ledit procédé comprenant les étapes suivantes:

a) homogénéiser à une température $T_D$ un ensemble composé du mélange racémique de cristaux sous forme

de conglomérat, du premier énantiomère de l'acide modafinique et de solvant, dont le point figuratif E, défini par les variables concentration et température $T_D$, se trouve dans le domaine monophasé de la solution diluée;
b) refroidir rapidement la solution préparée à l'étape a) initialement à la température $T_D$ jusqu'à la température $T_F$;
c) ensemencer la solution de l'étape b) en cours ou à la fin du refroidissement ($T_F$) en germes très purs du premier énantiomère;
d) récolter les cristaux du premier énantiomère;
e) additionner aux liqueurs-mères résultant de la récolte réalisée à l'étape d) le mélange racémique de cristaux sous forme de conglomérat, et homogénéiser le nouvel ensemble en chauffant à une température $T_D$, de sorte que le point figuratif E' soit symétrique de E par rapport au plan de mélange racémique du système solvant, antipode (-), antipode (+), ledit point E' se situant dans le domaine monophasé de la solution diluée;
f) refroidir rapidement la solution obtenue à l'étape e) initialement à la température $T_D$, jusqu'à la température $T_F$;
g) ensemencer la solution obtenue à l'étape f) en germes très purs du second énantiomère;
h) récolter les cristaux du second énantiomère;
i) additionner aux liqueurs-mères résultant de la récolte cristalline réalisée à l'étape h) le mélange racémique sous forme de conglomérat de cristaux et homogénéiser le nouvel ensemble en chauffant à une température $T_D$ pour obtenir une composition identique à celle de l'ensemble du point figuratif E initial;
j) répéter les étapes a), b), c), d), e), f), h) et j) pour obtenir successivement le premier puis le second des deux énantiomères.

39. Procédé selon l'aspect 37, dans lequel le procédé de dédoublement des deux énantiomères optiques de l'acide (±) modafinique ou des sels de celui-ci par cristallisation préférentielle est un procédé auto- ensemencé AS3PC, ledit procédé comprenant les étapes suivantes:

a) réaliser un ensemble composé du mélange racémique de cristaux sous forme de conglomérat, du premier énantiomère de l'acide modafinique et de solvant, dont le point figuratif E, défini par les variables concentration et température $T_B$, se situe dans le domaine biphasé de l'énantiomère en excès, et est en équilibre avec sa solution saturée;
b) appliquer une loi de programmation du refroidissement de la température au mélange biphasé préparé à l'étape a), ladite loi de programmation étant telle que les liqueurs-mères gardent une faible sursaturation qui privilégie la croissance de l'énantiomère présent sous forme de cristaux, tout en interdisant la nucléation spontanée du second énantiomère présent dans la solution;
c) adapter pendant toute la durée de la croissance cristalline de l'étape b) une vitesse d'agitation légèrement croissante en fonction du temps, de façon à ce que celle-ci soit à tout moment suffisamment lente pour favoriser une croissance du premier énantiomère en évitant de générer des forces de striction trop importantes provoquant une nucléation non maîtrisée et suffisamment rapide pour réaliser une suspension homogène et un renouvellement rapide de la liqueur-mère autour de chaque cristallite du premier énantiomère;
d) récolter les cristaux du premier énantiomère;
e) additionner aux liqueurs-mères résultant de la récolte réalisée à l'étape d) le mélange racémique de cristaux sous forme de conglomérat, et porter le nouvel ensemble à un palier de température $T_B$ pendant la durée nécessaire à l'obtention de l'équilibre thermodynamique, de sorte que le point figuratif E' soit symétrique de E par rapport au plan des mélanges racémiques du système solvant, antipode (-), antipode (+), ledit point E' se situant dans le domaine biphasé du second énantiomère en excès et en équilibre avec sa solution saturée;
f) appliquer la même loi de programmation du refroidissement qu'à l'étape b) au mélange biphasé préparé à l'étape e) contenant le second énantiomère, de sorte que les liqueurs-mères gardent une faible sursaturation pendant la cristallisation afin de privilégier la croissance de l'énantiomère présent sous forme de cristaux tout en interdisant la nucléation spontanée du premier énantiomère présent dans la solution;
g) adapter, pendant toute la durée de la croissance cristalline de l'étape une vitesse d'agitation légèrement croissante en fonction du temps, de façon à ce que celle-ci soit, à tout moment, suffisamment lente pour favoriser la croissance du second énantiomère en évitant de générer des forces de striction trop importantes provoquant une nucléation non maîtrisée, et suffisamment rapide pour obtenir une suspension homogène et un renouvellement rapide de la liqueur-mère autour de chaque cristallite du second énantiomère;
h) récolter les cristaux du second énantiomère;
i) additionner aux liqueurs-mères résultant de la récolte cristalline réalisée à l'étape g) le mélange racémique de cristaux sous forme de conglomérat pour obtenir un ensemble dont la composition est identique à celle de l'ensemble E initial;
j) répéter les étapes a), b), c), d), e), f) g), h) et i) pour obtenir successivement le premier puis le second des deux énantiomères.

40. Procédé selon l'aspect 39, caractérisé en ce qu'à l'étape a) le choix du ou des solvants et de la gamme de température de travail sont définis de façon à avoir simultanément:

- des antipodes qui réalisent un conglomérat et dont l'éventuel racémate est métastable dans la gamme de température de travail;
- des liqueurs suffisamment concentrées mais de faible viscosité et de faible tension de vapeur;
- une absence de solvolyse et de racémisation;
- une stabilité des solvates si ceux-ci sont présents à l'équilibre et s'il s'agit d'énantiomères dédoublables.

41. Procédé selon l'un quelconque des aspects 39 à 40, caractérisé en ce qu'aux étapes (a) et (e) la température $T_B$ est supérieure à la température $T_L$ d'homogénéisation de la quantité de mélange racémique contenue dans la suspension initiale, et en ce que, à partir de la courbe de variation de $T_{HOMO}$ en fonction de l'excès énantiomérique et pour une concentration constante en mélange racémique $X_L$, ladite température $T_B$ est définie de façon à ce que la masse de fins cristaux du premier énantiomère des étapes (a) et (i) et du second énantiomère de l'étape (e), en équilibre avec leur solution saturée, représente au maximum 50% et de préférence entre environ 25% et 40% de la récolte attendue.

42. Procédé selon l'un quelconque des aspects 39 à 41, caractérisé en ce qu'aux étapes (b) et (f) la loi de programmation du refroidissement de la température $T_B$ à $T_F$ adaptée au montage expérimental est définie de façon:

- à obtenir une faible sursaturation pendant toute la durée de la cristallisation de l'énantiomère présent sous forme de cristaux au début de chaque cycle, cette faible sursaturation provoquant une croissance et une nucléation secondaires douces;
- à atteindre à $T_F$ le maximum de sursaturation de l'autre énantiomère sans nucléation primaire;
- à obtenir une récolte en cristaux aux étapes (d) et (h) qui, après addition de mélange racémique et compensation aux étapes (e) et (i), permet la cyclicité des opérations.

43. Procédé selon l'aspect 42, caractérisé en ce que la loi de programmation du refroidissement est déterminée pour sa partie de $T_L$ à $T_F$ par refroidissement de la solution de concentration $X_L$ de $T_L$ + 1°C à $T_F$, $T_F$ étant inférieur à $T_L$ - ($T_{HOMO}$-$T_L$) afin d'obtenir une solution saturée stable sans nucléation primaire tout en permettant une récolte double de l'excès énantiomérique initial, et en ce que ladite loi de programmation du refroidissement est déterminée pour sa partie de $T_B$ à $T_L$ par extrapolation de cette même loi déterminée de $T_L$ + 1°C à $T_F$.

44. Procédé selon l'un quelconque des aspects 39 à 43, caractérisé en ce qu'aux étapes (b) et (f) la thermicité accompagnant le dépôt du premier énantiomère et du second énantiomère est intégrée dans la loi de programmation du refroidissement.

45. Procédé selon l'un quelconque des aspects 39 à 44, caractérisé en ce qu'aux étapes (e) et (i) on effectue des compensations en solvant.

46. Procédé selon l'un quelconque des aspects 39 à 45, caractérisé en ce qu'aux étapes (a), (e) et (i) les fins cristaux du mélange racémique sous forme de conglomérat, qui sont ajoutés, ont subi, avant d'être introduits, un traitement préalable accélérant l'étape de dissolution, tel qu'un broyage et un tamisage, un traitement par des ondes ultrasonores, une lyophilisation partielle.

47. Procédé selon l'un quelconque des aspects 39 à 46, caractérisé en ce qu'aux étapes (a), (e) et (i), on élève la vitesse d'agitation.

48. Procédé selon l'un quelconque des aspects 38 ou 39 à 47, dans lequel le solvant mis en oeuvre à l'étape a) est l'éthanol, le 2-méthoxyéthanol ou le méthanol.

49. Procédé selon l'aspect 48, dans lequel la température $T_F$ est comprise entre 0 et 40°C.

50. Procédé selon l'un quelconque des aspects 48 ou 49, dans lequel la concentration du mélange racémique à l'étape a) est comprise entre 2 et 50 % massique.

51. Procédé selon l'un quelconque des aspects 48 à 50, dans lequel l'excès énantiomérique à l'étape a) est compris entre 1 et 50 % massique.

52. Procédé selon l'aspect 51, dans lequel la température $T_B$ est comprise entre 25°C et 50°C.

53. Procédé selon l'un quelconque des aspects 48 à 52, dans lequel la durée du palier de température $T_B$ est comprise entre 15 et 60 mn.

54. Procédé de préparation d'un des énantiomères du modafinil comprenant les étapes suivantes:

a) réaliser un dédoublement des deux énantiomères optiques de l'acide (±) modafinique ou des sels de celui-ci selon un procédé de cristallisation préférentielle, tel que défini dans les revendications 35 à 53;
b) convertir ledit énantiomère en amide;
c) récupérer l'énantiomère du modafinil obtenu.

55. Procédé selon l'aspect 54, dans lequel l'étape b) est réalisée en deux temps:

b1) convertir ledit énantiomère en ester d'alkyle inférieur;
b2) convertir le produit obtenu à l'étape b1) en amide.

56. Composition pharmaceutique comprenant la forme polymorphique du (-)-modafinil ou du (+)-modafinil, désignée forme (II), éventuellement en association avec un véhicule pharmaceutiquement acceptable.

57. Composition pharmaceutique comprenant la forme polymorphique du (-)-modafinil ou du (+)-modafinil, désignée forme (III), éventuellement en association avec un véhicule pharmaceutiquement acceptable.

58. Composition pharmaceutique comprenant la forme polymorphique du (-)-modafinil ou du (+)-modafinil, désignée forme (IV), éventuellement en association avec un véhicule pharmaceutiquement acceptable.

59. Composition pharmaceutique comprenant la forme polymorphique du (-)- modafinil ou du (+)-modafinil, désignée forme (V), éventuellement en association avec un véhicule pharmaceutiquement acceptable.

60. Utilisation de la forme polymorphique du (-)-modafinil ou du (+)-modafinil désignée forme II pour la fabrication d'un médicament destiné à la prévention ou au traitement d'une affection choisie parmi l'hypersomnie, dont notamment l'hypersomnie idiopathique et l'hypersomnie chez les patients atteints d'un cancer traités par des analgésiques morphiniques pour soulager la douleur ; les apnées du sommeil, la somnolence excessive associée à une maladie, les apnées du sommeil obstructives, la narcolepsie ; les somnolences, les somnolences excessives, les somnolences excessives liées à la narcolepsie ; les troubles du système nerveux central tels que la maladie de Parkinson ; la protection du tissu cérébral vis-à-vis de l'ischémie, les troubles de la vigilance, dont notamment les troubles de la vigilance liés à la maladie de Steinert, les troubles de l'attention, par exemple liés à l'hyperactivité (ADHD) ; l'état de fatigue, en particulier celui lié à des scléroses multiples et autres maladies dégénératives ; la dépression, l'état dépressif lié au faible ensoleillement, la schizophrénie, le travail par roulement, le décalage horaire ; les troubles du comportement alimentaire, dans lequel le modafinil agit en tant que stimulateur d'appétit, la stimulation des fonctions cognitives à faibles doses.

61. Utilisation de la forme polymorphique du (-)-modafinil ou du (+)-modafinil désignée forme III pour la fabrication d'un médicament destiné à la prévention ou au traitement d'une affection choisie parmi l'hypersomnie, dont notamment l'hypersomnie idiopathique et l'hypersomnie chez les patients atteints d'un cancer traités par des analgésiques morphiniques pour soulager la douleur ; les apnées du sommeil, la somnolence excessive associée à une maladie, les apnées du sommeil obstructives, la narcolepsie ; les somnolences, les somnolences excessives, les somnolences excessives liées à la narcolepsie ; les troubles du système nerveux central tels que la maladie de Parkinson ; la protection du tissu cérébral vis-à-vis de l'ischémie, les troubles de la vigilance, dont notamment les troubles de la vigilance liés à la maladie de Steinert, les troubles de l'attention, par exemple liés à l'hyperactivité (ADHD) ; l'état de fatigue, en particulier celui lié à des scléroses multiples et autres maladies dégénératives ; la dépression, l'état dépressif lié au faible ensoleillement, la schizophrénie, le travail par roulement, le décalage horaire ; les troubles du comportement alimentaire, dans lequel le modafinil agit en tant que stimulateur d'appétit, la stimulation des fonctions cognitives à faibles doses.

62. Utilisation de la forme polymorphique du (-)-modafinil ou du (+)-modafinil désignée forme IV pour la fabrication d'un médicament destiné à la prévention ou au traitement d'une affection choisie parmi l'hypersomnie, dont notamment l'hypersomnie idiopathique et l'hypersomnie chez les patients atteints d'un cancer traités par des analgésiques

morphiniques pour soulager la douleur ; les apnées du sommeil, la somnolence excessive associée à une maladie, les apnées du sommeil obstructives, la narcolepsie ; les somnolences, les somnolences excessives, les somnolences excessives liées à la narcolepsie ; les troubles du système nerveux central tels que la maladie de Parkinson ; la protection du tissu cérébral vis-à-vis de l'ischémie, les troubles de la vigilance, dont notamment les troubles de la vigilance liés à la maladie de Steinert, les troubles de l'attention, par exemple liés à l'hyperactivité (ADHD) ; l'état de fatigue, en particulier celui lié à des scléroses multiples et autres maladies dégénératives ; la dépression, l'état dépressif lié au faible ensoleillement, la schizophrénie, le travail par roulement, le décalage horaire ; les troubles du comportement alimentaire, dans lequel le modafinil agit en tant que stimulateur d'appétit, la stimulation des fonctions cognitives à faibles doses.

63. Utilisation de la forme polymorphique du (-)-modafinil ou du (+)-modafinil désignée forme V pour la fabrication d'un médicament destiné à la prévention ou au traitement d'une affection choisie parmi l'hypersomnie, dont notamment l'hypersomnie idiopathique et l'hypersomnie chez les patients atteints d'un cancer traités par des analgésiques morphiniques pour soulager la douleur ; les apnées du sommeil, la somnolence excessive associée à une maladie, les apnées du sommeil obstructives, la narcolepsie ; les somnolences, les somnolences excessives, les somnolences excessives liées à la narcolepsie ; les troubles du système nerveux central tels que la maladie de Parkinson ; la protection du tissu cérébral vis-à-vis de l'ischémie, les troubles de la vigilance, dont notamment les troubles de la vigilance liés à la maladie de Steinert, les troubles de l'attention, par exemple liés à l'hyperactivité (ADHD) ; l'état de fatigue, en particulier celui lié à des scléroses multiples et autres maladies dégénératives ; la dépression, l'état dépressif lié au faible ensoleillement, la schizophrénie, le travail par roulement, le décalage horaire ; les troubles du comportement alimentaire, dans lequel le modafinil agit en tant que stimulateur d'appétit, la stimulation des fonctions cognitives à faibles doses.

## Revendications

1. Forme polymorphique de l'énantiomère dextrogyre ou lévogyre du modafinil, désignée forme IV, caractérisée en que (i) elle produit un spectre de diffraction X aux distances réticulaires : 12,38 ; 8,58 ; 7,34 ; 5,00 ; 4,09 (Å) ; ou (ii) elle produit un spectre de diffraction X comprenant des raies d'intensité aux valeurs d'angle 2 theta : 7,14 ; 10,30 ; 12,04 ; 17,72 ; 21,70 (degrés), mesurées en utilisant un diffractomètre Siemens AG en utilisant une radiation de cuivre filtrée au nickel à $\lambda$ = 1,540 Å, l'erreur pour les valeurs 2 theta étant de $\pm$ 0,2 degrés 2 theta.

2. Forme polymorphique selon la revendication 1, **caractérisée en ce qu'**elle produit un spectre de diffraction X comprenant des raies d'intensité aux distances réticulaires : 12,38 ; 8,58 ; 7,34 ; 5,00 ; 4,09 (Å).

3. Forme polymorphique selon la revendication 2, **caractérisée en ce qu'**elle produit un spectre de diffraction X comprenant des raies d'intensité aux distances réticulaires : 13,88 ; 12,38 ; 10,27 ; 8,58 ; 7,34 ; 6,16 ; 5,660 ; 5,120 ; 5,00 ; 4,64 ; 4,48 ; 4,26 ; 4,18 ; 4,09 ; 3,82 ; 3,66 ; 3,53 ; 3,42 ; 3,28 ; 3,20 (Å).

4. Forme polymorphique selon la revendication 1, **caractérisée en ce qu'**elle produit un spectre de diffraction X comprenant des raies d'intensité aux valeurs d'angle 2 theta : 7,14 ; 10,30 ; 12,04 ; 17,72 ; 21,70 (degrés), mesurées en utilisant un diffractomètre Siemens AG en utilisant une radiation de cuivre filtrée au nickel à $\lambda$ = 1,540 Å, l'erreur pour les valeurs 2 theta étant de $\pm$ 0,2 degrés 2 theta.

5. Forme polymorphique selon la revendication 4, **caractérisée en ce qu'**elle produit un spectre de diffraction X comprenant des raies d'intensité aux valeurs d'angle 2 theta : 6,37 ; 7,14 ; 8,60 ; 10,30 ; 12,04 ; 14,37 ; 15,65 ; 17,30 ; 17,72 ; 19,12 ; 19,81 ; 20,82 ; 21,24 ; 21,70 ; 23,28 ; 24,30 ; 25,18 ; 26,02 ; 27,13 ; 27,90 (degrés), mesurées en utilisant un diffractomètre Siemens AG en utilisant une radiation de cuivre filtrée au nickel à $\lambda$ = 1,540 Å, l'erreur pour les valeurs 2 theta étant de $\pm$ 0,2 degrés 2 theta.

6. Forme polymorphique selon l'une quelconque des revendications 1 à 5, dans laquelle la forme polymorphique est caractérisée comme suit :

| CRL 40982 FORME IV | | |
|---|---|---|
| 2 Theta (degrés) | d (Å) | I/Io(%) |
| 6,37 $\pm$ 0,2 | 13,88 | 26 |

(suite)

| CRL 40982 FORME IV | | |
|---|---|---|
| 2 Theta (degrés) | d (Å) | I/Io(%) |
| 7,14 ± 0,2 | 12,38 | 69 |
| 8,60 ± 0,2 | 10,27 | 23 |
| 10,30 ± 0,2 | 8,58 | 100 |
| 12,04 ± 0,2 | 7,34 | 49 |
| 14,37 ± 0,2 | 6,16 | 24 |
| 15,65 ± 0,2 | 5,66 | 11 |
| 17,30 ± 0,2 | 5,12 | 29 |
| 19,12 ± 0,2 | 4,64 | 15 |
| 19,81 ± 0,2 | 4,48 | 25 |
| 20,82 ± 0,2 | 4,26 | 10 |
| 21,24 ± 0,2 | 4,18 | 12 |
| 21,70 ± 0,2 | 4,09 | 51 |
| 23,28 ± 0,2 | 3,82 | 9 |
| 24,30 ± 0,2 | 3,66 | 30 |
| 25,18 ± 0,2 | 3,53 | 9 |
| 26,02 ± 0,2 | 3,42 | 21 |
| 27,13 ± 0,2 | 3,28 | 9 |
| 27,90 ± 0,2 | 3,20 | 15 |

ces valeurs étant telles que mesurées en utilisant un diffractomètre Siemens AG en utilisant une radiation de cuivre filtrée au nickel à $\lambda$ = 1,540 Å

**7.** Forme polymorphique du modafinil selon l'une quelconque des revendications 1 à 6, dans laquelle l'énantiomère est l'énantiomère (-).

**8.** Forme polymorphique du modafinil selon l'une quelconque des revendications 1 à 6, dans laquelle l'énantiomère est l'énantiomère (+).

**9.** Composition pharmaceutique comprenant la forme polymorphique du (-)-modafinil ou du (+)-modafinil, désignée forme IV, selon l'une quelconque des revendications 1 à 8, éventuellement en association avec un véhicule pharmaceutiquement acceptable.

**10.** Composition pharmaceutique constituée par la forme polymorphique du modafinil dextrogyre ou lévogyre, désignée forme IV, selon l'une quelconque des revendications 1 à 8, éventuellement en association avec un véhicule pharmaceutiquement acceptable.

**11.** Utilisation de la forme polymorphique du (-)-modafinil ou du (+)-modafinil selon l'une quelconque des revendications 1 à 8, désignée forme IV, pour la fabrication d'un médicament destiné à la prévention ou au traitement d'une affection choisie parmi l'hypersomnie, dont notamment l'hypersomnie idiopathique et l'hypersomnie chez les patients atteints d'un cancer traités par des analgésiques morphiniques pour soulager la douleur ; les apnées du sommeil, la somnolence excessive associée à une maladie, les apnées du sommeil obstructives, la narcolepsie ; les somnolences, les somnolences excessives, les somnolences excessives liées à la narcolepsie ; les troubles du système nerveux central tels que la maladie de Parkinson ; la protection du tissu cérébral vis-à-vis de l'ischémie, les troubles de la vigilance, dont notamment les troubles de la vigilance liés à la maladie de Steinert, les troubles de l'attention, par exemple liés à l'hyperactivité (ADHD) ; l'état de fatigue, en particulier celui lié à des scléroses multiples et autres

maladies dégénératives ; la dépression, l'état dépressif lié au faible ensoleillement, la schizophrénie, le travail par roulement, le décalage horaire ; les troubles du comportement alimentaire, dans lequel le modafinil agit en tant que stimulateur d'appétit, la stimulation des fonctions cognitives à faibles doses.

**12.** Procédé de préparation d'une forme polymorphique de l'énantiomère lévogyre ou dextrogyre du modafinil, désignée forme IV, caractérisé en qu'elle produit un spectre de diffraction X comprenant des raises d'intensité aux distances réticulaires : 12,38 ; 8,58 ; 7,34 ; 5,00 ; 4,09 (Å), ledit procédé comprenant les étapes suivantes :

i) dissoudre l'un des énantiomères optiques du modafinil dans un solvant ;
ii) cristalliser l'énantiomère du modafinil ;
iii) récupérer la forme cristalline de l'énantiomère du modafinil ainsi obtenue, dans lequel le solvent mis en oeuvre à l'étape i) est le tétrahydrofuranne, le chloroforme, ou la méthyléthylcétone.

**13.** Procédé selon la revendication 12, dans lequel la cristallisation consiste en un refroidissement de la solution obtenue à l'étape i) et dans lequel le refroidissement est lent.

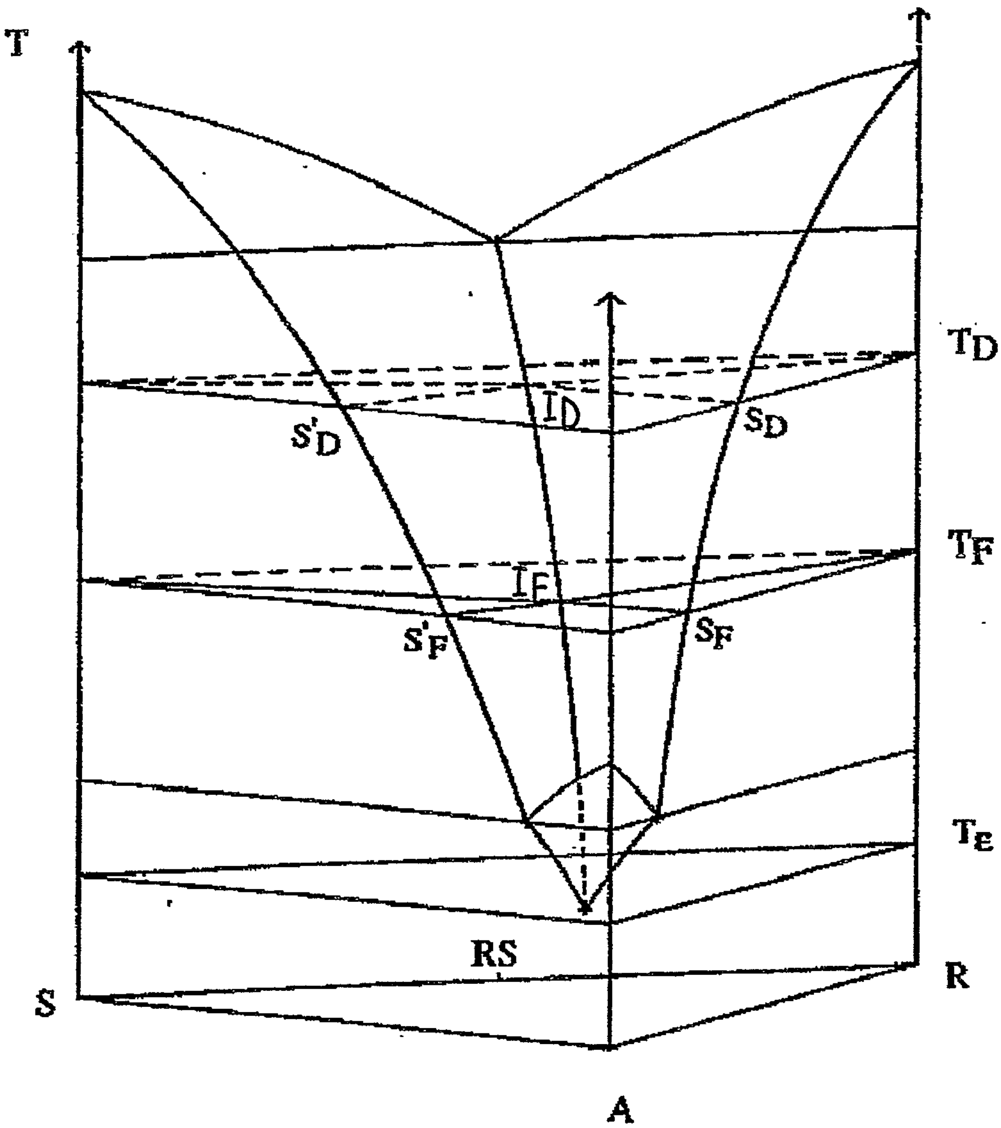


FIG.1

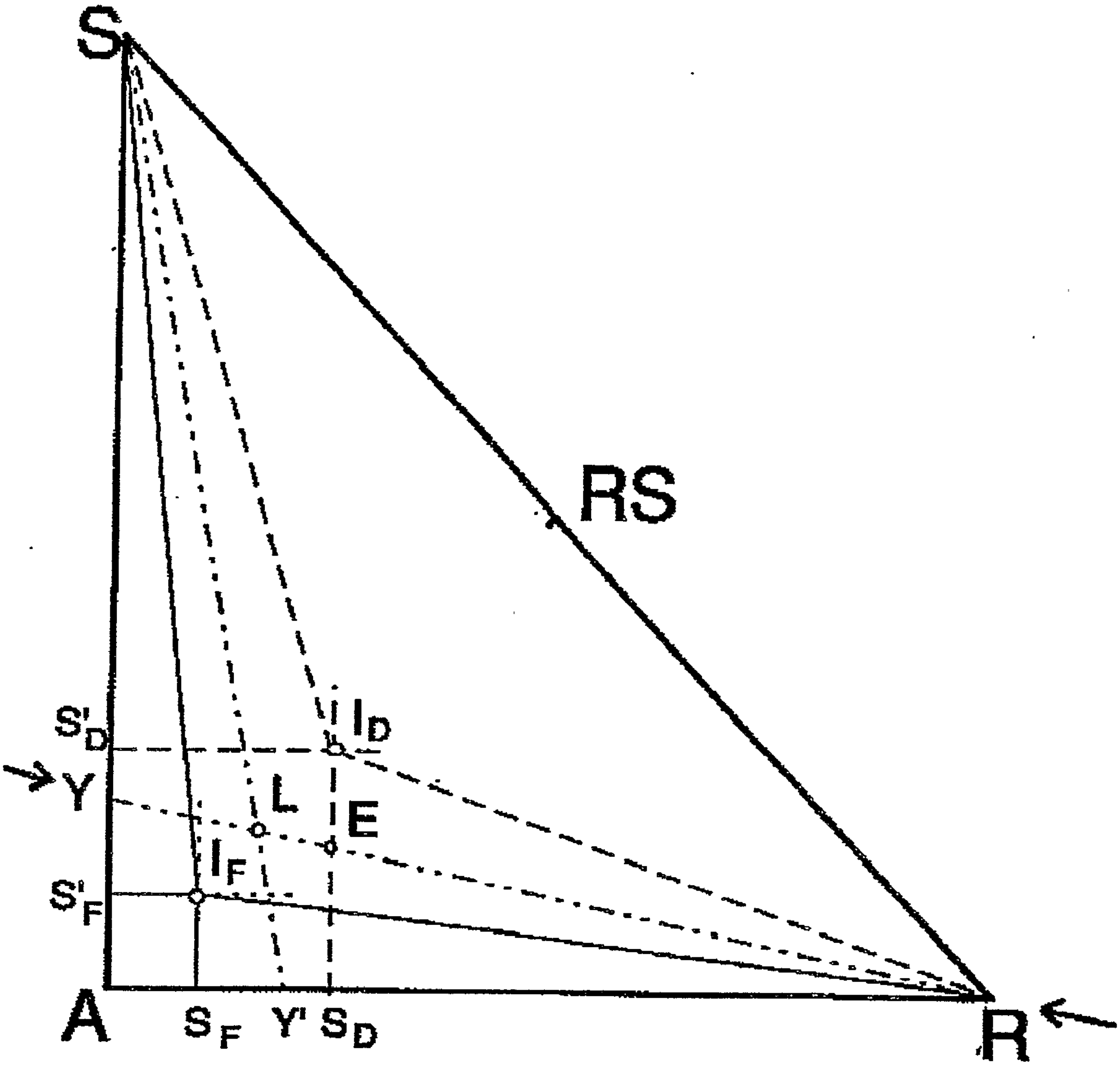
S
RS
S'D
ID
Y
L
E
IF
S'F
A
SF
Y'
SD
R
LEGENDE : Equilibre à TF
Equilibre à TD
Coupe isoplèthe RY

FIG.2

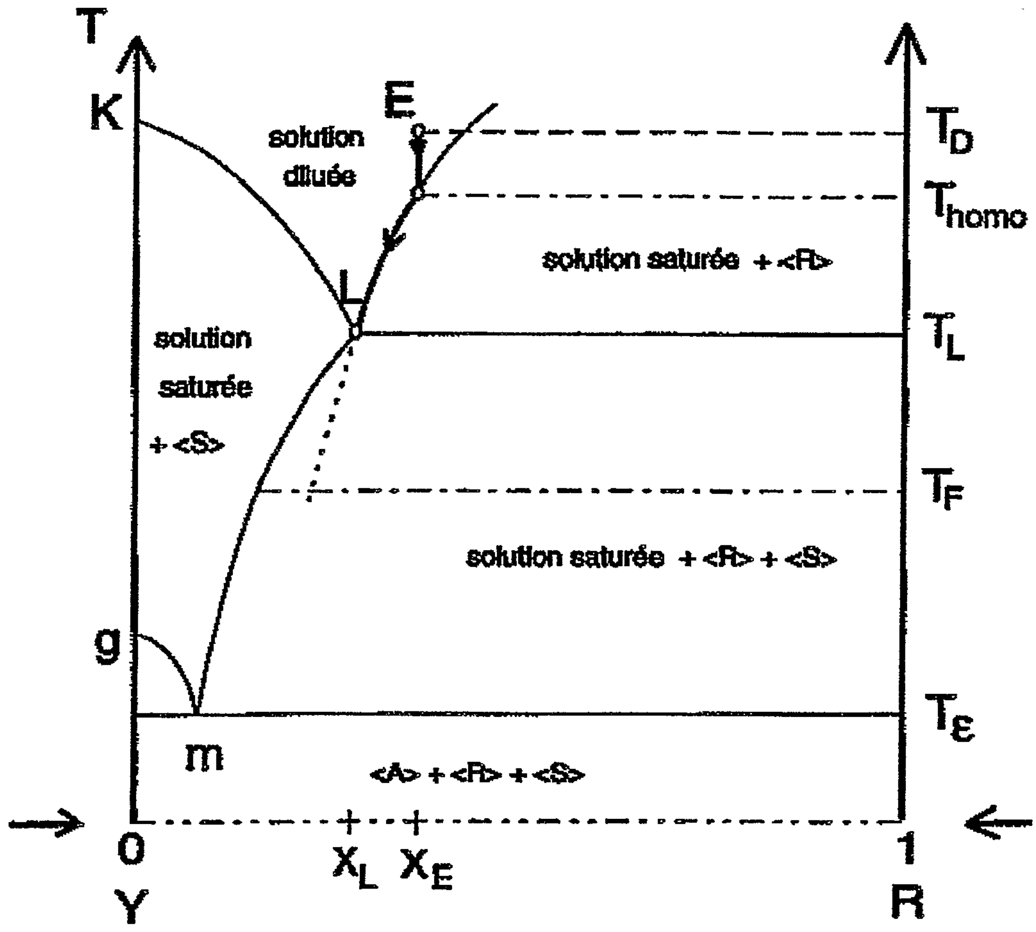
T
K
E
solution diluée
$T_D$
$T_{homo}$
solution saturée + <R>
L
solution saturée + <S>
$T_L$
$T_F$
solution saturée + <R> + <S>
g
$T_\varepsilon$
m
<A> + <R> + <S>
0
$X_L$ $X_E$
1
Y
R

FIG.3

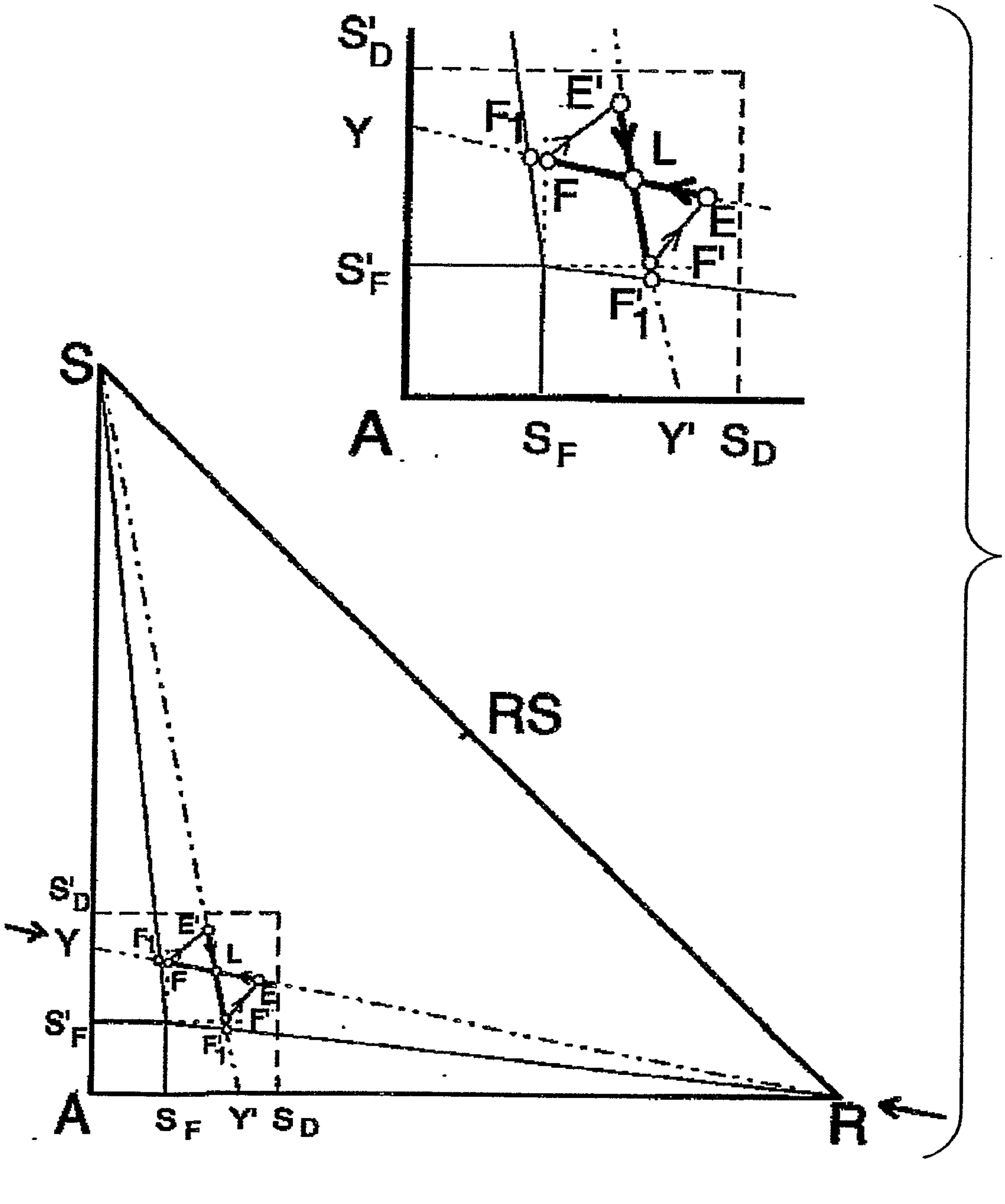


FIG.4

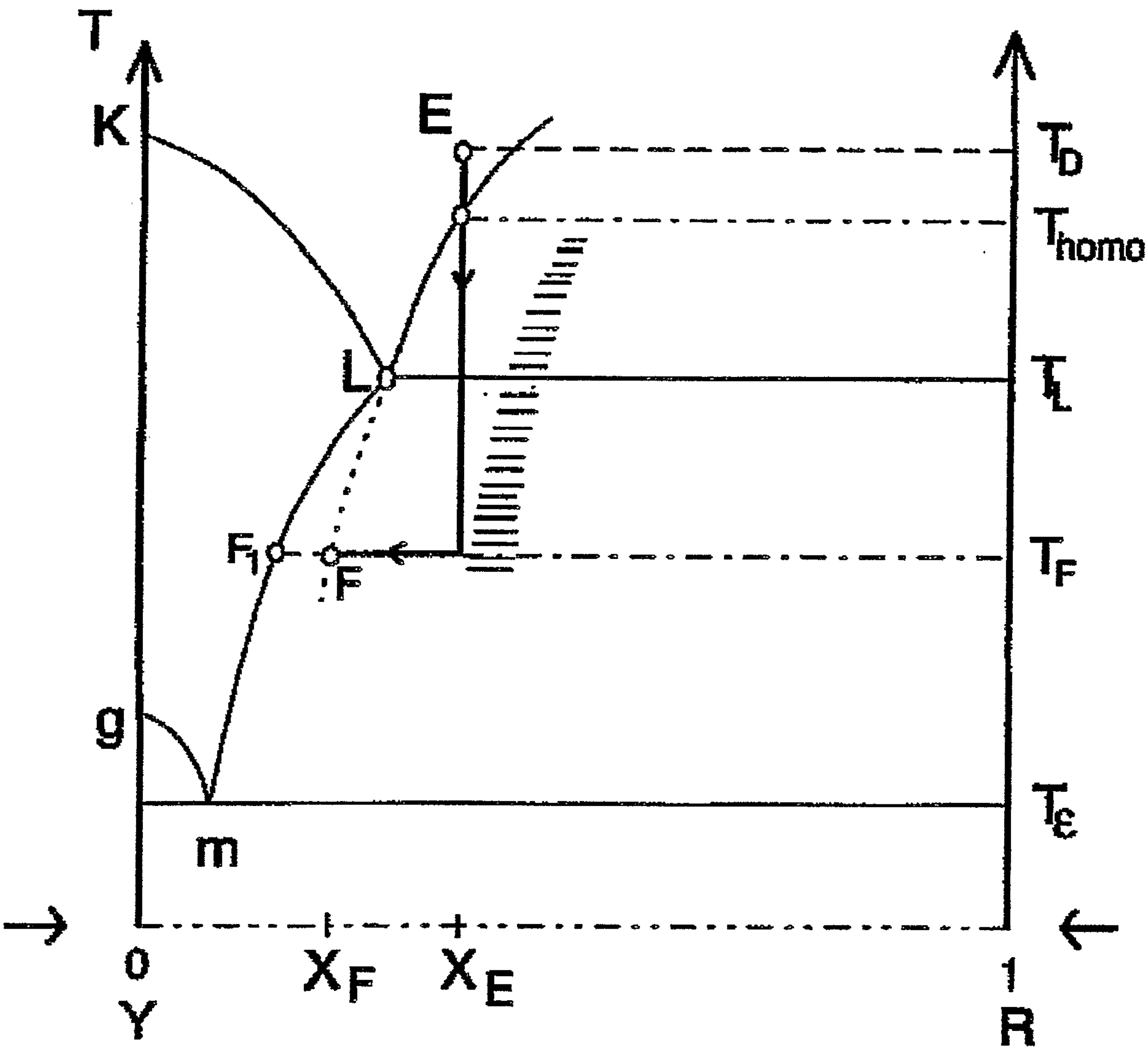


FIG.5

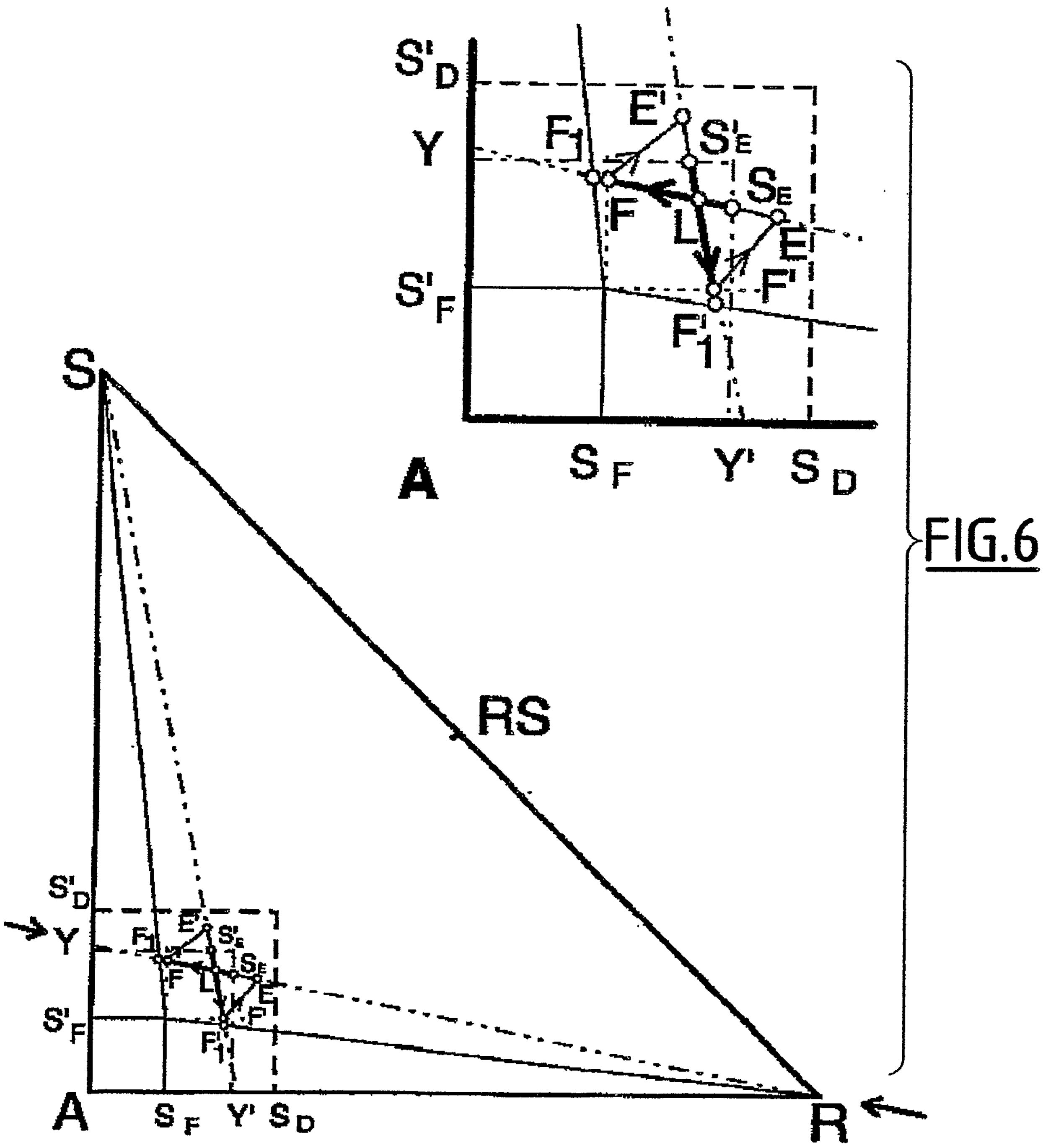
S'D
Y
E'
F1
S'E
F
L
SE
E
S'F
F'
F'1
A
SF
Y'
SD
S
RS
S'D
Y
F1
E'
S'E
SE
F
L
E
S'F
F'
F'1
A
SF
Y'
SD
R

FIG.6

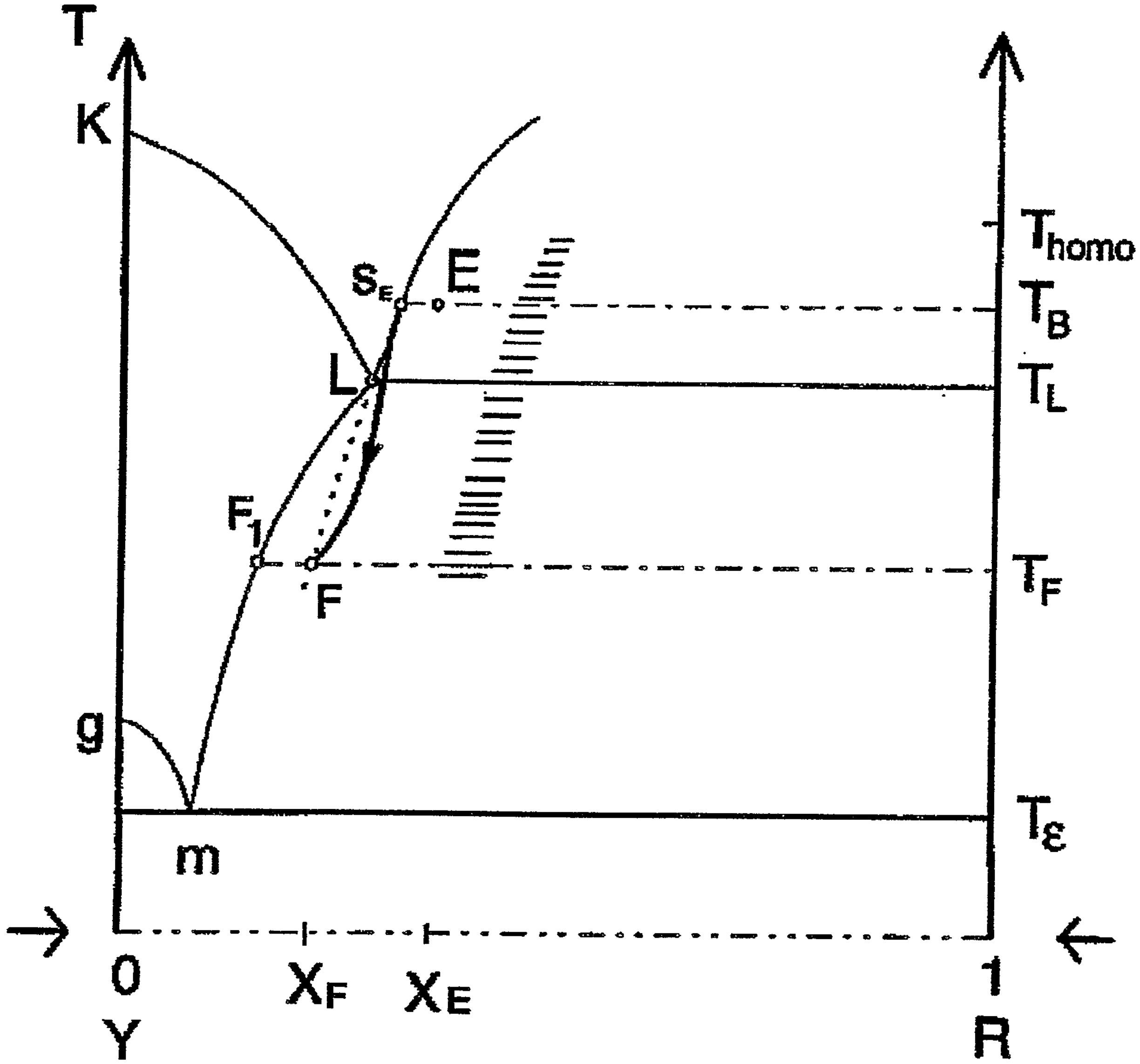


FIG.7

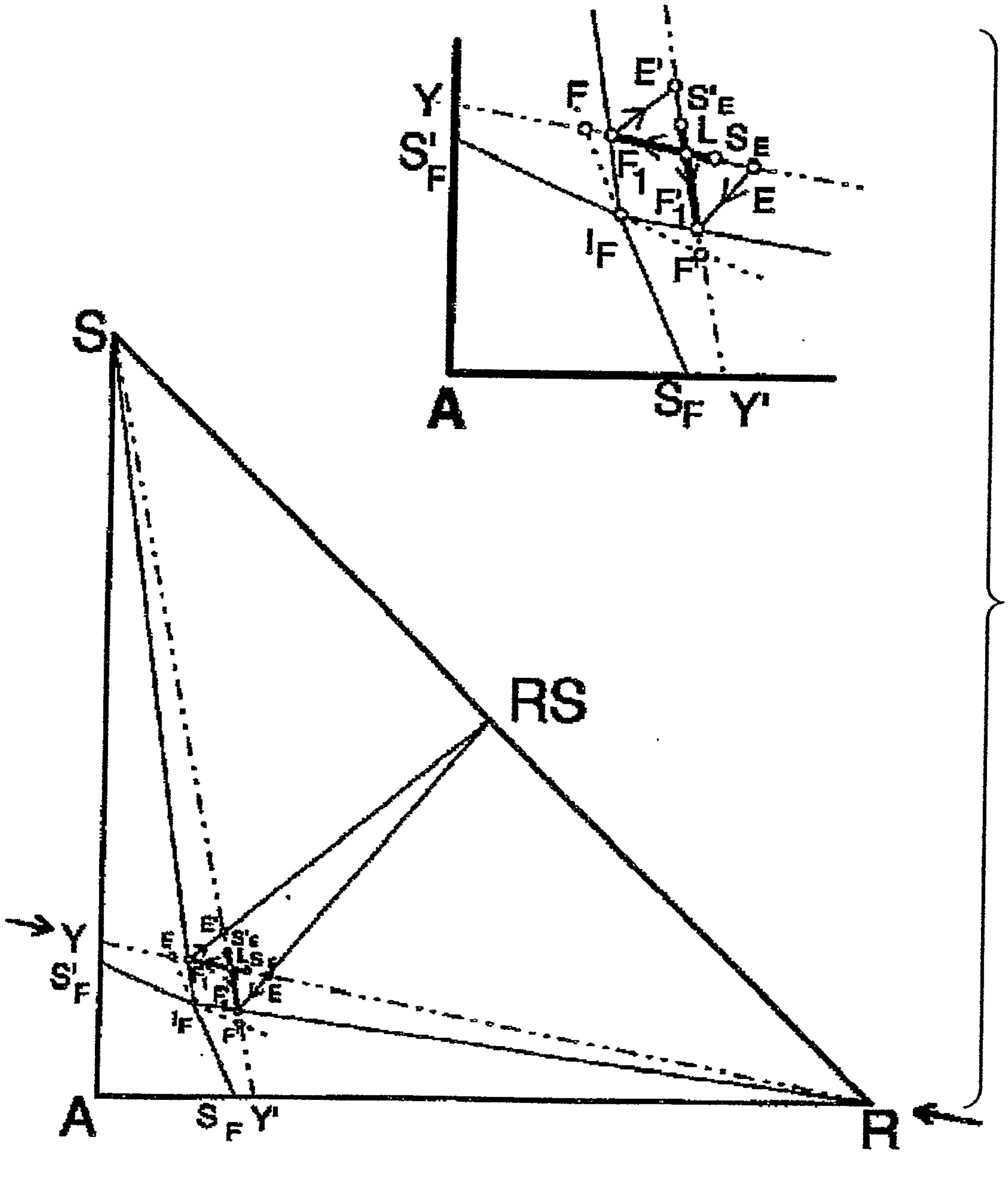


FIG.8

FORME II

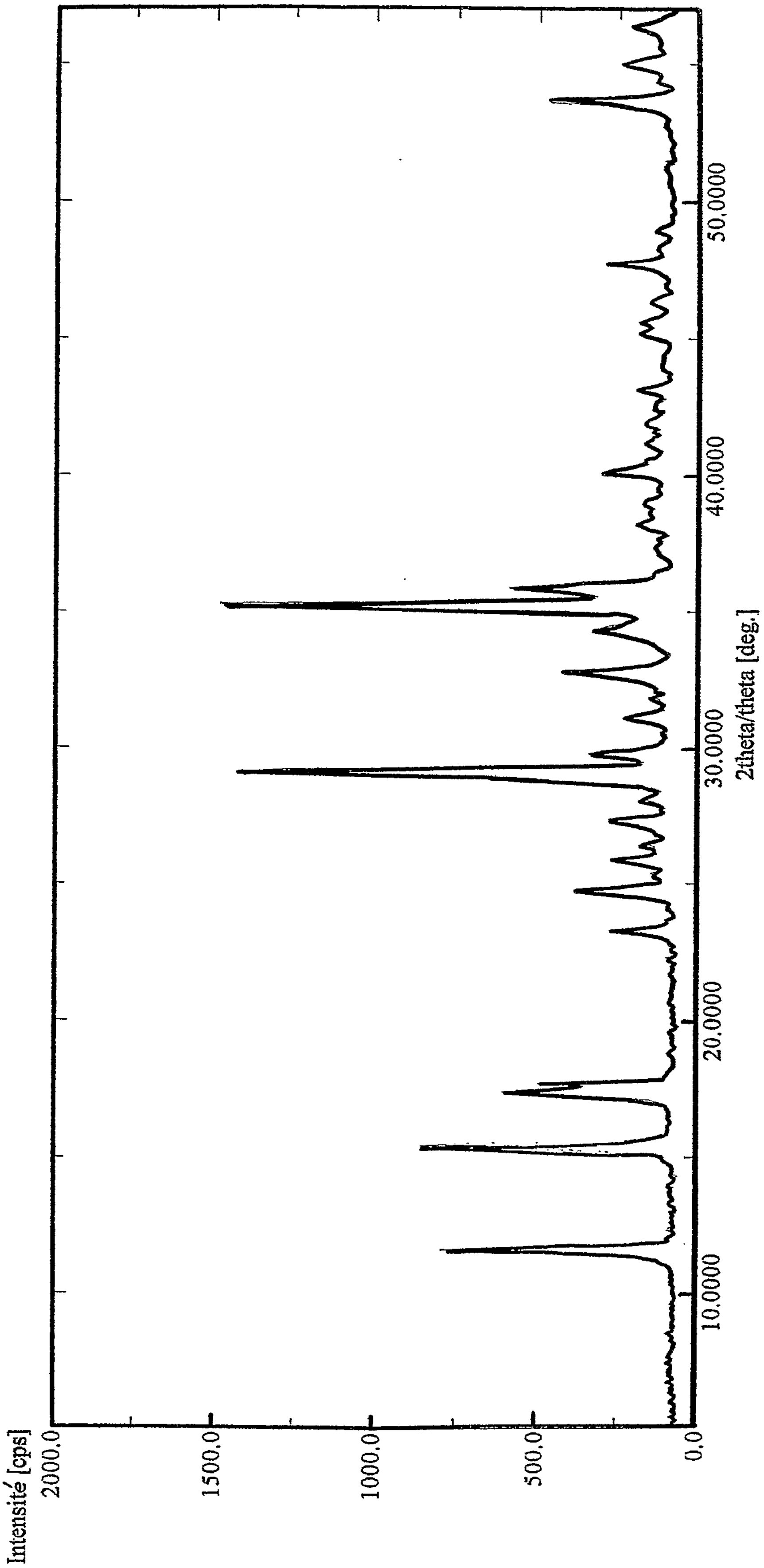


FIG.9

FORME III

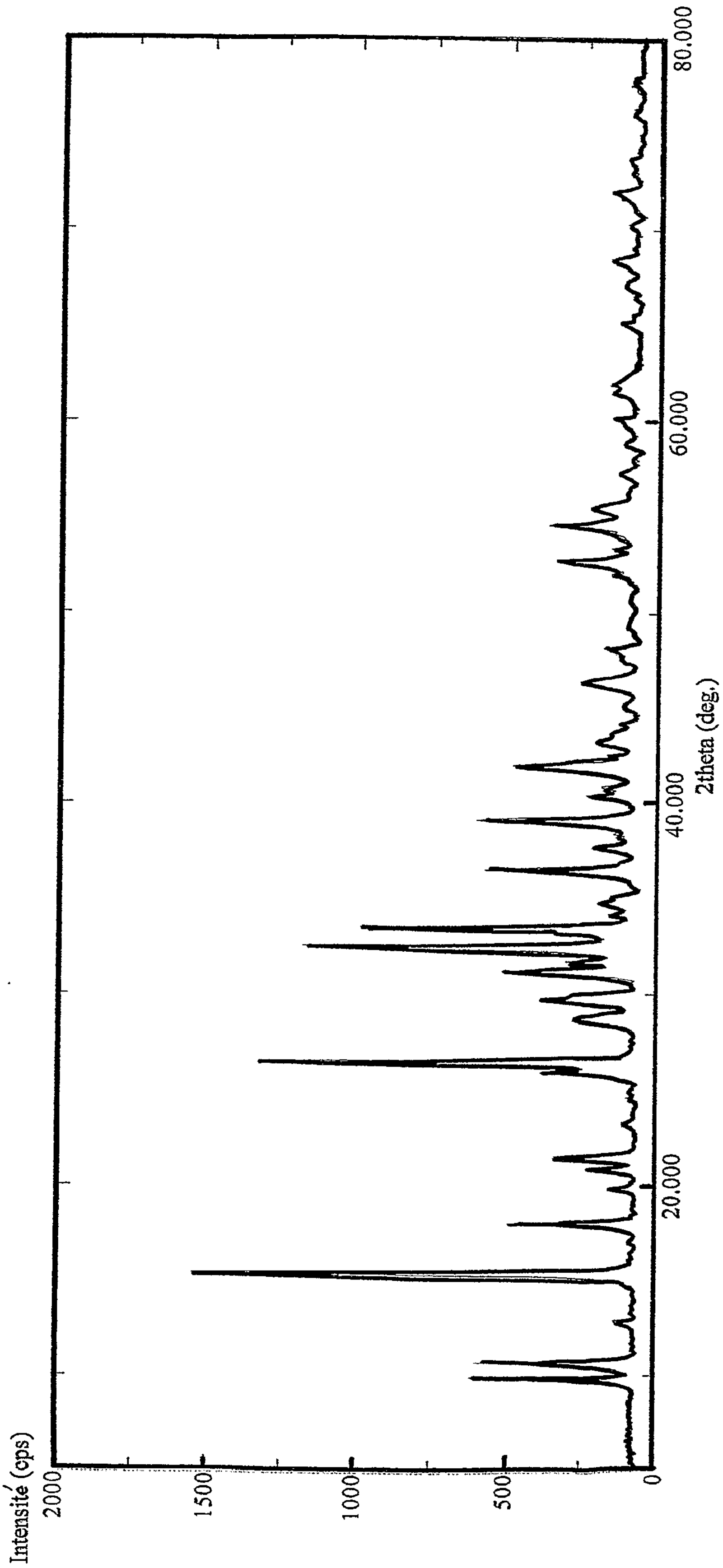


FIG.10

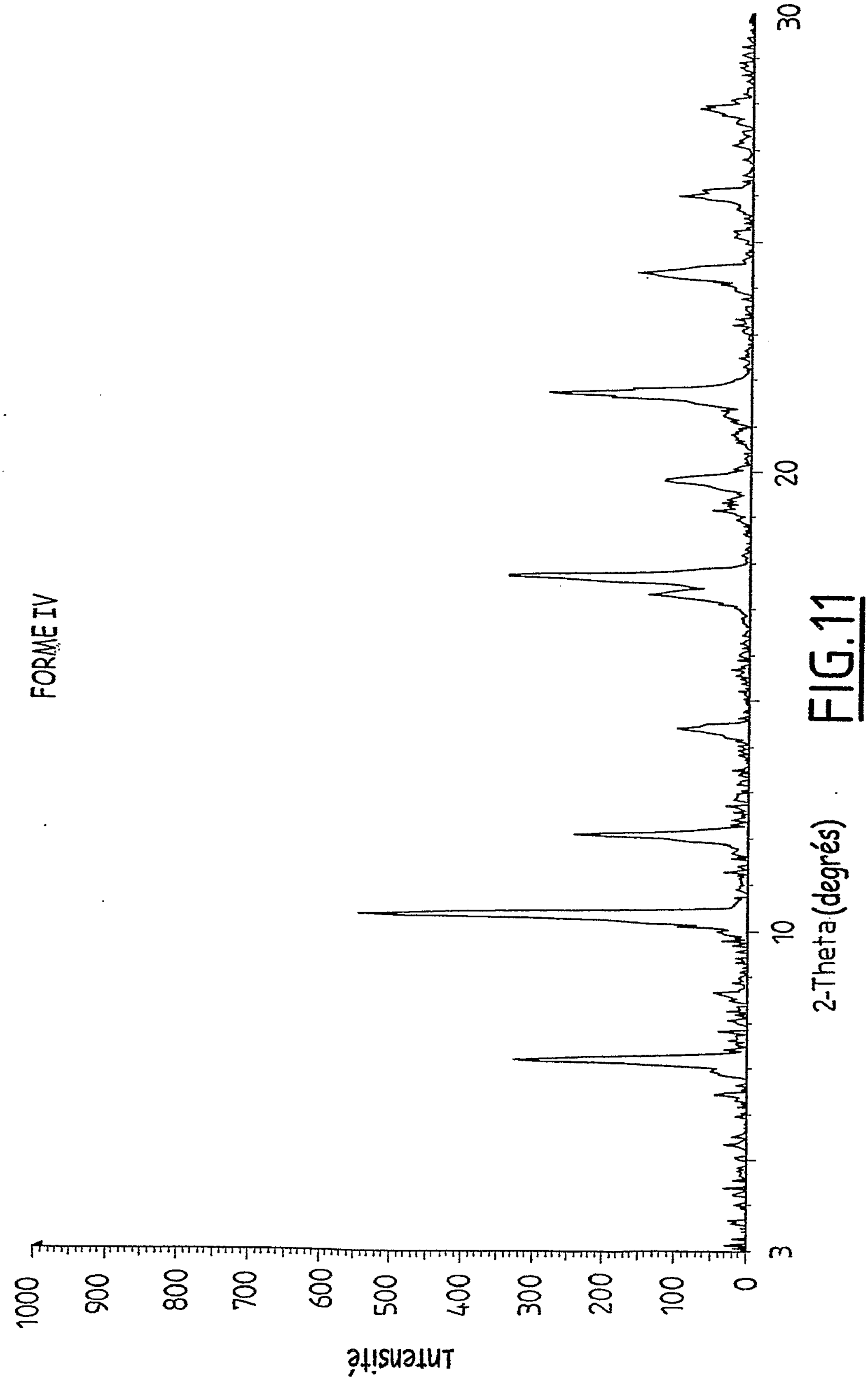
FORME IV
Intensité
0
100
200
300
400
500
600
700
800
900
1000
3
10
20
30
2-Theta (degrés)

FIG.11

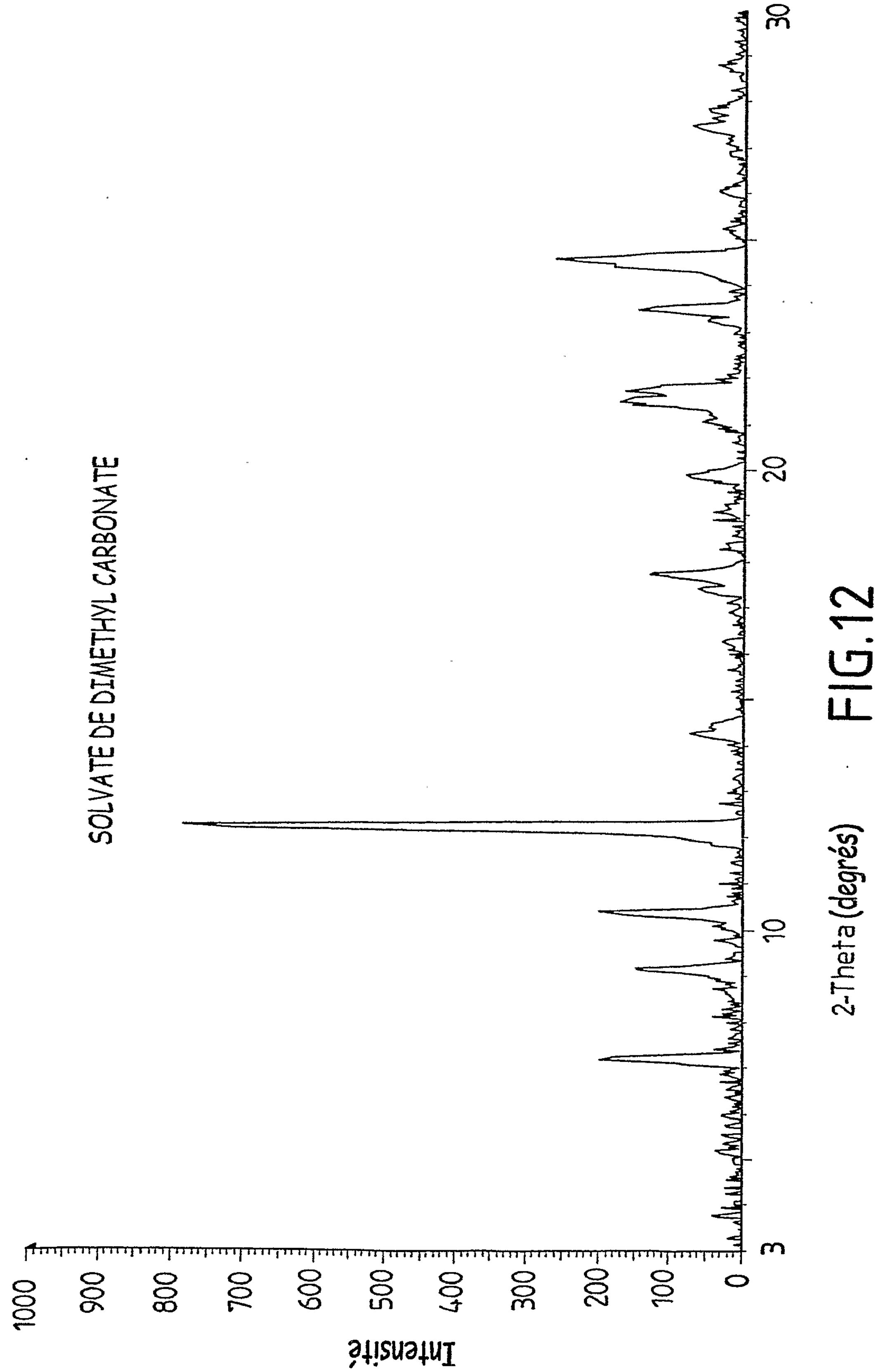
SOLVATE DE DIMETHYL CARBONATE
Intensité
1000
900
800
700
600
500
400
300
200
100
0
3
10
20
30
2-Theta (degrés)

FIG.12

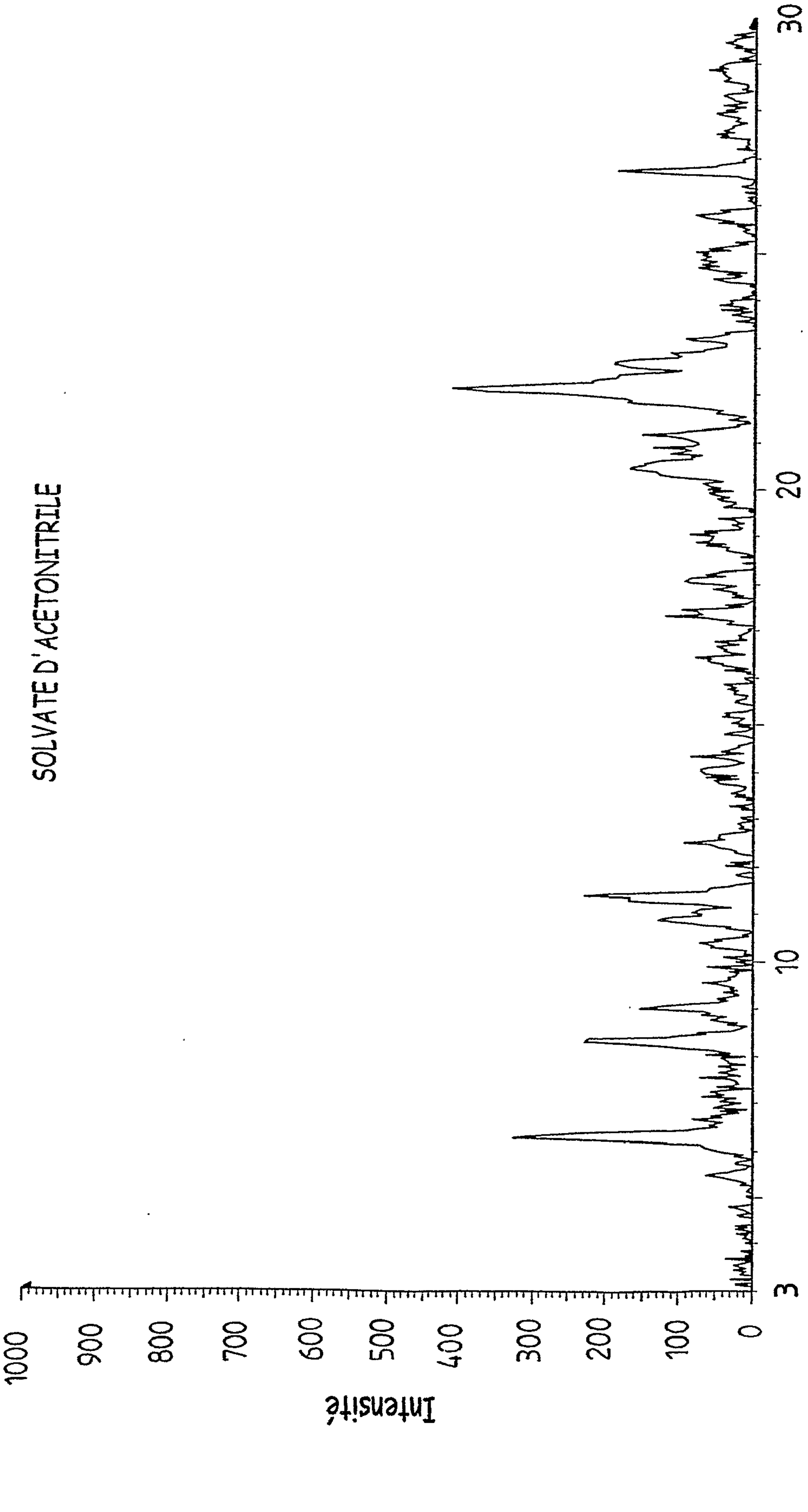
SOLVATE D'ACETONITRILE
Intensité
1000
900
800
700
600
500
400
300
200
100
0
3
10
20
30
2-Theta (degrés)

FIG.13

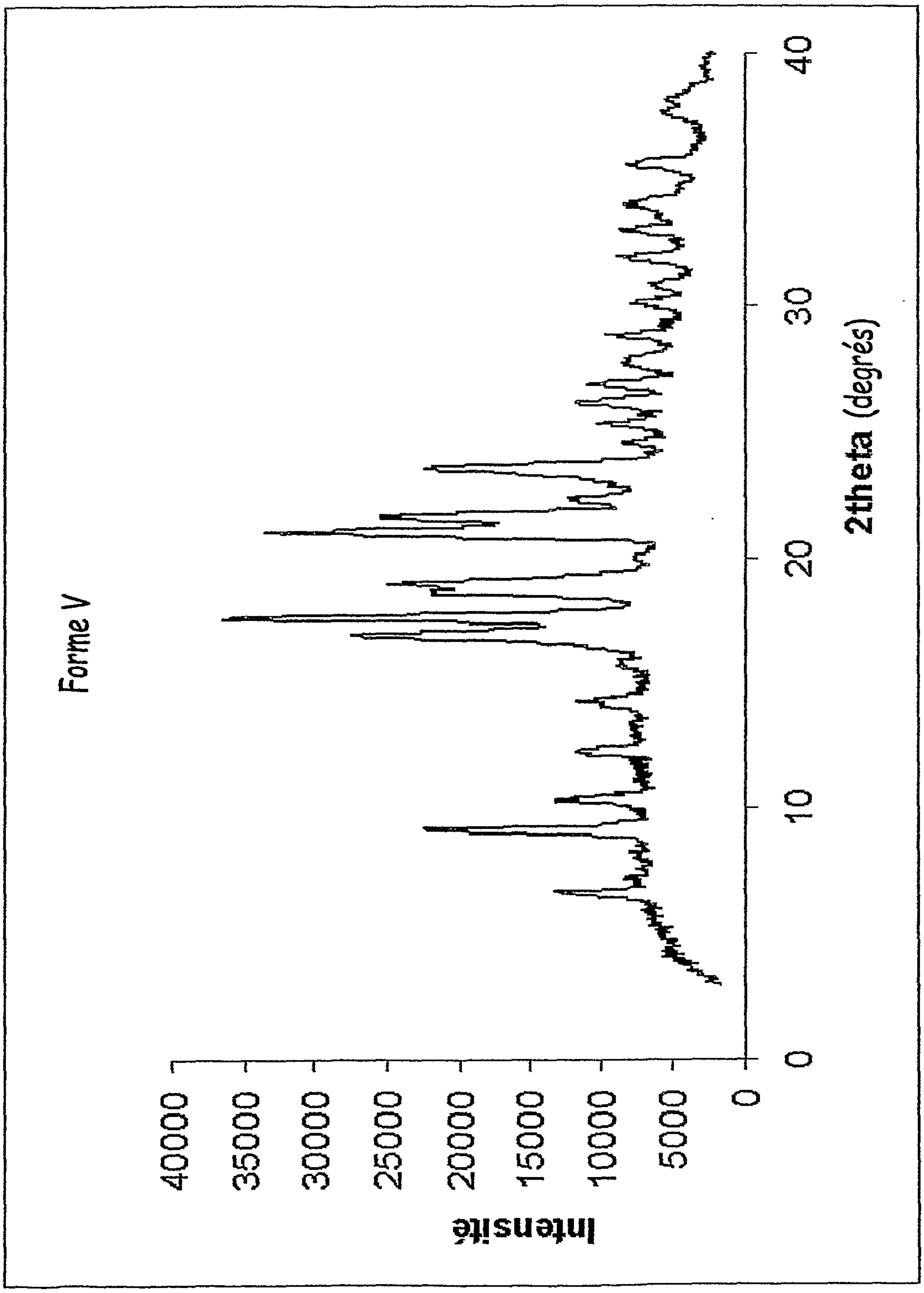
Forme V
Intensité
40000
35000
30000
25000
20000
15000
10000
5000
0
10
20
30
40
2theta (degrés)

FIG.14

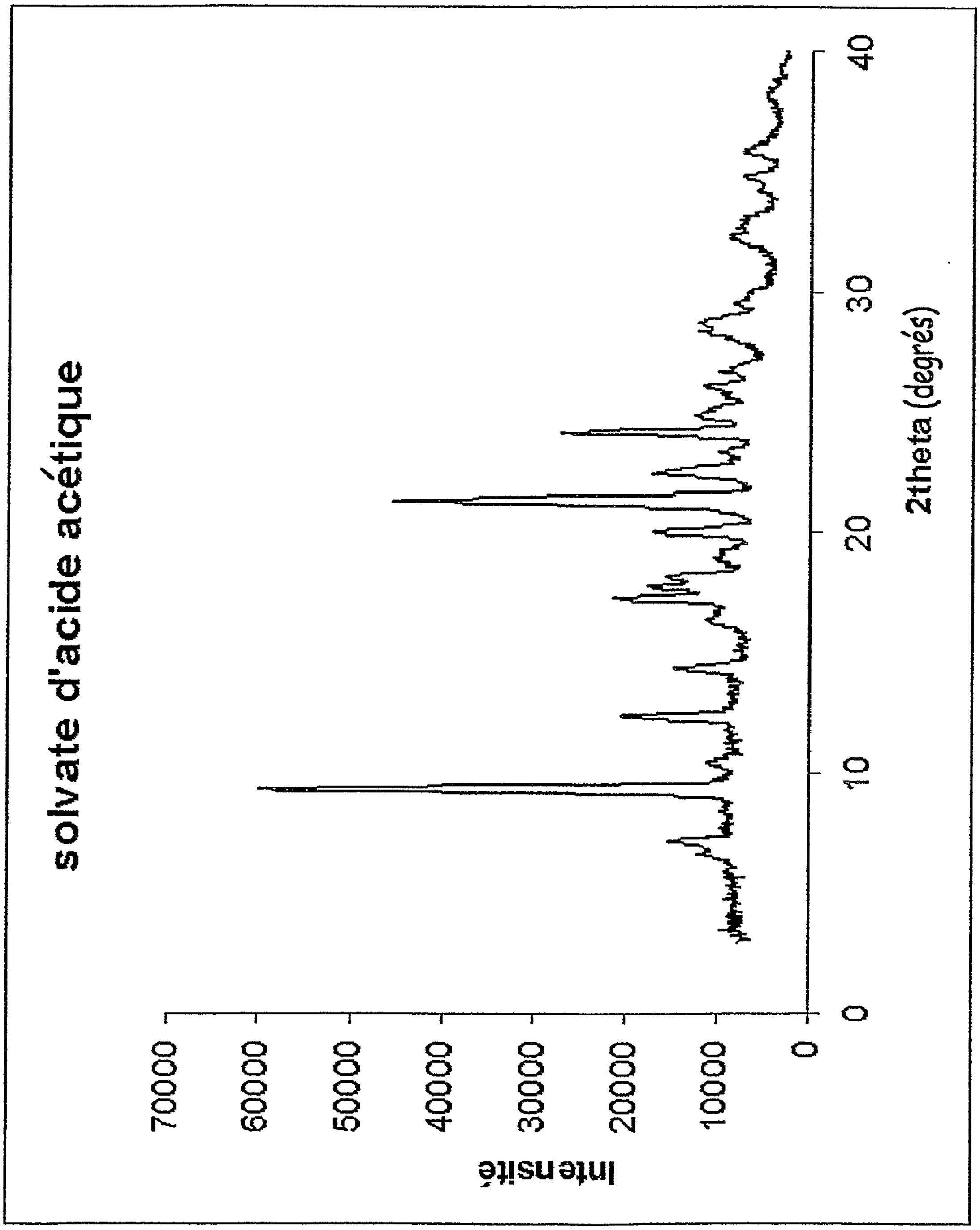
solvate d'acide acétique
Intensité
70000
60000
50000
40000
30000
20000
10000
0
0
10
20
30
40
2theta (degrés)

FIG.15

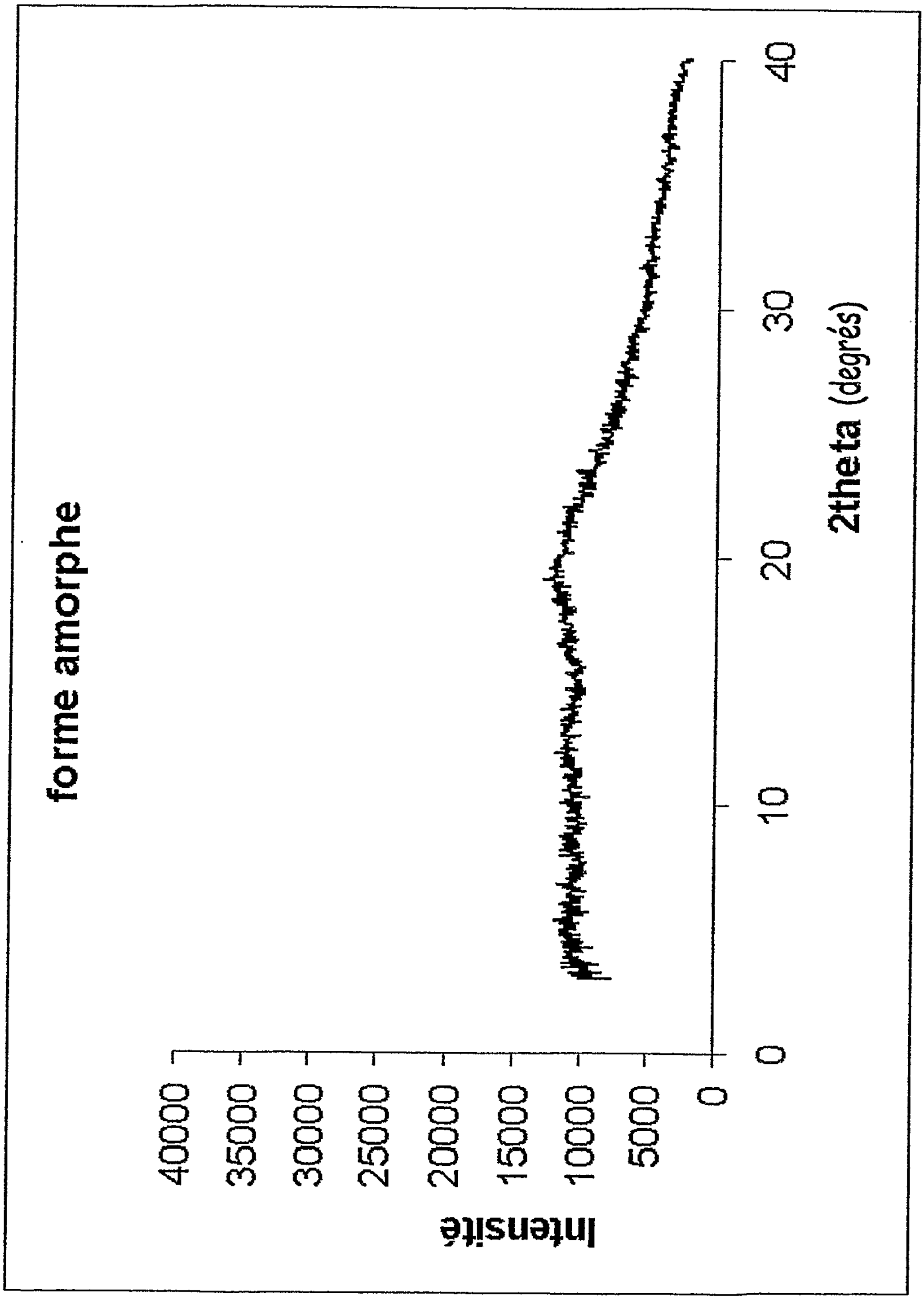
forme amorphe
Intensité
40000
35000
30000
25000
20000
15000
10000
5000
0
0
10
20
30
40
2theta (degrés)

FIG.16

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4177290 A
- US 4927855 A
- EP 0720595 B1, G. Coquerel, M.-N. Petit et R. Bouaziz
- GB 1197809 A


### Littérature non-brevet citée dans la description


- **DE MIN., M. ; LEVY, G. ; MICHEAU J.-C.** *J. Chem. Phys.,* 1988, vol. 85, 603-19
- **J. BERNSTEIN.** Polymorphism in molecular crystals. University Press, 2002
- **DE G. COQUEREL.** Enantiomer. Gordon and Breach Science Publishers, 2000, vol. 5, 481-498
- **E. NDZIÉ ; P. CARDINAËL ; A.-R. SCHOOFS ; G. COQUEREL.** *Tetrahedron Asymmetry,* 1997, vol. 8 (17), 2913-2920
- **L. COURVOISIER ; E. NDZIÉ ; M.-N. PETIT ; U. HEDTMANN ; U. SPRENGARD ; G. COQUEREL.** *Chemistry Letters,* 2001, vol. 4, 364-365
- **AMIARD, G.** *Bull. Soc. Chim. Fr.,* 1956, 447
- **COLLET, A. ; BRIENNE, M. J. ; JACQUES, J.** *Chemical reviews,* 1980, vol. 80 (3), 215-30
- **COLLET, A. ; BRIENNE, M. J. ; JACQUES, J.** *Chemical Reviews,* 1980, vol. 80 (3), 215-30